(19) Europäisches Patentamt
European Patent Office
Office européen des brevets

(11) EP 2 332 936 B1

# (12) EUROPEAN PATENT SPECIFICATION

(45) Date of publication and mention of the grant of the patent:
**04.09.2013 Bulletin 2013/36**

(21) Application number: **09816309.0**

(22) Date of filing: **25.09.2009**

(51) Int Cl.:
***C07D 413/04*** (2006.01) ***A01N 43/82*** (2006.01)

(86) International application number:
**PCT/JP2009/067198**

(87) International publication number:
**WO 2010/035899 (01.04.2010 Gazette 2010/13)**

(54) **PYRIDINE COMPOUND AND USE THEREOF FOR PEST CONTROL**

PYRIDINVERBINDUNG UND IHRE VERWENDUNG ZUR SCHÄDLINGSBEKÄMPFUNG

COMPOSÉ DE PYRIDINE ET SES UTILISATIONS POUR LA LUTTE CONTRE LES ANIMAUX NUISIBLES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.09.2008 JP 2008247645**

(43) Date of publication of application:
**15.06.2011 Bulletin 2011/24**

(73) Proprietor: **Sumitomo Chemical Company, Limited**
**Tokyo 104-8260 (JP)**

(72) Inventor: **MIZUNO, Hajime**
**Toyonaka-shi**
**Osaka 561-0802 (JP)**

(74) Representative: **Winter, Brandl, Fürniss, Hübner, Röss, Kaiser, Polte - Partnerschaft**
**Alois-Steinecker-Straße 22**
**85354 Freising (DE)**

(56) References cited:
**WO-A1-98/47369**
**JP-A- 2 104 505**
**JP-A- 2002 205 991**
**JP-T- 2001 520 666**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 332 936 B1

## Description

Technical Field

The present invention relates to a pyridine compound and its use for control of a pest.

Background Art

Conventionally, a lot of compounds have been developed for control of a pest. For example, certain pyridine compounds are known to be effective for control of a pest (see, Japanese Patent Application National Publication (Laid-Open) No. 2001-520666, JP-A No. 2002-205991). However, these pyridine compounds do not necessarily have a sufficient effect on control of a pest in some cases, thus, there is a desire for development of a compound having an excellent effect on control of a pest.

Disclosure of the Invention

The present inventors have intensively investigated to find a compound having an excellent effect on control of a pest, and resultantly found that a compound represented by following general formula (I) has an excellent effect on control of a pest, leading to completion of the present invention.

That is, the present invention includes the following inventions.

[1] A pyridine compound represented by general formula (I):

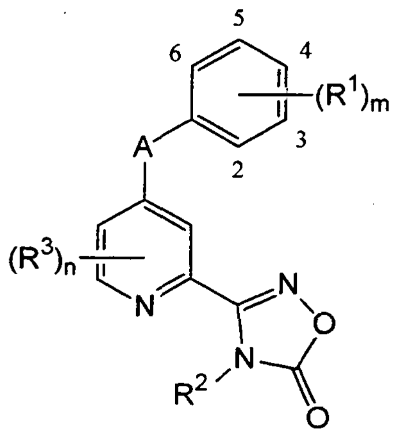

(I)

wherein, $R^1$ represents a C1-C7 alkyl group optionally substituted by halogen, a C1-C7 alkoxy group optionally substituted by halogen, a C1-C7 alkylthio group optionally substituted by halogen, a nitro group, a cyano group or halogen,
m represents an integer of 1 to 5,
A represents a single bond, an oxygen atom, a sulfur atom, $NR^{10}$, $CH_2$ or $CH_2O$,
$R^{10}$ represents a C1-C7 alkyl group optionally substituted by halogen, a C3-C7 alkenyl group optionally substituted by halogen, a C3-C7 alkynyl group optionally substituted by halogen, a C2-C7 alkoxyalkyl group, a cyanomethyl group or hydrogen,
$R^2$ represents a C1-C7 alkyl group optionally substituted by halogen, a cyanomethyl group, a (C3-C7 cycloalkyl) methyl group optionally substituted by one or more members selected from Group $\alpha$, a benzyl group optionally substituted by one or more members selected from Group $\beta$ or hydrogen, alternatively represents any one group selected from the following $Q^1$, $Q^2$, $Q^3$, $Q^4$ and $Q^5$

$Q^1=$ 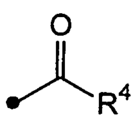 $Q^2=$ 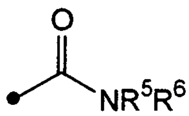

$Q^3$= 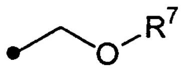 $Q^4$= 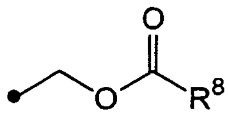 $Q^5$= 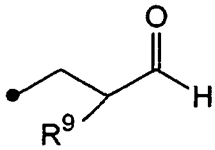

wherein, $R^4$ represents hydrogen, a C1-C7 alkyl group optionally substituted by halogen or a C3-C7 cycloalkyl group optionally substituted by one or more members selected from Group α,
$R^5$ and $R^6$ are the same or mutually different and represent a C1-C7 alkyl group optionally substituted by halogen, a C3-C7 alkenyl group optionally substituted by halogen, a C1-C7 alkoxy group optionally substituted by halogen or a C3-C7 cycloalkyl group optionally substituted by one or more members selected from Group α, alternatively $R^5$, $R^6$ and the nitrogen atom to which they are bonding represent a pyrrolidin-1-yl group optionally substituted by one or more members selected from Group α, a piperidino group optionally substituted by one or more members selected from Group α, a hexamethyleneimin-1-yl group optionally substituted by one or more members selected from Group α, a morpholino group optionally substituted by one or more members selected from Group α or a thiomorpholin-4-yl group optionally substituted by one or more members selected from Group α,
$R^7$ represents a C1-C7 alkyl group optionally substituted by halogen, a phenyl group optionally substituted by one or more members selected from Group β, a benzyl group optionally substituted by one or more members selected from Group β or a C3-C7 cycloalkyl group optionally substituted by one or more members selected from Group α,
$R^8$ represents a C1-C7 alkyl group optionally substituted by halogen, a phenyl group optionally substituted by one or more members selected from Group β or a C3-C7 cycloalkyl group optionally substituted by one or more members selected from Group α,
$R^9$ represents a C1-C7 alkyl group optionally substituted by halogen, or hydrogen;
$R^3$ represents a C1-C7 alkyl group optionally substituted by halogen, a C1-C7 alkoxy group optionally substituted by halogen, a C3-C7 cycloalkyl group optionally substituted by one or more members selected from Group α, a C3-C7 cycloalkyloxy group optionally substituted by one or more members selected from Group α, or halogen, and
n represents an integer of 0 to 3;
Group α: a group consisting of halogen, C1-C7 alkyl groups and C1-C7 haloalkyl groups.
Group β: a group consisting of halogen, a cyano group, a nitro group, C1-C7 alkyl groups, C1-C7 haloalkyl groups, C1-C7 alkoxy groups and C1-C7 haloalkoxy groups (hereinafter, referred to as the inventive compound);
[2] The pyridine compound according to [1], wherein at least one $R^1$ is a C1-C7 haloalkyl group or a C1-C7 haloalkoxyl group;
[3] The pyridine compound according to [1], wherein at least one $R^1$ is a C1-C3 fluoroalkyl group or a C1-C3 fluoroalkoxy group;
[4] The pyridine compound according to [2] or [3], wherein $R^1$ is a substituent at the 2-position or 3-position on a benzene ring;
[5] The pyridine compound according to any one of [1] to [4], wherein n is 0;
[6] The pyridine compound according to any one of [1] to [5], wherein $R^2$ is hydrogen;
[7] A pesticidal composition comprising as an active ingredient the pyridine compound according to any one of [1] to [6];
[8] A method of controlling pest comprising applying an effective amount of the pyridine compound according to any one of [1] to [6] to a pest or a place where a pest inhabits.

Effect of the Invention

The inventive compound is useful as an active ingredient of a pesticidal composition since the compound has an excellent pest control effect.

Modes for Carrying Out the Invention

In the present invention, the halogen atom means a fluorine atom, a chlorine atom, a bromine atom or an iodine atom.
In the present invention, examples of the "C1-C7 haloalkyl group" include a fluoromethyl group, chloromethyl group, bromomethyl group, difluoromethyl group, dichloromethyl group, dibromomethyl group, trifluoromethyl group, trichloromethyl group, dichlorofluoromethyl group, chlorodifluoromethyl group, trifluoromethyl group, difluoromethyl group, 2,2,2-trifluoroethyl group, 1,1,2,2,2-pentafluoroethyl group, 3,3,3-trifluoropropyl group, 2,2,3,3,3-pentafluoropropyl group, heptafluoropropyl group, 1-methyl-2,2,2-trifluoroethyl group, 1-trifluoromethyl-2,2,2-trifluoroethyl group and heptafluoroisopropyl group.
In the present invention, examples of the "C1-C7 haloalkoxyl group" include a fluoromethoxy group, chloromethoxy group, bromomethoxy group, difluoromethoxy group, dichloromethoxy group, dibromomethoxy group, trifluoromethoxy group, trichloromethoxy group, dichlorofluoromethoxy group, chlorodifluoromethoxy group, trifluoromethoxy group, difluoromethoxy group, difluorochloromethoxy group, difluorobromomethoxy group, 2,2,2-trifluoroethoxy group, pentafluoroethoxy group, 3,3,3,2,2-pentafluoropropoxy group, 1-methyl-2,2,2-trifluoroethoxy group and 1-trifluoromethyl-2,2,2-trifluoroethoxy group.

In the present invention, the "C1-C7 alkyl group optionally substituted by halogen " includes C1-C7 alkyl groups and the above-described C1-C7 haloalkyl groups.

Examples of the above-described C1-C7 alkyl group include a methyl group, ethyl group, propyl group, 2-methylpropyl group, 1-methylpropyl group, 1,1-dimethylethyl group, 3-methylbutyl group, 2,2-dimethylpropyl group, 1,1-dimethylpropyl group, hexyl group, 4-methylpentyl group, 3-methylpentyl group, 1,3-dimethylbutyl group, heptyl group and 1-ethyl-1-methylbutyl group.

In the present invention, the "C1-C7 alkoxy group optionally substituted by halogen " includes C1-C7 alkoxy groups and the above-described C1-C7 haloalkoxyl groups. Examples of the above-described C1-C7 alkoxy group include a methoxy group, ethoxy group, propoxy group, isopropoxy group, hexyloxy group, 5-methylpentyloxy group, 3-methylpentyloxy group, 1,3-dimethylbutoxy group, heptyloxy group and 1-ethyl-1-methylbutoxy group.

In the present invention, examples of the "C1-C7 alkylthio group optionally substituted by halogen " include C1-C7 alkylthio groups such as a methylthio group, ethylthio group, propylthio group, 2-methylpropylthio group, 1-methylpropylthio group, 1,1-dimethylethylthio group, 3-methylbutylthio group, 2,2-dimethylpropylthio group, 1,1-dimethylpropylthio group, hexylthio group, 4-methylpentylthio group, 3-methylpentylthio group, 1,3-dimethylbutylthio group and the like; C1-C7 haloalkylthio groups such as a trifluoromethylthio group, trichloromethylthio group, difluoromethylthio group, 2,2,2-trifluoroethylthio group, 1,1,2,2,2-pentafluoroethylthio group, 3,3,3-trifluoropropylthio group, 2,2,3,3,3-pentafluoropropylthio group, heptafluoropropylthio group, 1-methyl-2,2,2-trifluoroethylthio group, 1-trifluoromethyl-2,2,2-trifluoroethylthio group, heptafluoroisopropylthio group and the like.

In the present invention, examples of the "C3-C7 alkenyl group optionally substituted by halogen " include C3-C7 alkenyl groups such as a 2-propenyl group, 3-butenyl group, 1-methyl-2-propenyl group, 2-methyl-2-propenyl group, 2-pentenyl group, 1-methyl-2-butenyl group, 3-methyl-3-butenyl group, 1-ethyl-2-propenyl group, 2-hexenyl group, 2-methyl-2-pentenyl group, 3-methyl-2-pentenyl group, 4-methyl-2-pentenyl group, 1-methyl-3-pentenyl group, 4-methyl-3-pentenyl group, 1-methyl-4-pentenyl group, 4-methyl-4-pentenyl group and the like; C3-C7 haloalkenyl groups such as a 3-chloro-2-propenyl group, 3,3-dichloro-2-propenyl group, 4,4-dichloro-3-butenyl group, 2-chloro-2-propenyl group and the like.

In the present invention, examples of the "C3-C7 alkynyl group optionally substituted by halogen" include C3-C7 alkynyl groups such as a 2-propynyl group, 2-butynyl group, 3-butynyl group and the like; C3-C7 haloalkynyl groups such as a 4-chlorobutynyl group, 4,4,4-trifluoro-2-butynyl group, 1-(trifluoromethyl)-2-butynyl group, 1-(trifluoromethyl)-2-propynyl group and the like.

In the present invention, examples of the "C2-C7 alkoxyalkyl group" include a methoxymethyl group, ethoxymethyl group, 2-methoxyethyl group, 2-ethoxyethyl group and the like.

In the present invention, examples of the "(C3-C7 cycloalkyl)methyl group optionally substituted by one or more members selected from Group α" include a (cyclopropyl)methyl group, (1-methylcyclopropyl)methyl group, (2,2-dimethylcyclopropyl)methyl group, (cyclopentyl)methyl group, cyclohexylmethyl group and the like.

In the present invention, examples of the "benzyl group optionally substituted by one or more members selected from Group β" include a benzyl group, 1-phenylethyl group, 2-chlorobenzyl group, 3-chlorobenzyl group, 4-chlorobenzyl group, 3-bromobenzyl group, 4-bromobenzyl group, 2-fluorobenzyl group, 3-fluorobenzyl group, 2-cyanobenzyl group, 3-cyanobenzyl group, 4-cyanobenzyl group, 2-nitrobenzyl group, 3-nitrobenzyl group, 4-nitrobenzyl group, 2-methylbenzyl group, 3-methylbenzyl group, 4-methylbenzyl group, 2-(trifluoromethyl)benzyl group, 3-(trifluoromethyl)benzyl group, 4-(trifluoromethyl)benzyl group, 2-methoxybenzyl group, 3-methoxybenzyl group, 4-methoxybenzyl group and the like.

In the present invention, examples of the "C3-C7 cycloalkyl group optionally substituted by one or more members selected from Group α" include a cyclopropyl group, 1-methylcyclopropyl group, 2-methylcyclopropyl group, 2,2-dimethylcyclopropyl group, 2-fluorocyclopropyl group, cyclobutyl group, 1-trifluoromethylcyclobutyl group, cyclopentyl group, 2-methylcyclopentyl group, cyclohexyl group, 1-methylcyclohexyl group, 2-methylcyclohexyl group, 3-methylcyclohexyl group, 4-methylcyclohexyl group, 4-trifluoromethylcyclohexyl group, 2-fluorohexyl group, 3-fluorohexyl group, 4-fluorocyclohexyl group and cycloheptyl group.

In the present invention, examples of the "pyrrolidin-1-yl group optionally substituted by one or more members selected from Group α" include a pyrrolidin-1-yl group, 2-methylpyrrolidin-1-yl group and 3,5-dimethylpyrrolidin-1-yl group.

In the present invention, examples of the "piperidino group optionally substituted by one or more members selected from Group α" include a piperidino group, 2-methylpiperidino group, 3-methylpiperidino group, 3,5-dimethylpiperidino group and 4-tert-butylpiperidino group.

In the present invention, examples of the "hexamethyleneimin-1-yl group optionally substituted by one or more

members selected from Group α" include a hexamethyleneimin-1-yl group.

In the present invention, examples of the "morpholino group optionally substituted by one or more members selected from Group α" include a morpholino group and 3, 5-dimethyl morpholino group.

In the present invention, examples of the "thiomorpholin-4-yl group optionally substituted by one or more members selected from Group α" include a thiomorpholin-4-yl group.

In the present invention, examples of the "phenyl group optionally substituted by one or more members selected from Group β" include a phenyl group, 2-chlorophenyl group, 3-chlorophenyl group, 4-chlorophenyl group, 3-fluorophenyl group, 4-fluorophenyl group, 3-bromophenyl group, 3-iodophenyl group, 2-cyanophenyl group, 3-cyanophenyl group, 4-cyanophenyl group, 2-nitrophenyl group, 3-nitrophenyl group, 4-nitrophenyl group, 2-methylphenyl group, 3-methylphenyl group, 4-methylphenyl group, 2-(trifluoromethyl)phenyl group, 3-(trifluoromethyl)phenyl group, 4-(trifluoromethyl) phenyl group, 2-methoxyphenyl group, 3-methoxyphenyl group, 4-methoxyphenyl group, 3-(trifluoromethoxy)phenyl group, 4-(trifluoromethoxy)phenyl group, 3-t-butylphenyl group, 2,4-dichlorophenyl group, 2,4-difluorophenyl group, 2,3-dichlorophenyl group, 2,3-difluorophenyl group, 3,4-dichlorophenyl group, 3,4-difluorophenyl group, 2,4,6-trifluorophenyl group and 2,4,6-trichlorophenyl group.

In the present invention, examples of the "C3-C7 cycloalkyloxy group optionally substituted by one or more members selected from Group α" include a cyclopropyloxy group, cyclobutyloxy group, cyclopentyloxy group and cyclohexyloxy group.

In the present invention, examples of the "C1-C3 fluoroalkyl group" include a trifluoromethyl group, difluoromethyl group, 2,2,2-trifluoroethyl group, 1,1,2,2,2-pentafluoroethyl group, 3,3,3-trifluoropropyl group, 2,2,3,3,3-pentafluoropropyl group, heptafluoropropyl group, 1-methyl-2,2,2-trifluoroethyl group, 1-trifluoromethyl-2,2,2-trifluoroethyl group and heptafluoroisopropyl group.

In the present invention, examples of the "C1-C3 fluoroalkoxy group" include a fluoromethoxy group, difluoromethoxy group, trifluoromethoxy group, 2,2,2-trifluoroethoxy group, pentafluoroethoxy group, 3,3,3,2,2-pentafluoropropyloxy group, 1-methyl-2.2,2-trifluoroethoxy group and 1-trifluoromethyl-2,2,2-trifluoroethoxy group.

Examples of embodiments of the inventive compound include the following pyridine compounds.

Pyridine compounds represented by general formula (I) in which at least one $R^1$ represents a C1-C7 haloalkyl group or C1-C7 haloalkoxyl group.

Pyridine compounds represented by general formula (I) in which at least one $R^1$ represents a C1-C3 fluoroalkyl group or C1-C3 fluoroalkoxy group.

Pyridine compounds represented by general formula (I) in which m represents 1 and $R^1$ represents a C1-C7 haloalkyl group or C1-C7 haloalkoxyl group.

Pyridine compounds represented by general formula (I) in which m represents 1 and $R^1$ represents a C1-C3 fluoroalkyl group or C1-C3 fluoroalkoxy group.

Pyridine compounds represented by general formula (I) in which n represents 0.

Pyridine compounds represented by general formula (I) in which $R^2$ represents hydrogen.

Pyridine compounds represented by general formula (I) in which $R^2$ represents any one group selected from $Q^1$, $Q^2$, $Q^3$, $Q^4$ and $Q^5$.

Pyridine compounds represented by general formula (I) in which $R^2$ represents $Q^1$.

Pyridine compounds represented by general formula (I) in which $R^2$ represents $Q^2$.

Pyridine compounds represented by general formula (I) in which $R^2$ represents $Q^3$.

Pyridine compounds represented by general formula (I) in which $R^2$ represents $Q^4$.

Pyridine compounds represented by general formula (I) in which $R^2$ represents $Q^5$.

Pyridine compounds represented by general formula (I) in which at least one $R^1$ represents a C1-C3 fluoroalkyl group and $R^2$ represents hydrogen.

Pyridine compounds represented by general formula (I) in which at least one $R^1$ represents a C1-C3 fluoroalkyl group and $R^2$ represents $Q^1$.

Pyridine compounds represented by general formula (I) in which at least one $R^1$ represents a C1-C3 fluoroalkyl group and $R^2$ represents $Q^2$.

Pyridine compounds represented by general formula (I) in which at least one $R^1$ represents a C1-C3 fluoroalkyl group and $R^2$ represents $Q^3$.

Pyridine compounds represented by general formula (I) in which at least one $R^1$ represents a C1-C3 fluoroalkyl group and $R^2$ represents $Q^4$.

Pyridine compounds represented by general formula (I) in which at least one $R^1$ represents a C1-C3 fluoroalkyl group and $R^2$ represents $Q^5$.

Pyridine compounds represented by general formula (I) in which at least one $R^1$ represents a C1-C3 fluoroalkoxy group and $R^2$ represents hydrogen.

Pyridine compounds represented by general formula (I) in which at least one $R^1$ represents a C1-C3 fluoroalkoxy group and $R^2$ represents $Q^1$.

Pyridine compounds represented by general formula (I) in which at least one $R^1$ represents a C1-C3 fluoroalkoxy group and $R^2$ represents $Q^2$.

Pyridine compounds represented by general formula (I) in which at least one $R^1$ represents a C1-C3 fluoroalkoxy group and $R^2$ represents $Q^3$.

Pyridine compounds represented by general formula (I) in which at least one $R^1$ represents a C1-C3 fluoroalkoxy group and $R^2$ represents $Q^4$.

Pyridine compounds represented by general formula (I) in which at least one $R^1$ represents a C1-C3 fluoroalkoxy group and $R^2$ represents $Q^5$.

Pyridine compounds represented by general formula (I) in which $R^1$ represents a C1-C3 fluoroalkyl group, C1-C3 fluoroalkoxy group, C1-C3 alkoxy group or halogen, $R^2$ represents hydrogen, n=0, m=1 or 2, and A represents an oxygen atom or single bond.

Pyridine compounds represented by general formula (I) in which at least one $R^1$ is situated at the 2-position or 3-position on a benzene ring.

Pyridine compounds represented by general formula (I) in which $R^1$ is situated at the 2-position or 3-position on a benzene ring and $R^2$ represents hydrogen.

Pyridine compounds represented by general formula (I) in which $R^1$ is situated at the 2-position or 3-position on a benzene ring and $R^2$ represents $Q^1$.

Pyridine compounds represented by general formula (I) in which $R^1$ is situated at the 2-position or 3-position on a benzene ring and $R^2$ represents $Q^2$.

Pyridine compounds represented by general formula (I) in which m represents 1, $R^1$ is situated at the 2-position or 3-position on a benzene ring and $R^2$ represents $Q^3$.

Pyridine compounds represented by general formula (I) in which m represents 1, $R^1$ is situated at the 2-position or 3-position on a benzene ring and $R^2$ represents $Q^4$.

Pyridine compounds represented by general formula (I) in which m represents 1, $R^1$ is situated at the 2-position or 3-position on a benzene ring and $R^2$ represents $Q^5$.

Pyridine compounds represented by general formula (I) in which m represents 1, $R^1$ is situated at the 2-position or 3-position on a benzene ring, A represents a single bond and $R^2$ represents hydrogen.

Pyridine compounds represented by general formula (I) in which m represents 1, $R^1$ is situated at the 2-position or 3-position on a benzene ring, A represents a single bond and $R^2$ represents $Q^1$.

Pyridine compounds represented by general formula (I) in which m represents 1, $R^1$ is situated at the 2-position or 3-position on a benzene ring, A represents a single bond and $R^2$ represents $Q^2$.

Pyridine compounds represented by general formula (I) in which m represents 1, $R^1$ is situated at the 2-position or 3-position on a benzene ring, A represents a single bond and $R^2$ represents $Q^3$.

Pyridine compounds represented by general formula (I) in which m represents 1, $R^1$ is situated at the 2-position or 3-position on a benzene ring, A represents a single bond and $R^2$ represents $Q^4$.

Pyridine compounds represented by general formula (I) in which m represents 1, $R^1$ is situated at the 2-position or 3-position on a benzene ring, A represents a single bond and $R^2$ represents $Q^5$.

Next, the method for producing the inventive compound will be described.

The inventive compound can be produced, for example, by the following Production Method I.

Production Method I

Among the inventive compounds, a compound represented by the following formula (1-0) can be produced via the following steps (I-1) and (I-2).

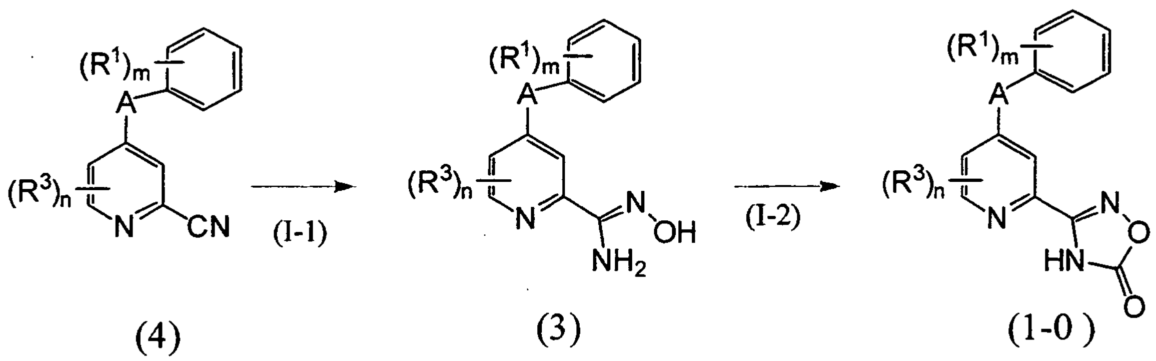


(4) (3) (1-0)

[in each formula, $R^1$, $R^3$, A, m and n represent the same meaning as described above.]

Step (I-1): a step of reacting a nitrile compound (4) and hydroxylamine in the presence of a base having a metal compound to produce a compound represented by general formula (3).

The above-described reaction is usually carried out in a solvent. Examples of the solvent include alcohols such as methanol, ethanol, 2-propanol and the like, water and mixtures thereof, and the like.

Examples of the above-described base include alkali metal compounds such as sodium hydride and the like, carbonates such as potassium carbonate and the like; etc. The amount of the above-described base is usually 1 to 4 mol with respect to 1 mol of the nitrile compound (4).

The above-described hydroxylamine includes hydroxylamine, hydroxylamine hydrochloride, hydroxylamine sulfate and the like. The amount of the above-described hydroxylamine is usually 1 to 3 mol with respect to 1 mol of a compound represented by general formula (4). The reaction can be usually carried out in the range of 0 to 120°C. The reaction time is usually in the range of 0.1 to 48 hours.

The reaction mixture after completion of the reaction can be subjected to general post treatments such as extraction with an organic solvent, concentration and the like, to isolate a compound represented by general formula (3). The isolated compound represented by general formula (3) can be further purified by re-crystallization, chromatography and the like.

Step (I-2): a step of reacting a pyridine compound represented by general formula (3) and a carbonylating agent in the presence of a base to produce a compound represented by general formula (1-0).

The reaction is usually carried out in a solvent. Examples of the solvent include ethers such as 1, 4-dioxane, diethyl ether, tetrahydrofuran, tert-butyl methyl ether and the like; halogenated hydrocarbons such as dichloromethane, chloroform, carbon tetrachloride, 1,2-dichloroethane, chlorobenzene and the like; hydrocarbons such as toluene, benzene, xylene and the like; nitriles such as acetonitrile and the like; aprotic polar solvents such as N,N-dimethylformamide, N-methylpyrrolidone, dimethyl sulfoxide and the like; and mixtures thereof, and the like.

Examples of the above-described base include nitrogen-containing heterocyclic compounds such as pyridine, picoline, 2,6-lutidine, 1,8-diazabicyclo[5,4,0]7-undecene, 1,5-diazabicyclo[4,3,0]5-nonene and the like; tertiary amines such as triethylamine, N,N-diisopropylethylamine and the like; etc. The amount of the above-described base is usually 1 to 3 mol with respect to 1 mol of a pyridine compound represented by general formula (3).

The above-described carbonylating agent includes phosgene, 1,1'-carbonyldiimidazole and the like. The amount of the above-described carbonylating agent is usually 1 to 3 mol with respect to 1 mol of a pyridine compound represented by general formula (3).

The reaction can be usually carried out in the range of 0 to 100°C. The reaction time is usually in the range of 0.1 to 48 hours.

The reaction mixture after completion of the reaction can be subjected to usual post treatments such as extraction with an organic solvent, concentration and the like to isolate a compound represented by general formula (1-0). The isolated compound represented by general formula (1-0) can be further purified by re-crystallization, chromatography and the like.

The inventive compound can be obtained as a compound having a desired substituent by the following steps a and b.

Step a: a step of reacting a compound represented by general formula (1-0) and a compound represented by general formula (12) in the presence of a base.

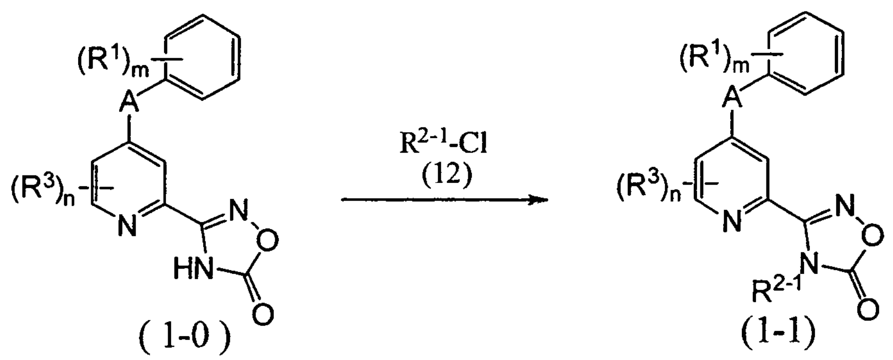


[in each formula, $R^1$, $R^3$, A, m and n represent the same meaning as described above, and $R^{2-1}$ represents a group other than hydrogen among groups represented by $R^2$.]

The reaction is usually carried out in a solvent. Examples of the solvent include ethers such as 1,4-dioxane, diethyl ether, tetrahydrofuran, tert-butyl methyl ether and the like, hydrocarbons such as toluene, benzene, xylene and

the like, nitriles such as acetonitrile and the like, aprotic polar solvents such as N,N-dimethylformamide, N-methylpyrrolidone, dimethyl sulfoxide and the like, nitrogen-containing heterocyclic compounds such as pyridine, picoline, 2, 6-lutidine and the like, and mixtures thereof, and the like.

Examples of the above-described base include alkali metal compounds such as sodium hydride and the like, carbonates such as potassium carbonate and the like, nitrogen-containing heterocyclic compounds such as 1,8-diazabicyclo[5,4,0]7-undecene, 1,5-diazabicyclo[4,3,0]5-nonene and the like, tertiary amines such as triethylamine, N,N-diisopropylethylamine and the like; etc, and the base can be appropriately selected depending on the solvent to be used in the reaction.

The amount of the above-described base is usually 1 to 3 mol with respect to 1 mol of a compound represented by general formula (1-0).

The amount of a compound represented by the above-described general formula (12) is usually 1 to 3 mol with respect to 1 mol of a compound represented by general formula (1-0).

The reaction can be usually carried out in the range of 0 to 120°C. The reaction time is usually in the range of 0.1 to 36 hours.

The reaction mixture after completion of the reaction can be subjected to usual post treatments such as extraction with an organic solvent, concentration and the like to isolate an inventive compound represented by general formula (1-1). The isolated inventive compound represented by general formula (1-1) can be further purified by re-crystallization, chromatography and the like.

Step b: a step of reacting a compound represented by general formula (2) and an aldehyde compound represented by general formula (13) in the presence of a base.

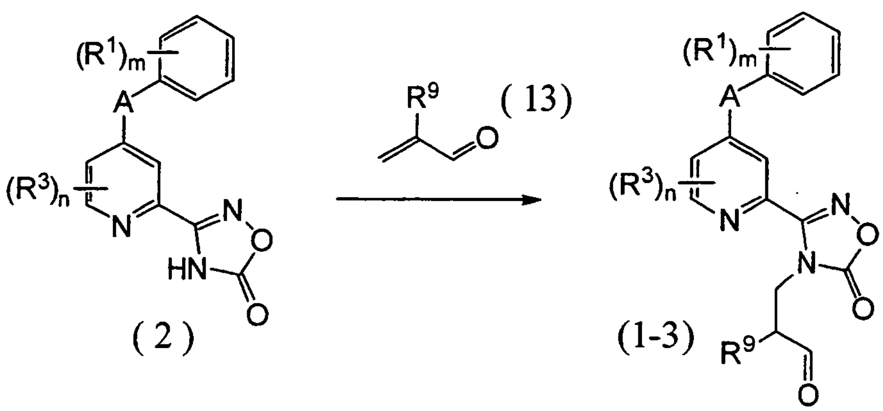

[in each formula, $R^1$, $R^3$, $R^9$, A, m and n represent the same meaning as described above.]

The above-described reaction is usually carried out in a solvent. Examples of the solvent include alcohols such as methanol, ethanol and the like; halogenated hydrocarbons such as chloroform, dichloromethane and the like; and mixtures thereof, and the like.

Examples of the above-described base include nitrogen-containing heterocyclic compounds such as pyridine, picoline, 2,6-lutidine, 1,8-diazabicyclo[5,4,0]7-undecene, 1,5-diazabicyclo[4,3,0]5-nonene and the like, tertiary amines such as triethylamine, N,N-diisopropylethylamine and the like; etc. The amount of the base is usually 1 to 3 mol with respect to 1 mol of a compound represented by general formula (2).

The amount of the above-described aldehyde compound represented by general formula (13) is usually 1 to 3 mol with respect to 1 mol of a compound represented by general formula (2).

The above-described reaction can be carried out usually in the range of 0 to 100°C. The reaction time is usually in the range of 0.1 to 48 hours.

The reaction mixture after completion of the reaction can be subjected to usual post treatments such as extraction with an organic solvent, concentration and the like to isolate a compound represented by general formula (1-3). The isolated compound represented by general formula (1-3) can be further purified by re-crystallization, chromatography and the like.

Next, a method for producing a nitrile compound represented by general formula (4) (hereinafter, this compound is referred to as "nitrile compound (4)"), among production intermediates of inventive compounds, will be described.

Reference Production Method A

The nitrile compound (4) can be produced via the following step (A-1) and step (A-2).

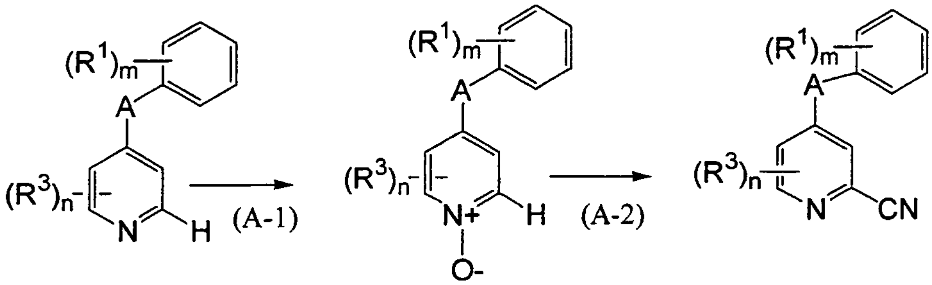

(6) (5) (4)

[in each formula, $R^1$, $R^3$, A, m and n represent the same meaning as described above.]

Step (A-1): a step of reacting a pyridine compound represented by general formula (6) and a peroxide to produce a compound represented by general formula (5).

The reaction is usually carried out in a solvent. Examples of the solvent include halogenated hydrocarbons such as dichloromethane, chloroform, carbon tetrachloride, 1,2-dichloroethane, chlorobenzene and the like, and mixtures thereof.

The above-described peroxide includes m-chloroperbenzoic acid, hydrogen peroxide water, peracetic acid and the like. The amount of the above-described peroxide is usually 1 to 3 mol with respect to 1 mol of a pyridine compound represented by general formula (6).

The reaction temperature of the reaction can be usually carried out in the range of 0 to 100°C. The reaction time is usually in the range of 0.1 to 72 hours.

The reaction mixture after completion of the reaction can be subjected to usual post treatments such as extraction with an organic solvent, concentration and the like to isolate a compound represented by general formula (5). The isolated compound represented by general formula (5) can be further purified by chromatography and the like.

Step (A-2): a step of reacting a compound represented by general formula (5) and a cyanating agent in the presence of a base to produce a nitrile compound (4).

The reaction is usually carried out in a solvent. Examples of the solvent include ethers such as 1, 4-dioxane, diethyl ether, tetrahydrofuran, tert-butyl methyl ether and the like; hydrocarbons such as toluene, benzene, xylene and the like; nitriles such as acetonitrile and the like, aprotic polar solvents such as N,N-dimethylformamide, N-methylpyrrolidone, dimethyl sulfoxide and the like, and mixtures thereof, and the like.

Examples of the above-described base include nitrogen-containing heterocyclic compounds such as pyridine, picoline, 2,6-lutidine, 1,8-diazabicyclo[5,4,0]7-undecene, 1,5-diazabicyclo[4,3,0]5-nonene and the like, tertiary amines such as triethylamine, N,N-diisopropylethylamine and the like; etc. The amount of the above-described base is usually 2 to 6 mol with respect to 1 mol of a compound represented by general formula (5).

The above-described cyanating agent includes trimethylsilyl cyanide and the like. The amount of the above-described cyanating agent is usually 2 to 6 mol with respect to 1 mol of a compound represented by general formula (5).

The reaction can be usually carried out in the range of 0 to 120°C. The reaction time is usually in the range of 0.1 to 72 hours.

The reaction mixture after completion of the reaction can be subjected to usual post treatments such as extraction with an organic solvent, concentration and the like to isolate a nitrile compound (4). The isolated nitrile compound (4) can be further purified by chromatography and the like.

Reference Production Method B

A nitrile compound represented by general formula (4-B), among nitrile compounds (4), can be produced via the following step (B).

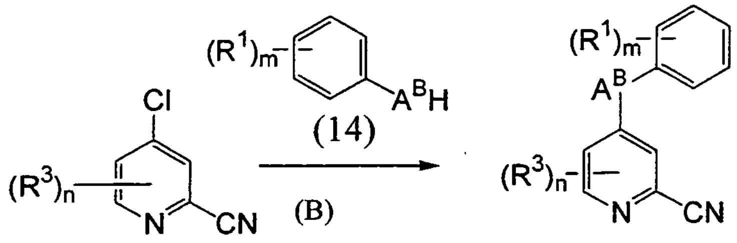


(8) (4-B)

[in the formulae (4-B), $A^B$ represents an oxygen atom, a sulfur atom or $NR^{10}$. In each formula, $R^1$, $R^3$, $R^{10}$, m and n represent the same meaning as described above.]

Step (B): a step of reacting a compound represented by general formula (8) and a compound represented by general formula (14) in the presence of a base to produce a nitrile compound represented by general formula (4-B).

The reaction is carried out usually in a solvent. Examples of the solvent include ethers such as 1,4-dioxane, diethyl ether, tetrahydrofuran, tert-butyl methyl ether and the like; hydrocarbons such as toluene, benzene, xylene and the like; nitriles such as acetonitrile and the like; aprotic polar solvents such as N,N-dimethylformamide, N-methylpyrrolidone, dimethyl sulfoxide and the like; and mixtures thereof, and the like.

Examples of the above-described base include alkali metal-containing compounds such as sodium hydride and the like, carbonates such as potassium carbonate and the like; etc. The amount of the above-described base is usually 1 to 3 mol with respect to 1 mol of the nitrile compound represented by general formula (8).

The amount of the above-described compound represented by general formula (14) is usually 1 to 3 mol with respect to 1 mol of the nitrile compound represented by general formula (8).

The reaction can be carried out usually in the range of 0 to 100°C. The reaction time is usually in the range of 0.1 to 12 hours.

The reaction mixture after completion of the reaction can be subjected to usual post treatments such as extraction with an organic solvent, concentration and the like to isolate the compound represented by general formula (4-B). The isolated compound represented by general formula (4-B) can be further purified by chromatography and the like.

Reference Production Method C

A nitrile compound represented by general formula (4-C), among nitrile compounds (4), can be produced via the following step (C-1), step (C-2) and step (C-3).

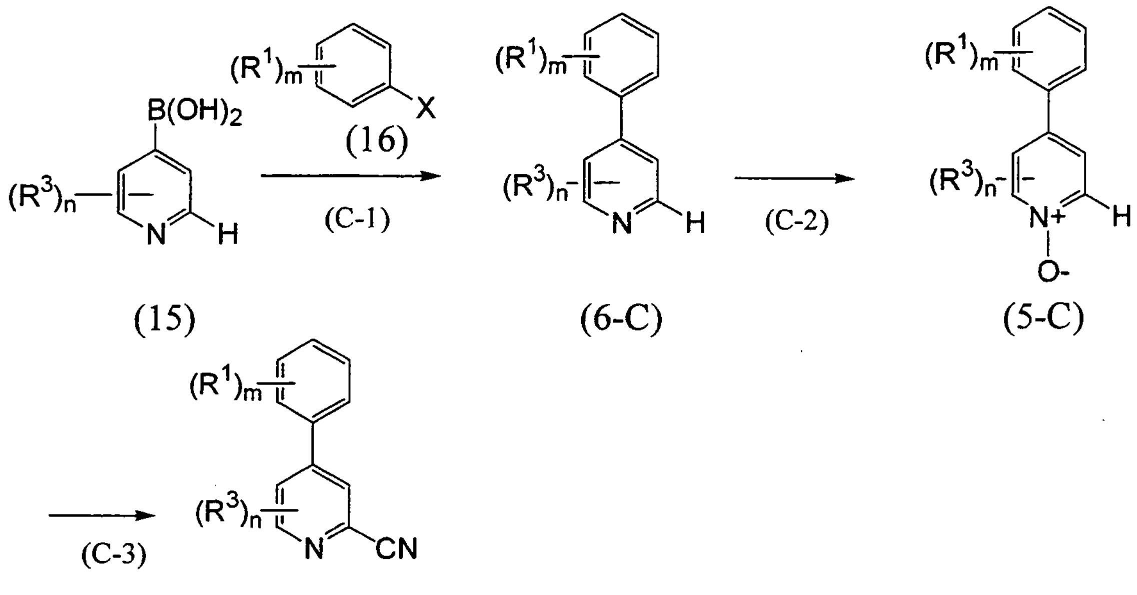


(15) (6-C) (5-C)

(4-C)

[in each formula, $R^1$, $R^3$, m and n represent the same meaning as described above, and X represents a chlorine atom, a bromine atom or an iodine atom.].

Step (C-1): a step of reacting a compound represented by general formula (16) and a pyridine compound represented by general formula (15) in the presence of a transition metal compound to produce a pyridine compound represented by general formula (6-C).

The reaction is carried out usually in a solvent. Examples of the solvent include water, ethers such as 1, 4-dioxane, diethyl ether, tetrahydrofuran, tert-butyl methyl ether and the like; hydrocarbons such as toluene, benzene, xylene and the like; nitriles such as acetonitrile and the like; aprotic polar solvents such as N,N-dimethylformamide, N-methylpyrrolidone, dimethyl sulfoxide and the like; and mixtures thereof.

The above-described transition metal compound includes, for example, palladium compounds, specifically, palladium acetate, tetrakis(triphenylphosphinepalladium), {1,1'-bis(diphenylphosphino)ferrocene}dichloropalladium (II)-methylene chloride complex and bis(triphenylphosphine)palladium(II) chloride. The amount of the above-described transition metal compound can be varied depending on reaction conditions and the like, and usually 0.01 to 0.1 mol with respect to 1 mol of the pyridine compound represented by general formula (15).

The amount of the compound represented by general formula (16) is usually 1 to 2 mol with respect to 1 mol of the pyridine compound represented by general formula (15).

The reaction can be carried out usually in the range of 0 to 150°C. The reaction time is usually in the range of 0.1 to 96 hours.

The reaction mixture after completion of the reaction can be subjected to usual post treatments such as extraction with an organic solvent, concentration and the like to isolate the compound represented by general formula (6-C). The isolated compound represented by general formula (6-C) can be further purified by chromatography and the like.

Step (C-2): a step of reacting a pyridine compound represented by general formula (6-C) and a peroxide to produce a compound represented by general formula (5-C).

The reaction is usually carried out in a solvent. Examples of the solvent include halogenated hydrocarbons such as dichloromethane, chloroform, carbon tetrachloride, 1,2-dichloroethane, chlorobenzene and the like, and mixtures thereof, and the like.

The above-described peroxide includes m-chloroperbenzoic acid, hydrogen peroxide water, peracetic acid and the like. The amount of the above-described peroxide is usually 1 to 3 mol with respect to 1 mol of the pyridine compound represented by general formula (6-C).

The reaction can be carried out usually in the range of 0 to 100°C. The reaction time is usually in the range of 0.1 to 72 hours.

The reaction mixture after completion of the reaction can be subjected to usual post treatments such as extraction with an organic solvent, concentration and the like to isolate the compound represented by general formula (5-C). The isolated compound represented by general formula (5-C) can be further purified by chromatography and the like.

Step (C-3): a step of reacting a compound represented by general formula (5-C) and a cyanating agent in the presence of a base to produce a nitrile compound represented by general formula (4-C).

The reaction is carried out usually in a solvent. Examples of the solvent include ethers such as 1,4-dioxane, diethyl ether, tetrahydrofuran, tert-butyl methyl ether and the like, hydrocarbons such as toluene, benzene, xylene and the like, nitriles such as acetonitrile and the like, aprotic polar solvents such as N,N-dimethylformamide, N-methylpyrrolidone, dimethyl sulfoxide and the like, and mixtures thereof, and the like.

Examples of the above-described base include nitrogen-containing heterocyclic compounds such as pyridine, picoline, 2,6-lutidine, 1,8-diazabicyclo[5,4,0]7-undecene, 1,5-diazabicyclo[4,3,0]5-nonene and the like; tertiary amines such as triethylamine, N,N-diisopropylethylamine and the like; etc. The amount of the above-described base is usually 2 to 6 mol with respect to 1 mol of the compound represented by general formula (5-C) .

The above-described cyanating agent includes trimethylsilyl cyanide and the like. The amount of the above-described cyanating agent is usually 2 to 6 mol with respect to 1 mol of the compound represented by general formula (5-C).

The reaction can be usually carried out in the range of 0 to 120°C. The reaction time is usually in the range of 0.1 to 72 hours.

The reaction mixture after completion of the reaction can be subjected to usual post treatments such as extraction with an organic solvent, concentration and the like to isolate the compound represented by general formula (4-C). The isolated compound represented by general formula (4-C) can be further purified by chromatography and the like.

Reference Production Method D

A nitrile compound represented by general formula (4-D), among nitrile compounds (4), can be produced via the following step (D).

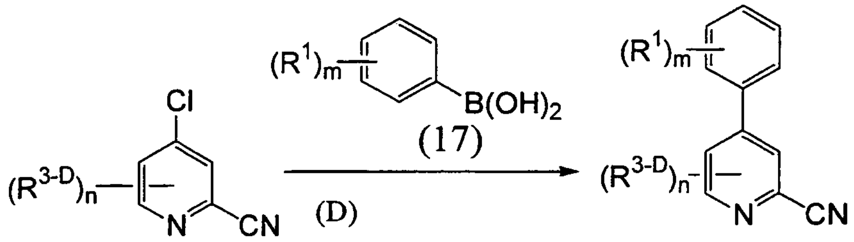


(8-D) (4-D)

[in each formula, $R^1$ m and n represent the same meaning as described above, and $R^{3-D}$ represents a fluorine atom, a chlorine atom, a C1-C7 alkyl group, a C1-C7 alkoxy group and a C3-C7 cycloalkyloxy group.].

Step (D): a step of reacting a compound represented by general formula (17) and a pyridine compound represented by general formula (8-D) in the presence of a transition metal compound to produce a nitrile compound represented by general formula (4-D).

The reaction is carried out usually in a solvent. Examples of the solvent include water, ethers such as 1, 4-dioxane, diethyl ether, tetrahydrofuran, tert-butyl methyl ether and the like; hydrocarbons such as toluene, benzene, xylene and the like; nitriles such as acetonitrile and the like; aprotic polar solvents such as N,N-dimethylformamide, N-methylpyrrolidone, dimethyl sulfoxide and the like; and mixtures thereof.

The above-described transition metal compound includes, for example, palladium compounds, specifically, palladium acetate, tetrakis(triphenylphosphinepalladium), {1,1'-bis(diphenylphosphino)ferrocene}dichloropalladium (II)-methylene chloride complex and bis(triphenylphosphine)palladium(II) chloride and the like. The amount of the above-described transition metal compound can be varied depending on reaction conditions and the like, and usually 0.01 to 0.1 mol with respect to 1 mol of the pyridine compound represented by general formula (8-D).

The amount of the compound represented by general formula (17) is usually 1 to 2 mol with respect to 1 mol of the pyridine compound represented by general formula (8-D).

The reaction can be carried out usually in the range of 0 to 200°C. The reaction time is usually in the range of 0.1 to 96 hours.

The reaction mixture after completion of the reaction can be subjected to usual post treatments such as extraction with an organic solvent, concentration and the like to isolate the compound represented by general formula (4-D). The isolated compound represented by general formula (4-D) can be further purified by chromatography and the like.

Reference Production Method E

A nitrile compound represented by general formula (4-E), among nitrile compounds (4), can be produced via the following step (E).

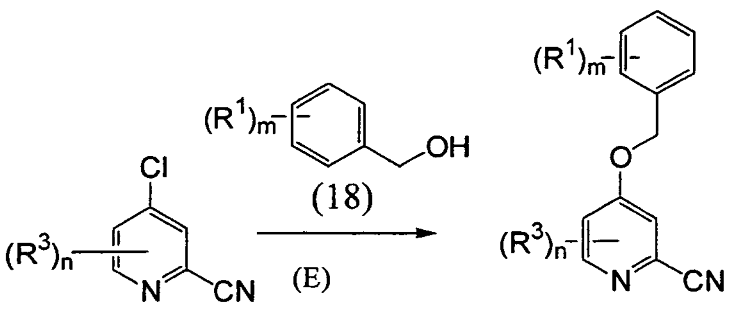


(8) (4-E)

[in each formula, $R^1$, $R^3$, m and n represent the same meaning as described above.].

Step (E): a step of reacting a compound represented by general formula (8) and a compound represented by general formula (18) in the presence of a base to produce a nitrile compound represented by general formula (4-E).

The above-described reaction is carried out usually in a solvent. Examples of the solvent include ethers such as 1,4-dioxane, diethyl ether, tetrahydrofuran, tert-butyl methyl ether and the like, hydrocarbons such as toluene, benzene,

xylene and the like, nitriles such as acetonitrile and the like, aprotic polar solvents such as N,N-dimethylformamide, N-methylpyrrolidone, dimethyl sulfoxide and the like, and mixtures thereof.

Examples of the above-described base include alkali metal-containing compounds such as sodium hydride and the like, carbonates such as potassium carbonate and the like; etc. The amount of the above-described base is usually 1 to 3 mol with respect to 1 mol of the nitrile compound represented by general formula (8).

The amount of the above-described compound represented by general formula (18) is usually 1 to 3 mol with respect to 1 mol of the nitrile compound represented by general formula (8).

The reaction can be carried out usually in the range of 0 to 100°C. The reaction time is usually in the range of 0.1 to 12 hours.

The reaction mixture after completion of the reaction can be subjected to usual post treatments such as extraction with an organic solvent, concentration and the like to isolate the compound represented by general formula (4-E). The isolated compound represented by general formula (4-E) can be further purified by chromatography and the like.

Reference Production Method F

A compound represented by general formula (20), among compounds represented by general formula (12) in the step a in the general production example, can be produced via the following step (F) .

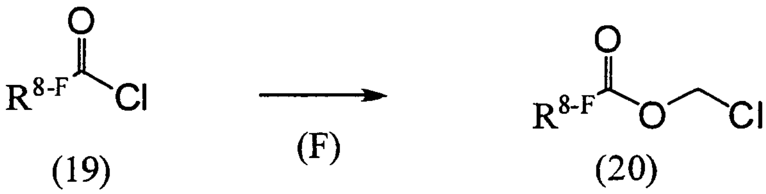

[in each formula, $R^{8-F}$ represents a phenyl group optionally substituted by one or more members selected from Group β.]. [0148]

Step (F): a step of reacting a compound represented by general formula (19), zirconium tetrachloride and trioxane to produce a compound represented by general formula (20).

The above-described reaction is in general carried out under an atmosphere of a gas inactive on the reaction such as nitrogen, argon and the like.

The above-described reaction is carried out usually in a solvent. Examples of the solvent include ethers such as 1,4-dioxane, diethyl ether, tetrahydrofuran, tert-butyl methyl ether and the like; halogenated hydrocarbons such as dichloromethane, chloroform, carbon tetrachloride, 1,2-dichloroethane, chlorobenzene and the like; and mixtures thereof, and the like.

The amount of the above-described zirconium tetrachloride is usually 0.9 to 2 mol with respect to 1 mol of the compound represented by general formula (19).

The amount of the above-described trioxane is usually 0.3 to 1 mol with respect to 1 mol of the compound represented by general formula (19).

The reaction can be carried out usually in the range of -20 to 80°C. The reaction time is usually in the range of 0.1 to 72 hours.

The reaction mixture after completion of the reaction can be subjected to usual post treatments such as extraction with an organic solvent, concentration and the like to isolate the compound represented by general formula (20). The isolated compound represented by general formula (20) can be further purified by chromatography and the like.

The compound represented by general formula (17) is a known compound, or can be produced according to known methods (for example, Organic Letters, 2006, 581-584 or Journal of Organic Chemistry, 1995, 60, 3020-3027).

The pyridine compound represented by general formula (15) is a known compound, or can be produced according to known methods (for example, Tetrahedron, 2002, 58, 4369-4373).

The compound represented by general formula (16) is a known compound, or can be produced according to known methods (for example, Journal of Organic Chemistry, 1987, 52, 748-753).

Next, specific examples of the inventive compound will be shown below.

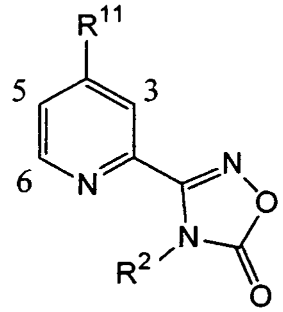

(1-a)

Compounds represented by general formula (1-a) in which $R^2$ represents hydrogen and $R^{11}$ represents any group shown in the following Table 1;

Table 1

| | | | |
|---|---|---|---|
| 2-FPh | 3-FPh | 2-ClPh | 3-ClPh |
| 2,3-diFPh | 2,4-diFPh | 2,5-diFPh | 2,6-diFPh |
| 2-$CF_3$Ph | 3-$CF_3$Ph | 4-$CF_3$Ph | 2-$CF_3CF_2$Ph |
| 3-$CF_3CF_2$Ph | 4-$CF_3CF_2$Ph | 2-($CF_3$)2CFPh | 3-$(CF_3)_2$CFPh |
| 4-$(CF_3)_2$CFPh | 2-$CF_3$OPh | 3-$CF_3$OPh | 4-$CF_3$OPh |
| 2-$CF_3CF_2$OPh | 3-$CF_3CF_2$OPh | 4-$CF_3CF_2$OPh | 2-$CF_3CH_2$Ph |
| 3-$CF_3CH_2$Ph | 4-$CF_3CH_2$Ph | 2-$CF_3CH_2$OPh | 3-$CF_3CH_2$OPh |
| 4-$CF_3CH_3$OPh | 2-$CF_3$-3-F-Ph | 2-$CF_3$-4-F-Ph | 2-$CF_3$-5-F-Ph |
| 2-$CF_3$-6-F-Ph | 3-$CF_3$-2-F-Ph | 3-$CF_3$-4-F-Ph | 3-$CF_3$-5-F-Ph |
| 3-$CF_3$-6-F-Ph | 4-$CF_3$-2-F-Ph | 4-$CF_3$-3-F-Ph | 2-$CF_3$-3-Cl-Ph |
| 2-$CF_3$-4-Cl-Ph | 2-$CF_3$-5-Cl-Ph | 2-$CF_3$-6-Cl-Ph | 2-$CF_3$-3-$CH_3$-Ph |
| 2-$CF_3$-4-$CH_3$-Ph | 2-$CF_3$-5-$CH_3$-Ph | 2-$CF_3$-6-CH3-Ph | 3-$CF_3$-2-$CH_3$-Ph |
| 3-$CF_3$-4-$CH_3$-Ph | 3-$CF_3$-5-$CH_3$-Ph | 3-$CF_3$-6-CH3-Ph | 2,3-bis$CF_3$Ph |
| 2,4-bis$CF_3$Ph | 2,6-bis$CF_3$Ph | 3,5-bisCF3Ph | 2-$CF_3$-6-$CH_3$O-Ph |
| 2-$CF_3$-3-$CH_3$O-Ph | 2-$CF_3$-4-$CH_3$O-Ph | 2-$CF_3$-5-CH3O-Ph | 2-$CF_3$-6-CN-Ph |
| 2-$CF_3$-5-CN-Ph | 2-$CF_3$-3-CN-Ph | 3-$CF_3$-2-CN-Ph | 3-$CF_3$-6-CN-Ph |
| 3-$CF_3$-5-CN-Ph | 2-$CF_3$-6-BrPh | 2-$CF_3$-3-Br-Ph | 2-$CF_3$-5-Br-Ph |
| 3-$CF_3$-2-Br-Ph | 3-$CF_3$-6-Br-Ph | 3-$CF_3$-5-Br-Ph | 2-$CF_3$-6-I-Ph |
| 2-$CF_3$-3-I-Ph | 2-$CF_3$-5-I-Ph | 3-$CF_3$-2-I-Ph | 3-$CF_3$-6-I-Ph |
| 2-$CF_3PhCH_2O$ | 3-$CF_3PhCH_2O$ | 2-$CF_3CF_2PhCH_2O$ | 3-$CF_3CF_2PhCH_2O$ |
| 2-$(CF_3)_2CFPhCH_2$ O | 3-$(CF_3)_2CFPhCH_2$ O | 2-$CF_3OPhCH_2O$ | 3-$CF_3OPhCH_2O$ |
| 2-$CF_3CF_2OPhCH_2$ O | 3-$CF_3CF_2OPhCH_2$ O | 2-$CF_3CH_2PhCH_2O$ | 3-$CF_3CH_2PhCH_2O$ |
| 2-$CF_3CH_2OPhCH_2$ O | 3-$CF_3CH_2$OPhCH $_2$O | 2-$CF_3$-3-F-Ph$CH_2$O | 2-$CF_3$-5-F-Ph$CH_2$O |
| 2-$CF_3$-6-F-Ph$CH_2$O | 3-$CF_3$-2-F-Ph$CH_2$O | 3-$CF_3$-5-F-Ph$CH_2$O | 3-$CF_3$-6-F-Ph$CH_2$O |
| 2-$CF_3$-3-Cl-Ph$CH_2$ O | 2-$CF_3$-5-Cl-Ph$CH_2$ O | 2-$CF_3$-6-Cl-Ph$CH_2$O | 2-$CF_3$-3-$CH_3$-Ph$CH_2$ O |
| 2-$CF_3$-5-$CH_3$-PhC $H_2$O | 2-$CF_3$-6-$CH_3$-PhC $H_2$O | 3-$CF_3$-2-$CH_3$-Ph$CH_2$ O | 3-$CF_3$-5-$CH_3$-Ph$CH_2$ O |
| 3-$CF_3$-6-$CH_3$-PhC $H_2$O | 2,3-bis$CF_3PhCH_2$ O | 2,6-bis$CF_3PhCH_2O$ | 3,5-bis$CF_3PhCH_2O$ |
| 2-$CF_3$-6-$CH_3$O-Ph $CH_2$O | 2-$CF_3$PhO | 3-$CF_3$PhO | 2-$CF_3CF_2$PhO |
| 3-$CF_3CF_2$PhO | 2-$(CF_3)_2$CFPhO | 3-$(CF_3)_2$CFPhO | 2-$CF_3$OPhO |

(continued)

| | | | |
|---|---|---|---|
| 3-$CF_3$OPhO | 2-$CF_3CF_2$OPhO | 3-$CF_3CF_2$OPhO | 2-$CF_3CH_2$PhO |
| 2-$CF_3CH_2$OPhO | 3-$CF_3CH_2$OPhO | 2-$CF_3$-3-F-PhO | 3-$CF_3CH_2$PhO |
| 2-$CF_3$-5-F-PhO | 2-$CF_3$-6-F-PhO | 3-$CF_3$-2-F-PhO | 3-$CF_3$-5-F-PhO |
| 3-$CF_3$-6-F-PhO | 2-$CF_3$-3-Cl-PhO | 2-$CF_3$-5-Cl-PhO | 2-$CF_3$-6-Cl-PhO |
| 2-$CF_3$-3-$CH_3$-PhO | 2-$CF_3$-5-$CH_3$-PhO | 2-$CF_3$-6-$CH_3$-PhO | 3-$CF_3$-2-$CH_3$-PhO |
| 3-$CF_3$-5-$CH_3$-PhO | 3-$CF_3$-6-$CH_3$-PhO | 2,3-bis$CF_3$PhO | 2,6-bis$CF_3$PhO |
| 3,5-bis$CF_3$PhO | 2-$CF_3$-6-$CH_3$O-Ph O | 2-$CF_3$PhN$CH_3$ | 3-$CF_3$PhN$CH_3$ |
| 2-$CF_3CF_2$PhN$CH_3$ | 3-$CF_3CF_2$PhN$CH_3$ | 2-$(CF_3)_2$CFPhN$CH_3$ | 3-$(CF_3)_2$CFPhN$CH_3$ |
| 2-$CF_3$OPhN$CH_3$ | 3-$CF_3$OPhN$CH_3$ | 2-$CF_3CF_2$OPhN$CH_3$ | 3-$CF_3CF_2$OPhN$CH_3$ |
| 2-$CF_3CH_2$OPhNC $H_3$ | 3-$CF_3CH_2$OPhNC $H_3$ | 2-$CF_3$-3-F-PhNC $H_3$ | 2-$CF_3CH_2$PhN$CH_3$ |
| 2-$CF_3$-5-F-PhNC $H_3$ | 2-$CF_3$-6-F-PhNCH $_3$ | 3-$CF_3$-2-F-PhNC $H_3$ | 3-$CF_3CH_2$PhN$CH_3$ |
| 3-$CF_3$-6-F-PhNC $H_3$ | 2-$CF_3$-3-Cl-PhNC $H_3$ | 2-$CF_3$-5-Cl-PhNC $H_3$ | 3-$CF_3$-5-F-PhN$CH_3$ |
| 2-$CF_3$-3-$CH_3$-PhN $CH_3$ | 2-$CF_3$-5-$CH_3$-PhN $CH_3$ | 2-$CF_3$-6-$CH_3$-Ph N$CH_3$ | 2-$CF_3$-6-Cl-PhN$CH_3$ |
| 3-$CF_3$-5-$CH_3$-PhN $CH_3$ | 3-$CF_3$-6-$CH_3$-PhN $CH_3$ | 2,3-bis$CF_3$PhNC $H_3$ | 3-$CF_3$-2-$CH_3$-PhNC $H_3$ |
| 3,5-bis$CF_3$PhNC $H_3$ | 2-$CF_3$-6-$CH_3$O-Ph N$CH_3$ | 2,6-bis$CF_3$PhNC $H_3$ | |

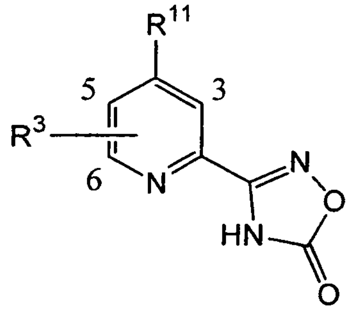

( 1-b )

Compounds represented by general formula (1-b) in which $R^{11}$ represents a 2-$CF_3$Ph group and $R^3$ represents any group shown in the following Table 2;

Table 2

| | | | |
|---|---|---|---|
| H | 3-F | 3-Cl | 3-Br |
| 3-$CH_3$ | 3-$CH_2CH_3$ | 3-$CH(CH_3)_2$ | 3-$CH(CH_3)CH_2CH_3$ |
| 3-$OCH_3$ | 3-$OCH_2CH_3$ | 3-$OCH(CH_3)_2$ | 3-$OCH(CH_3)CH_2CH_3$ |
| 3-$OCH_2CF_3$ | 3-$OCF_3$ | 3-cyclopropyl | 3-cyclobutyl |
| 3-cyclopentyl | 3-$CF_3$ | 3-$CF_2CF_3$ | 3-$CF(CF_3)_2$ |
| 3-$OCH(CH_3)CF_3$ | 5-F | 5-Cl | 5-Br |
| 5-$CH_3$ | 5-$CH_2CH_3$ | 5-$CH_2CH_3$ | 5-$CH_2CH_3$ |
| 5-$OCH_3$ | 5-$OCH_2CH_3$ | 5-$OCH(CH_3)_2$ | 5-$OCH(CH_3)CH_2CH_3$ |
| 5-$OCH_2CF_3$ | 5-$OCF_3$ | 5-$OCH(CH_3)CF_3$ | 5-$CF_3$ |
| 5-$CF_2CF_3$ | 5-$CF(CF_3)_2$ | 5-cyclopropyl | 5-cyclobutyl |
| 6-F | 6-I | 6-Cl | 6-Br |
| 6-$CH_3$ | 6-$CH_2CH_3$ | 6-$CH(CH_3)_2$ | 6-$CH(CH_3)CH_2CH_3$ |
| 6-$OCH_3$ | 6-$OCH_2CH_3$ | 6-$OCH(CH_3)_2$ | 6-$OCH(CH_3)CH_2CH_3$ |

(continued)

| | | | |
|---|---|---|---|
| 6-$OCH_2CF_3$ | 6-$OCF_3$ | 6-$OCF_2CF_3$ | 6-$OCH(CH_3)CF_3$ |
| 6-$CF_3$ | 6-$CF_2CF_3$ | 6-$CF(CF_3)_2$ | 6-cyclopropyl |
| 6-cyclobutyl | 6-cyclopentyl | 6-(1-$CH_3$-cyclopropyl) | 6-cyclopropyloxy |
| 6-cyclobutyloxy | | | |

Compounds represented by general formula (1-b) in which $R^{11}$ represents a 3-$CF_3$Ph group and $R^3$ represents any group shown in the above-described Table 2;

Compounds represented by general formula (1-b) in which $R^{11}$ represents a 3-$CF_3$OPh group and $R^3$ represents any group shown in the above-described Table 2;

Compounds represented by general formula (1-b) in which $R^{11}$ represents a 2-$CF_3$-3-F-Ph group and $R^3$ represents any group shown in the above-described Table 2;

Compounds represented by general formula (1-b) in which $R^{11}$ represents a 2-$CF_3$-5-F-Ph group and $R^3$ represents any group shown in the above-described Table 2;

Compounds represented by general formula (1-b) in which $R^{11}$ represents a 2-$CF_3$-6-F-Ph group and $R^3$ represents any group shown in the above-described Table 2;

Compounds represented by general formula (1-b) in which $R^{11}$ represents a 2-$CF_3$-3-Cl-Ph group and $R^3$ represents any group shown in the above-described Table 2;

Compounds represented by general formula (1-b) in which $R^{11}$ represents a 2-$CF_3$-5-Cl-Ph group and $R^3$ represents any group shown in the above-described Table 2;

Compounds represented by general formula (1-b) in which $R^{11}$ represents a 2-$CF_3$-6-Cl-Ph group and $R^3$ represents any group shown in the above-described Table 2;

Compounds represented by general formula (1-b) in which $R^{11}$ represents a 2-$CF_3$-3-$CH_3$-Ph group and $R^3$ represents any group shown in the above-described Table 2;

Compounds represented by general formula (1-b) in which $R^{11}$ represents a 2-$CF_3$-5-$CH_3$-Ph group and $R^3$ represents any group shown in the above-described Table 2;

Compounds represented by general formula (1-b) in which $R^{11}$ represents a 2-$CF_3$-6-$CH_3$-Ph group and $R^3$ represents any group shown in the above-described Table 2;

Compounds represented by general formula (1-b) in which $R^{11}$ represents a 3-$CF_3$-2-F-Ph group and $R^3$ represents any group shown in the above-described Table 2;

Compounds represented by general formula (1-b) in which $R^{11}$ represents a 3-$CF_3$-5-F-Ph group and $R^3$ represents any group shown in the above-described Table 2;

Compounds represented by general formula (1-b) in which $R^{11}$ represents a 3-$CF_3$-6-F-Ph group and $R^3$ represents any group shown in the above-described Table 2;

Compounds represented by general formula (1-b) in which $R^{11}$ represents a 3-$CF_3$-2-Cl-Ph group and $R^3$ represents any group shown in the above-described Table 2;

Compounds represented by general formula (1-b) in which $R^{11}$ represents a 3-$CF_3$-5-Cl-Ph group and $R^3$ represents any group shown in the above-described Table 2;

Compounds represented by general formula (1-b) in which $R^{11}$ represents a 3-$CF_3$-6-Cl-Ph group and $R^3$ represents any group shown in the above-described Table 2;

Compounds represented by general formula (1-b) in which $R^{11}$ represents a 2, 6-bis$CF_3$Ph group and $R^3$ represents any group shown in the above-described Table 2;

Compounds represented by general formula (1-b) in which $R^{11}$ represents a 2,5-bis$CF_3$Ph group and $R^3$ represents any group shown in the above-described Table 2;

Compounds represented by general formula (1-b) in which $R^{11}$ represents a 3-$CF_3$O-2-F-Ph group and $R^3$ represents any group shown in the above-described Table 2;

Compounds represented by general formula (1-b) in which $R^{11}$ represents a 3-$CF_3$O-5-F-Ph group and $R^3$ represents any group shown in the above-described Table 2;

Compounds represented by general formula (1-b) in which $R^{11}$ represents a 3-$CF_3$O-6-F-Ph group and $R^3$ represents any group shown in the above-described Table 2;

Compounds represented by general formula (1-b) in which $R^{11}$ represents a 3-$CF_3$O-2-Cl-Ph group and $R^3$ represents any group shown in the above-described Table 2;

Compounds represented by general formula (1-b) in which $R^{11}$ represents a 3-$CF_3$O-5-Cl-Ph group and $R^3$ represents any group shown in the above-described Table 2;

Compounds represented by general formula (1-b) in which $R^{11}$ represents a 3-$CF_3$O-6-Cl-Ph group and $R^3$

represents any group shown in the above-described Table 2.

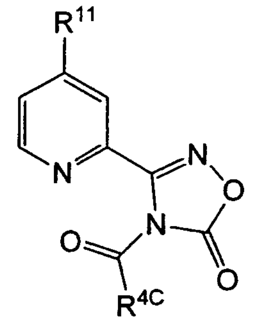

( 1-C )

Compounds represented by general formula (1-c) in which $R^{11}$ represents a 2-$CF_3$Ph group and $R^{4C}$ represents any group shown in the following Table 3;

Table 3

| | | | |
|---|---|---|---|
| $CH_3$ | $CH_2CH_3$ | $CH(CH_3)_2$ | $C(CH_3)_3$ |
| $CH_2C(CH_3)_3$ | $CH(CH_3)CH_2CH_3$ | $CH_2CH(CH_3)_2$ | $CH_2CH_2C(CH_3)_3$ |
| $C(CH_3)_2CH_2CH_3$ | cyclohexyl | 1-$CH_3$cyclohexyl | 2-$CH_3$cyclohexyl |
| 3-$CH_3$cylohexyl | 4-$CH_3$cyclohexyl | 1-$CH_3$cyclopentyl | cyclopentyl |
| cycloheptyl | cyclopropyl | 1-$CH_3$cyclopropyl | $CF_3$ |
| $N(CH_3)_2$ | $N(CH_2CH_3)_2$ | $N(CH_2CH_2CH_3)_2$ | $N(CH_3)CH_2CH_3$ |
| $N[CH_2CH(CH_3)_2]$ | $N(CH_3)OCH_3$ | $N(CH_2CH=CH_2)$ | $N(CH_2CCH)$ |
| 1-pyrrolidinyl | 2-$CH_3$pyrrolidin-1-yl | piperidino | 2-$CH_3$piperidin-1-yl |
| morpholino | | | |

Compounds represented by general formula (1-c) in which $R^{11}$ represents a 3-$CF_3$Ph group and $R^4$ represents any group shown in the above-described Table 3;

Compounds represented by general formula (1-c) in which $R^{11}$ represents a 2-$CF_3$-3-F-Ph group and $R^4$ represents any group shown in the above-described Table 3;

Compounds represented by general formula (1-c) in which $R^{11}$ represents a 2-$CF_3$-5-F-Ph group and $R^{4c}$ represents any group shown in the above-described Table 3;

Compounds represented by general formula (1-c) in which $R^{11}$ represents a 2-$CF_3$-6-F-Ph group and $R^{4c}$ represents any group shown in the above-described Table 3;

Compounds represented by general formula (1-c) in which $R^{11}$ represents a 2-$CF_3$-3-Cl-Ph group and $R^{4c}$ represents any group shown in the above-described Table 3;

Compounds represented by general formula (1-c) in which $R^{11}$ represents a 2-$CF_3$-5-Cl-Ph group and $R^4$ represents any group shown in the above-described Table 3;

Compounds represented by general formula (1-c) in which $R^{11}$ represents a 2-$CF_3$-6-Cl-Ph group and $R^{4c}$ represents any group shown in the above-described Table 3;

Compounds represented by general formula (1-c) in which $R^{11}$ represents a 3-$CF_3$-2-F-Ph group and $R^4$ represents any group shown in the above-described Table 3;

Compounds represented by general formula (1-c) in which $R^{11}$ represents a 3-$CF_3$-5-F-Ph group and $R^{4c}$ represents any group shown in the above-described Table 3;

Compounds represented by general formula (1-c) in which $R^{11}$ represents a 3-$CF_3$-6-F-Ph group and $R^{4c}$ represents any group shown in the above-described Table 3;

Compounds represented by general formula (1-c) in which $R^{11}$ represents a 3-$CF_3$-2-Cl-Ph group and $R^{4c}$ represents any group shown in the above-described Table 3;

Compounds represented by general formula (1-c) in which $R^{11}$ represents a 3-$CF_3$-5-Cl-Ph group and $R^4$ represents any group shown in the above-described Table 3;

Compounds represented by general formula (1-c) in which $R^{11}$ represents a 3-$CF_3$-6-Cl-Ph group and $R^{4c}$

represents any group shown in the above-described Table 3;

Compounds represented by general formula (1-c) in which $R^{11}$ represents a 2, 6-bis$CF_3$Ph group and $R^{4c}$ represents any group shown in the above-described Table 3;

Compounds represented by general formula (1-c) in which $R^{11}$ represents a 2, 5-bis$CF_3$Ph group and $R^{4c}$ represents any group shown in the above-described Table 3;

Compounds represented by general formula (1-c) in which $R^{11}$ represents a 3-$CF_3$O-2-F-Ph group and $R^3$ represents any group shown in the above-described Table 3;

Compounds represented by general formula (1-c) in which $R^{11}$ represents a 3-$CF_3$O-5-F-Ph group and $R^{4c}$ represents any group shown in the above-described Table 3;

Compounds represented by general formula (1-c) in which $R^{11}$ represents a 3-$CF_3$O-6-F-Ph group and $R^{4c}$ represents any group shown in the above-described Table 3;

Compounds represented by general formula (1-c) in which $R^{11}$ represents a 3-$CF_3$O-2-Cl-Ph group and $R^{4c}$ represents any group shown in the above-described Table 3;

Compounds represented by general formula (1-c) in which $R^{11}$ represents a 3-$CF_3$O-5-Cl-Ph group and $R^{4c}$ represents any group shown in the above-described Table 3;

Compounds represented by general formula (1-c) in which $R^{11}$ represents a 3-$CF_3$O-6-Cl-Ph group and $R^{4c}$ represents any group shown in the above-described Table 3.

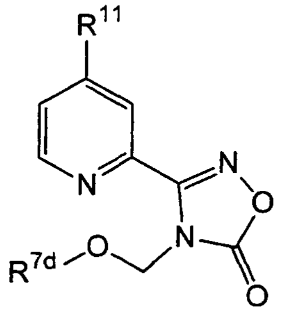

( 1-d)

Compounds represented by general formula (1-d) in which $R^{11}$ represents a 2-$CF_3$Ph group and $R^{7d}$ represents any group shown in the following Table 4;

Table 4

| | | | |
|---|---|---|---|
| $CH_3$ | $CH_2CH_3$ | $CH(CH_3)_2$ | $C(CH_3)_3$ |
| $CH_2C(CH_3)_3$ | $C(CH_3)_2CH_2CH_3$ | $CF_3$ | $CF_2CF_3$ |
| cyclopropyl | 1-$CH_3$cyclopropyl | cyclopentyl | 1-$CH_3$cyclopentyl |
| 1-$CH_3$cyclohexyl | cyclohexyl | Ph | 2-$CH_3$-Ph |
| 2-$CH_3CH_2$-Ph | 2-$CH_3$O-Ph | 2-F-Ph | 2-Cl-Ph |
| 2-Br-Ph | 2-$CF_3$O-Ph | 2-$CF_3CH_2$O-Ph | 2-CN-Ph |
| 2-$NO_2$Ph | 2-$CF_3$-Ph | 2-$CF_3CF_2$-Ph | 3-F-Ph |
| 3-Cl-Ph | 3-Br-Ph | 3-$CH_3$-Ph | 3-$CH_3CH_2$-Ph |
| 3-$CH_3$O-Ph | 3-$CF_3$-Ph | 3-$CF_3$O-Ph | 2-$CF_3CH_2$O-Ph |
| 3-CN-Ph | 3-$NO_2$Ph | 4-F-Ph | 4-Cl-Ph |
| 4-$CF_3$O-Ph | 4-$(CH_3)_3$C-Ph | 4-$CH_3$-Ph | 4-CN-Ph |
| 3,5-diF-Ph | 2,4-diF-Ph | 2,5-diFPh | 2,4-diCl-Ph |
| 3,5-diCl-Ph | 2,5-diCl-Ph | 2,6-diCl-Ph | 3,5-di$CH_3$-Ph |
| 2,5-di$CH_3$-Ph | 2,4-di$CH_3$-Ph | 2,6-di$CH_3$-Ph | 2-Cl-4-$CH_3$-Ph |
| 2-Cl-4-CN-Ph | 2-$CH_3$-4-CN-Ph | benzyl | 2-$CH_3$benzyl |
| 2-$CH_3CH_2$-benzyl | 2-$CH_3$O-benzyl | 2-F-benzyl | 2-Cl-benzyl |
| 2-Br-benzyl | 2-$CF_3$O-benzyl | 2-$CF_3CH_2$O-benzyl | 2-CN-benzyl |

(continued)

| | | | |
|---|---|---|---|
| 2-$NO_2$benzyl | 2-$CF_3$-benzyl | 2-$CF_3CF_2$-benzyl | 3-F-benzyl |
| 3-Cl-benzyl | 3-Br-benzyl | 3-$CH_3$-benzyl | 3-$CH_3CH_2$-benzyl |
| 3-$CH_3$O-benzyl | 3-$CF_3$-benzyl | 3-$CF_3$O-benzyl | 2-$CF_3CH_2$O-benzyl |
| 3-CN-benzyl | 3-$NO_2$benzyl | 4-F-benzyl | 4-Cl-benzyl |
| 4-$CF_3$O-benzyl | 4-$(CH_3)_3$C-benzyl | 4-$CH_3$-benzyl | 4-CN-benzyl |
| 3,5-diF-benzyl | 2,4-diF-benzyl | 2,5-diF-benzyl | 2,4-diCl-benzyl |
| 3,5-diCl-benzyl | 2,5-diCl-benzyl | 2,6-diCl-benzyl | 3,5-di$CH_3$-benzyl |
| 2,5-di$CH_3$-benzyl | 2,4-di$CH_3$-benzyl | 2,6-di$CH_3$-benzyl | 2-Cl-4-$CH_3$-benzyl |

Compounds represented by general formula (1-d) in which $R^{11}$ represents a 3-$CF_3$Ph group and $R^{7\ d}$ represents any group shown in the above-described Table 4;

Compounds represented by general formula (1-d) in which $R^{11}$ represents a 2-$CF_3$-3-F-Ph group and $R^{7d}$ represents any group shown in the above-described Table 4;

Compounds represented by general formula (1-d) in which $R^{11}$ represents a 2-$CF_3$-5-F-Ph group and $R^d$ represents any group shown in the above-described Table 4;

Compounds represented by general formula (1-d) in which $R^{11}$ represents a 2-$CF_3$-6-F-Ph group and $R^{7d}$ represents any group shown in the above-described Table 4;

Compounds represented by general formula (1-d) in which $R^{11}$ represents a 2-$CF_3$-3-Cl-Ph group and $R^{7\ d}$ represents any group shown in the above-described Table 4;

Compounds represented by general formula (1-d) in which $R^{11}$ represents a 2-$CF_3$-5-Cl-Ph group and $R^d$ represents any group shown in the above-described Table 4;

Compounds represented by general formula (1-d) in which $R^{11}$ represents a 2-$CF_3$-6-Cl-Ph group and $R^{7d}$ represents any group shown in the above-described Table 4;

Compounds represented by general formula (1-d) in which $R^{11}$ represents a 3-$CF_3$-2-F-Ph group and $R^{7d}$ represents any group shown in the above-described Table 4;

Compounds represented by general formula (1-d) in which $R^{11}$ represents a 3-$CF_3$-5-F-Ph group and $R^{7d}$ represents any group shown in the above-described Table 4;

Compounds represented by general formula (1-d) in which $R^{11}$ represents a 3-$CF_3$-6-F-Ph group and $R^{7d}$ represents any group shown in the above-described Table 4;

Compounds represented by general formula (1-d) in which $R^{11}$ represents a 3-$CF_3$-2-Cl-Ph group and $R^{7d}$ represents any group shown in the above-described Table 4;

Compounds represented by general formula (1-d) in which $R^{11}$ represents a 3-$CF_3$-5-Cl-Ph group and $R^{7d}$ represents any group shown in the above-described Table 4;

Compounds represented by general formula (1-d) in which $R^{11}$ represents a 3-$CF_3$-6-Cl-Ph group and $R^{7d}$ represents any group shown in the above-described Table 4;

Compounds represented by general formula (1-d) in which $R^{11}$ represents a 2,6-bis$CF_3$ Ph group and $R^{7d}$ represents any group shown in the above-described Table 4;

Compounds represented by general formula (1-d) in which $R^{11}$ represents a 2, 5-bis$CF_3$ Ph group and $R^{7d}$ represents any group shown in the above-described Table 4;

Compounds represented by general formula (1-d) in which $R^{11}$ represents a 3-$CF_3$O-2-F-Ph group and $R^{7d}$ represents any group shown in the above-described Table 4;

Compounds represented by general formula (1-d) in which $R^{11}$ represents a 3-$CF_3$O-5-F-Ph group and $R^7$ represents any group shown in the above-described Table 4;

Compounds represented by general formula (1-d) in which $R^{11}$ represents a 3-$CF_3$O-6-F-Ph group and $R^{7d}$ represents any group shown in the above-described Table 4;

Compounds represented by general formula (1-d) in which $R^{11}$ represents a 3-$CF_3$O-2-Cl-Ph group and $R^{7d}$ represents any group shown in the above-described Table 4;

Compounds represented by general formula (1-d) in which $R^{11}$ represents a 3-$CF_3$O-5-Cl-Ph group and $R^{7d}$ represents any group shown in the above-described Table 4;

Compounds represented by general formula (1-d) in which $R^{11}$ represents a 3-$CF_3$O-6-Cl-Ph group and $R^{7d}$ represents any group shown in the above-described Table 4.

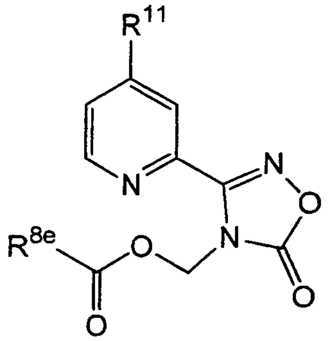

(1-e)

Compounds represented by general formula (1-e) in which $R^{11}$ represents a 2-$CF_3$ Ph group and $R^{8e}$ represents any group shown in the following Table 5;

Table 5

| | | | |
|---|---|---|---|
| $CH_3$ | $CH_2CH_3$ | $CH(CH_3)_2$ | $C(CH_3)_3$ |
| $CH_2C(CH_3)_3$ | $C(CH_3)_2CH_2CH_3$ | $CF_3$ | $CF_2CF_3$ |
| cyclopropyl | 1-$CH_3$cyclopropyl | cyclopentyl | 1-$CH_3$cyclopentyl |
| 1-$CH_3$cyclohexyl | cyclohexyl | Ph | 2-$CH_3$-Ph |
| 2-$CH_3CH_2$-Ph | 2-$CH_3O$-Ph | 2-F-Ph | 2-Cl-Ph |
| 2-Br-Ph | 2-$CF_3O$-Ph | 2-$CF_3CH_2O$-Ph | 2-CN-Ph |
| 2-$NO_2$Ph | 2-$CF_3$-Ph | 2-$CF_3CF_2$-Ph | 3-F-Ph |
| 3-Cl-Ph | 3-Br-Ph | 3-$CH_3$-Ph | 3-$CH_3CH_2$-Ph |
| 3-$CH_3O$-Ph | 3-$CF_3$-Ph | 3-$CF_3O$-Ph | 2-$CF_3CH_2O$-Ph |
| 3-CN-Ph | 3-$NO_2$Ph | 4-F-Ph | 4-Cl-Ph |
| 4-$CF_3O$-Ph | 4-$(CH_3)_3C$-Ph | 4-$CH_3$-Ph | 4-CN-Ph |
| 3,5-diF-Ph | 2,4-diF-Ph | 2,5-diFPh | 2,4-diCl-Ph |
| 3,5-diCl-Ph | 2,5-diCl-Ph | 2,6-diCl-Ph | 3,5-di$CH_3$-Ph |
| 2,5-di$CH_3$-Ph | 2,4-di$CH_3$-Ph | 2,6-di$CH_3$-Ph | 2-Cl-4-$CH_3$-Ph |
| 2-Cl-4-CN-Ph | 2-$CH_3$-4-CN-Ph | 3,4,5-triF-Ph | 2,4,6-triF-Ph |
| 3,4,5-triCl-Ph | 2,4,6-triCl-Ph | 3,4,5-tri$CH_3$-Ph | 2,4,6-tri$CH_3$-Ph |

Compounds represented by general formula (1-e) in which $R^{11}$ represents a 3-$CF_3$ Ph group and $R^{8e}$ represents any group shown in the above-described Table 5;

Compounds represented by general formula (1-e) in which $R^{11}$ represents a 2-$CF_3$-3-F-Ph group and $R^{8e}$ represents any group shown in the above-described Table 5;

Compounds represented by general formula (1-e) in which $R^{11}$ represents a 2-$CF_3$-5-F-Ph group and $R^{8e}$ represents any group shown in the above-described Table 5;

Compounds represented by general formula (1-e) in which $R^{11}$ represents a 2-$CF_3$-6-F-Ph group and $R^{8e}$ represents any group shown in the above-described Table 5;

Compounds represented by general formula (1-e) in which $R^{11}$ represents a 2-$CF_3$-3-Cl-Ph group and $R^{8e}$ represents any group shown in the above-described Table 5;

Compounds represented by general formula (1-e) in which $R^{11}$ represents a 2-$CF_3$-5-Cl-Ph group and $R^{8e}$ represents any group shown in the above-described Table 5;

Compounds represented by general formula (1-e) in which $R^{11}$ represents a 2-$CF_3$-6-Cl-Ph group and $R^{8e}$ represents any group shown in the above-described Table 5;

Compounds represented by general formula (1-e) in which $R^{11}$ represents a 3-$CF_3$-2-F-Ph group and $R^{8e}$ represents any group shown in the above-described Table 5;

Compounds represented by general formula (1-e) in which $R^{11}$ represents a 3-$CF_3$-5-F-Ph group and $R^{8e}$

represents any group shown in the above-described Table 5;

Compounds represented by general formula (1-e) in which $R^{11}$ represents a 3-$CF_3$-6-F-Ph group and $R^{8e}$ represents any group shown in the above-described Table 5;

Compounds represented by general formula (1-e) in which $R^{11}$ represents a 3-$CF_3$-2-Cl-Ph group and $R^{8e}$ represents any group shown in the above-described Table 5;

Compounds represented by general formula (1-e) in which $R^{11}$ represents a 3-$CF_3$-5-Cl-Ph group and $R^{8e}$ represents any group shown in the above-described Table 5;

Compounds represented by general formula (1-e) in which $R^{11}$ represents a 3-$CF_3$-6-Cl-Ph group and $R^{8e}$ represents any group shown in the above-described Table 5;

Compounds represented by general formula (1-e) in which $R^{11}$ represents a 2, 6-bis$CF_3$Ph group and $R^{8e}$ represents any group shown in the above-described Table 5;

Compounds represented by general formula (1-e) in which $R^{11}$ represents a 2, 5-bis$CF_3$Ph group and $R^{8e}$ represents any group shown in the above-described Table 5.

Examples of pests on which the inventive compound exerts an effect include arthropods such as insects, mites and the like, nemathelminths such as nematodes and the like; etc, and specific examples thereof include organisms shown below.

Hemiptera harmful insects: Planthoppers (Delphacidae) such as small brown planthopper (Laodelphax striatellus), brown rice planthopper (Nilaparvata lugens), white-backed rice planthopper (Sogatella furcifera) and the like; leafhoppers (Deltocephalidae) such as green rice leafhopper (Nephotettix cincticeps), green rice leafhopper (Nephotettix virescens) and the like; aphids (Aphididae) such as cotton aphid (Aphis gossypii), green peach aphid (Myzus persicae) and the like; stink bugs (Pentatomidae) such as green stink bug (Nezara antennata), bean bug (Riptortus clavetus), (Eysarcoris lewisi), white spotted spined bug (Eysarcoris parvus), Brownwinged green bug (Plautia stali), stink bug (Halyomorpha mista), sorghum plant bug (Stenotus rubrovittatus), rice leaf bug (Trigonotylus ruficornis) and the like; whiteflies (Aleyrodidae) such as greenhouse whitefly (Trialeurodes vaporariorum), silverleaf whitefly (Bemisia argentifolii) and the like; scales (Coccidae) such as Calfornia red scale (Aonidiella aurantii), San Jose scale (Comstockaspis perniciosa), citrus north scale (Unaspis citri), red wax scale (Ceroplastes rubens), cottonycushion scale (Icerya purchasi) and the like; lace bugs (Tingidae) ; cimicidae such as Cimex lectularius and the like; psyllids (Psyllidae); etc.

Lepidoptera harmful insects: Pyralid moths (Pyralidae) such as rice stem borer (Chilo suppressalis), rice leafroller (Cnaphalocrocis medinalis), cotton leafroller (Notarcha derogata), Indian meal moth (Plodia interpunctella) and the like; owlet moths (Noctuidae) such as common cutworm (Spodoptera litura), armyworm (Pseudaletia separata), Thoricoplusia spp., Heliothis spp. , Helicoverpa spp. and the like; white butterflies (Pieridae) such as common white (Pieris rapae) and the like; tortricid moths (Tortricidae) such as Adoxophyes spp., oriental fruit moth (Grapholita molesta), codling moth (Cydia pomonella) and the like; Carposinidae such as peach fruit moth (Carposina niponensis) and the like; lyonetiid moths (Lyonetiidae) such as Lyonetia spp. and the like; tussock moths (Lymantriidae) such as Lymantria spp., Euproctis spp. and the like; yponomeutid moths (Yponomeutidae) such as diamondback (Plutella xylostella) and the like; gelechiid moths (Gelechiidae) such as pink bollworm (Pectinophora gossypiella) and the like; tiger moths and allies (Arctiidae) such as fall webworm (Hyphantria cunea) and the like; tineid moths (Tineidae) such as casemaking clothes moth (Tinea translucens), webbing clothes moth (Tineola bisselliella) and the like; etc.

Diptera harmful insects: Culices such as common mosquito (Culex pipiens pallens), Cluex tritaeniorhynchus, Cluex quinquefasciatus and the like; Aedes spp. such as yellow fever mosquito (Aedes aegypti), Asian tiger mosquito (Aedes albopictus) and the like; Anopheles spp. such as Anopheles sinensis and the like; chironomids (Chironomidae); house flies (Muscidae) such as Musca domestica, Muscina stabulans and the like; blow flies (Calliphoridae); flesh flies (Sarcophagidae); little house flies (Fanniidae); anthomyiid flies (Anthomyiidae) such as seedcorn fly (Delia platura), onion fly (Delia antiqua) and the like; leafminer flies (Agromyzidae) such as legume leafminer (Liriomyza trifolii) and the like; Tephritidae; Drosophilidae; humpbacked flies (Phoridae) such as Megaselia spiracularis and the like; moth flies (Psychodidae) such as Clogmia albipunctata and the like; Simuliidae; Tabanidae; stable flies (stomoxys calcitrans); etc.

Coleoptera harmful insects: Corn root worms (Diabrotica spp.) such as Western corn root worm (Diabrotica virgifera virgifera), Sourthern corn root worm (Diabrotica undecimpunctata howardi) and the like; scarabs (Scarabaeidae) such as cupreous chafer (Anomala cuprea), soybean beetle (Anomala rufocuprea) and the like; weevils such as maize weevil (Sitophilus zeamais), rice water weevil (Lissorhoptrus oryzophilus), azuki bean weevil (Callosobruchus chinensis) and the like; darkling beetles (Tenebrionidae) such as yellow mealworm (Tenebrio molitor), red flour beetle (Tribolium castaneum) and the like; leaf beetles (Chrysomelidae) such as rice leaf beetle (Oulema oryzae), cucurbit leaf beetle (Aulacophora femoralis), striped flea beetle (Phyllotreta striolata), Colorado potato beetle (Leptinotarsa decemlineata) and the like; dermestid beetles (Dermestidae) such as hide beetle (Dermestes maculates) and the like; deathwatch beetles (Anobiidae); Epilachna such as Twenty-eight-spotted ladybird (Epilachna vigintioctopunctata); bark beetles (Scolytidae); false powder-post beetles (Bostrychidae); spider beetles (Ptinidae); longhorn beetles (Cerambycidae); Paederus fuscipens; etc.

Blattodea harmful insects: German cockroach (Blattella germanica), smokybrown cockroach (Periplaneta fuliginosa), American cockroach (Periplaneta americana), brown cockroach (Periplaneta brunnea), oriental cockroach (Blatta orientalis) and the like.

Thysanoptera harmful insects: melon thrips (Thrips palmi), onion thrips (Thrips tabaci), yellow citrus thrips (Frankliniella occidentalis), flower thrips (Frankliniella intonsa), etc.

Hymenoptera harmful insects: Ants (Formicidae) such as pharaoh ant (Monomorium pharaosis), negro ant (Formica fusca japonica), black house ant (Ochetellus glaber), Pristomyrmex pungens, Pheidole noda and the like; hornets (Vespidae) ; bethylid wasps (Betylidae); sawflies (Tenthredinidae) such as Athalia japonica and the like; etc.

Orthoptera harmful insects: Mole cricket (Gryllotalpidae), locust (Asiatic), cricket (Gryllidae), and the like.

Shiphonaptera harmful insects: Cat flea (Ctenocephalides felis), dog flea (Ctenocephalides canis), human flea (Pulex irritans), oriental rat flea (Xenopsylla cheopis), and the like.

Anoplura harmful insects: Human body louse (Pediculus humanus corporis), crab louse (Phthirus pubis), short-nosed cattle louse (Haematopinus eurysternus), sheep louse (Dalmalinia ovis), hog louse (Haematopinus suis), and the like.

Isoptera harmful insects: Subterranean termites such as Japanese subterranean termite (Reticulitermes speratus), Formosan subterranean termite (Coptotermesformosanus), eastern subterranean termite (Reticulitermes flavipes), western subterranean termite (Reticulitermes hesperus), dark southern subterranean termite (Reticulitermes virginicus), Arid land subterranean termite (Reticulitermes tibialis), desert subterranean termite (Heterotermes aureus) and the like; drywood termites such as western drywood termite (Incisitermes minor) and the like; dampwood termites such as Nevada dampwood termite (Zootermopsis nevadensis) and the like; ect.

Acarina harmful insects: Spider mites (Tetranychidae) such as two-spotted spider mite (Tetranychus urticae), Kanzawa spider mite (Tetranychus kanzawai), citrus red mite (Panonychus citri), European red mite (Panonychus ulmi), Oligonychus spp. And the like; eriophyidmites (Eriophyidae) such as tomato rust mite (Aculops lycopersici), pink citrus rust mite (Aculops pelekassi), apple rust mite (Aculus schlechtendali) and the like; tarosonemid mites (Tarsonemidae) such as broad mite (Polyphagotarsonemus latus) and the like; false spider mites (Tenuipalpidae); Tuckerellidae; ticks (Ixodidae) such as Haemaphysalis longicornis, Haemaphysalis flava, American dog tick (Dermacentor variabilis), Haemaphysalis flava, Dermacentor taiwanicus, Ixodes ovatus, Ixodes persulcatus, black legged tick (Ixodes scapularis), Boophilus microplus, lone star tick (Amblyomma americanum), Rhipicephalus sanguineus and the like; acarid mites (Acaridae) such as mold mite (Tyrophagus putrescentiae) and the like; house dust mites (Pyroglyphidae) such as Dermatophagoides farinae, Dermatophagoides ptrenyssnus and the like; cheyletide mites (Cheyletidae) such as Cheyletus eruditus, Cheyletus malaccensis, Cheyletus moorei and the like; parasitoid mites (Dermanyssidae) such as tropical rat mite (Ornithonyssus bacoti), northern fowl mite (Ornithonyssus sylviarum), poultry redmite (Dermanyssus gallinae) and the like; chiggers (Trombiculidae) such as Leptotrombidium akamushi and the like; etc.

Spiders (Araneae) : Japanese foliage spider (Chiracanthium japonicum), redback spider (Latrodectus hasseltii), and the like.

Chilopoda: house centipede (Thereuonema hilgendorfi), Scolopendra subspinipes, and the like.

Diplopoda: garden millipede (Oxidus gracilis), Nedyopus tambanus, and the like.

Isopoda: common pill bug (Armadillidium vulgare), and the like.

Gastropoda: Limax marginatus, Limax flavus, and the like.

Nematoca: coffee root-lesion nematode (Pratylenchus coffeae), cobb root-lesion nematode (Pratylenchus fallax), soybean cyst nematode (Heterodera glycines), potato cyst nematode (Globodera rostochiensis), northern root knot nematode (Meloidogyne hapla), sweet potato root knot nematode (Meloidogyne incognita) and the like.

The pesticidal composition of the present invention comprises the inventive compound as an active ingredient.

The pesticidal composition of the present invention is, in general, formulated into an oil solution, an emulsifiable concentrate, a flowable formulation, a granule, a dust, a poison bait, a microgranule and the like, by mixing the inventive compound and a solid carrier, a liquid carrier, a gaseous carrier and/or a poison bait and the like, and if necessary, adding a surfactant, and other formulation auxiliaries. The pesticidal composition of the present invention contains the inventive compound usually in an amount of 0.01 to 95 wt%.

Examples of the above-described solid carrier include fine powders or granular materials made of clays (kaolin clay, diatomaceous earth, synthetic hydrous silicon oxide, bentonite, Fubasami clay, acid clay and the like), talcs, ceramic, other inorganic minerals (sericite, quartz, sulfur, activated carbon, calcium carbonate, hydrated silica and the like), chemical fertilizers (ammonium sulfate, ammonium phosphate, ammonium nitrate, urea, ammonium chloride, and the like), etc. and examples of the liquid carrier include water, alcohols (methanol, ethanol and the like), ketones (acetone, methyl ethyl ketone and the like), aromatic hydrocarbons (benzene, toluene, xylene, ethylbenzene, methylnaphthalene and the like), aliphatic hydrocarbons (hexane, cyclohexane, kerosene, light oil and the like), esters (ethyl acetate, butyl acetate and the like), nitriles (acetonitrile, isobutyronitrile and the like), ethers (diisopropyl ether, dioxane and the like), acid amides (N,N-dimethylformamide, N,N-dimethylacetamide and the like), halogenated hydrocarbons (dichloromethane, trichloroethane, carbon tetrachloride and the like), dimethyl sulfoxide and vegetable oils (soybean oil, cottonseed oil and the like), etc.

Examples of the gaseous carrier include fluorocarbon, butane gas, LPG (liquefied petroleum gas), dimethyl ether, carbon dioxide gas and the like.

Examples of the surfactant include alkyl sulfate ester salts, alkyl sulfonate salts, alkyl aryl sulfonate salts, alkyl aryl ethers and polyoxyethylene compounds thereof, polyethylene glycol ethers, polyhydric alcohol esters, and sugar alcohol derivatives.

The other formulation auxiliaries include fixing agents, dispersing agents, stabilizers and the like, and specific examples thereof include casein, gelatin, polysaccharides (starch, gum arabic, cellulose derivatives, alginic acid and the like), lignin derivatives, bentonite, sugars, synthetic water-soluble polymers (polyvinyl alcohol, polyvinyl pyrrolidone, polyacrylic acids and the like), PAP (acid isopropyl phosphate), BHT (2,6-di-tert-butyl-4-methylphenol), BHA (a mixture of 2-tert-butyl-4-methoxyphenol and 3-tert-butyl-4-methoxyphenol), vegetable oils, mineral oils, and fatty acids or esters thereof, etc.

Examples of the bait include bait components such as cereal flour, vegerable oil, sugar, crystalline cellulose and the like, antioxidants such as dibutylhydroxytoluene, nordihydroguaiaretic acid and the like, preservatives such as dehydroacetic acid and the like, agents for preventing eating by mistake by children and pets such as a red pepper powder and the like, harmful insect attractive fragrances such as a cheese fragrance, onion fragrance, peanut oil and the like.

The method of controlling pestof the present invention is a method comprising applying the inventive compound to a pest or a place where a pest inhabits.

For the method of controlling a pest of the present invention, while the inventive compound may be used as it is, the pesticidal composition of the present invention may be used as the inventive compound. The method of controlling pest of the present invention includes, for example, a method in which the pesticidal composition of the present invention is applied to a pest or a place where a pest inhabits by the same manner as for conventional pesticidal compositions, and the pest is allowed to contact with or intake the composition, and the like.

The place where a pest inhabits in the present invention includes a paddy field, a dry field, a fruit orchard, a non-cultivated field, a house, and the like.

Examples of such an application method include a spray treatment, a soil treatment, a seed treatment and a water culture medium treatment.

The above-described spray treatment generally comprises treating the plant surface or a pest itself with an active ingredient (the inventive compound or the like) to exert a controlling effect on a pest, such as foliage spraying, trunk spraying and the like.

The soil treatment generally comprises adding an active ingredient to culture soil or irrigation liquid so as to allow the active ingredient to permeate from the root part or the like of a plant to inside of the plant, thereby the plant is protected from damages by a pest. Examples of the soil treatment include planting hole treatments (planting hole spraying, soil incorporation after planting hole treatment), plant foot treatments (plant foot spraying, soil incorporation at plant foot, plant foot drenching, plant foot treatment at a later seeding raising stage), planting furrow treatments (planting furrow spraying, soil incorporation after planting furrow treatment), planting row treatments (planting row spraying, soil incorporation after planting row treatment, planting row spraying at a growing stage), planting row treatmentd at the time of sowing (planting row spraying at the time of sowing, soil incorporation after planting row treatment at the time of sowing), broadcast treatments (overall soil surface spraying, soil incorporation after broadcast treatment), other soil spraying treatments (foliar spraying of a granule at a growth stage, spraying under trunks or around main stems, soil surface spraying, soil surface incorporation, sowing hole spraying, furrow surface spraying, spraying between plants), other drenching treatments (soil drenching, drenching at a raising seedling stage, chemical injection treatment, plant foot drenching, chemical drip irrigation, chemigation), nursery box treatments (nursery box surface spraying, drenching of nursery box), nursery tray treatments (nursery tray spraying, nursery tray irrigation), nursery bed treatments (nursery bed surface spraying, drenching of nursery bed, lowland nursery bed surface spraying, seedling immersion), bed soil incorporation treatments (bed soil incorporation, presowing bed soil incorporation), and other treatments (ridging incorporation, plowing and fertilizing, surface soil incorporation, soil incorporation under canopy edge, planting position treatment, flower cluster treatment with a granule, paste fertilizer incorporation).

The above-described seed treatment generally comprises treating the seeds, seed potatoes or bulbs of a crop plant to be protected from a pest, or the vicinity thereof with an active ingredient Examples of the above-described seed treatment include spraying, smearing, immersion, impregnation, application, film coating and pellet coating.

The water culture medium treatment generally comprises adding an active ingredient to a water culture medium so as to allow the active ingredient to permeate from the root part or the like of a crop plant to inside of the plant. Examples of the above-described water culture medium treatment include water culture medium incorporation, and water culture medium interfusion.

When the inventive compound is used in the agroforestry field, its application amount is usually 0.1 to 10000 g per 1000 $m^2$. When the inventive compound is formulated into an emulsifiable concentrate, wettable powder, flowable formulation, microcapsule and the like, the formulation is generally diluted with water so that the concentration of the

inventive compound is 10 to 10000 ppm and sprayed. When the inventive compound is formulated into a granule, dust and the like, the compound is used usually as it is.

The inventive compound can be used for a foliage treatment on plants such as a crop to be protective from a pest and the like, and can also be used for a treatment on a nursery bed before planting seedlings of a crop and on planting holes and planting foots in planting. Further, it may be used for a treatment of a soil for the purpose of controlling a pest living in a soil of a cultivated land. Moreover, it is also possible that a resin formulation processed into a sheet, string or the like is wound around a crop, stretched in the vicinity of a crop and/or laid on the soil surface at the plant foot, or the like.

When the inventive compound is used for control of pests living in a house (for example, fly, mosquito, cockroach), the application amount of the inventive compound is usually 0.01 to 1000 mg per 1 $m^2$ of the application area when treated on a plane, and usually 0.01 to 500 mg per 1 $m^3$ of the application space when treated in a space. When the inventive compound is formulated into an emulsifiable concentrate, wettable powder, flowable formulation and the like, the compound is usually diluted with water so that the concentration thereof is 0.1 to 1000 ppm and applied, and in the case of an oil solution, aerosol, smoking agent, poison bait and the like, it is applied as it is.

The inventive compound can be used as an insecticide for cultivated lands such as dry fields, paddy fields, lawns, fruit orchards and the like or non-cultivated lands. The inventive compound can control pests in a cultivated land for growing crops, without causing chemical crop injuries on the following crops.

"Crops"

Agricultural crops: corn, rice, wheat, barley, rye, oat, sorghum, cotton, soybean, peanut, sarrazin, sugar beet, rapeseed, sunflower, sugar cane, tobacco, etc.;

Vegetables: Solanaceae vegetables (eggplant, tomato, green pepper, hot pepper, potato etc.), Cucurbitaceae vegetables (cucumber, pumpkin, zucchini, watermelon, melon etc.), Cruciferae vegetables (Japanese radish, turnip, horseradish, kohlrabi, Chinese cabbage, cabbage, brown mustard, broccoli, cauliflower etc.), Compositae vegetables (burdock, garland chrysanthemum, artichoke, lettuce etc.), Liliaceae vegetables (Welsh onion, onion, garlic, asparagus), Umbelliferae vegetables (carrot, parsley, celery, parsnip etc.), Chenopodiaceae vegetables (spinach, Swiss chard etc.), Labiatae vegetables (Japanese basil, mint, basil etc.), strawberry, sweat potato, yam, aroid, etc.;

Fruit trees: pomaceous fruits (apple, common pear, Japanese pear, Chinese quince, quince etc.), stone fleshy fruits (peach, plum, nectarine, Japanese plum, cherry, apricot, prune etc.), citrus plants (Satsuma mandarin, orange, lemon, lime, grapefruit etc.), nuts (chestnut, walnut, hazel nut, almond, pistachio, cashew nut, macadamia nut etc.), berry fruits (blueberry, cranberry, blackberry, raspberry etc.), grape, persimmon, olive, loquat, banana, coffee, date, coconut palm, oil palm, etc.;

Trees other than fruit trees: tea, mulberry, street trees (ash tree, birch, dogwood, eucalyptus, ginkgo, lilac, maple tree, oak, poplar, cercis, Chinese sweet gum, plane tree, zelkova, Japanese arborvitae, fir tree, Japanese hemlock, needle juniper, pine, spruce, yew, elm, Japanese horse-chestnut, etc.), Sweet viburnum, Largeleaf podocarp, Japanese cedar, Hinoki cypress, croton, Japanese Spindle, Chinese hawthorn, etc.

Lawn: zoysia (Japanese lawn grass, mascarene grass, etc.), Bermuda grass (Cynodon dactylon, etc.), bent grass (creeping bent grass, Agrostis stolonifera, Agrostis tenuis, etc.), bluegrass (Kentucky bluegrass, rough bluegrass, etc.), fescue (tall fescue, chewing fescue, creeping fescue, etc.), ryegrass (darnel, perennial ryegrass, etc.), cocksfoot, timothy grass, etc.

Oil crops: oil palm, Jatropha curcas, etc.

Biofuel crops (fuel plants) : safflower, Camelina alyssum, switch grass, Jatropha curcas, Miscanthus, reed Canary grass, Great reed, kenaf, cassava, willow, eucalyptus, algae, etc.

Others: flowering herbs (rose, carnation, chrysanthemum, Eustoma grandiflorum Shinners (prairie gentian), gypsophila, gerbera, pot marigold, salvia, petunia, verbena, tulip, aster, gentian, lily, pansy, cyclamen, orchid, lily of the valley, lavender, stock, ornamental kale, primula, poinsttia, gladiolus, cattleya, daisy, verbena, cymbidium, begonia, etc.), ornamental foliage plants, etc.

The above-described "crops" include also crops having a resistance to a herbicide such as an HPPD inhibitor such as isoxaflutole and the like, an ALS inhibitor such as imazethapyr, thifensulfuron-methyl and the like, an EPSP synthesizing enzyme inhibitor such as glyphosate and the like, a glutamine synthesizing enzyme inhibitor such as glufosinate and the like, an acetyl CoA carboxylase inhibitor such as sethoxydim and the like, a PPO inhibitor such as Flumioxazin and the like; bromoxynil, dicamba, 2, 4-D and the like, which resistance has been imparted by a classical breeding method or a gene recombination technique.

Examples of the "crops" endowed with a resistance by classical breeding methods include rapeseed, wheat, sunflower, paddy, and corn which are resistant to imidazolinone ALS-inhibiting herbicides such as imazethapyr and the like, and are already marketed under the trade name of Clearfield (registered trademark). Likewise, there is soybean endowed with a resistance to sulfonylurea ALS-inhibiting herbicides such as thifensulfuron methyl and the like by classical

breeding methods, and it is already marketed under the trade name of STS soybean. Likewise, examples of the crops endowed with a resistance to acetyl CoA carboxylase inhibitors such as trione oxime herbicides, aryloxyphenoxypropionic acid herbicides and the like by classical breeding methods include SR corn, and the like. The crops endowed with a resistance to acetyl CoA carboxylase inhibitors are described in Proceedings of the National Academy of Sciences of the United States of America (Proc. Natl. Acad. Sci. USA) vol. 87, pp. 7175 to 7179 (1990) and the like. Further, mutated acetyl CoA carboxylases which are resistant to acetyl CoA carboxylase inhibitors are reported in Weed Science, vol. 53, pp. 728 to 746 (2005) and the like, and crops which are resistant to acetyl CoA carboxylase inhibitors can be produced by introducing such a mutated acetyl CoA carboxylase gene into a crop by a gene recombination technology or introducing a mutation correlated with a resistance impartation into a crop acetyl CoA carboxylase. Further, plants which are resistant to {acetyl CoA carboxylase inhibitors/herbicides} can be produced by introducing a base substitution mutation-introduced nucleic acid typified by chimera plasty technology (Gura T. 1999. Repairing the Genome's Spelling Mistakes. Science 285: 316-318) into a crop cell thereby inducing a site-specific amino acid substitution mutation in the crop (acetyl CoA carboxylase/herbicide target) gene.

Examples of the crops endowed with a resistance by gene recombination technologies include corn, soybean, cotton, rapeseed and beet plant varieties which are resistant to glyphosate, and these are already marketed under the trade name of Round up Ready (registered trademark), Agrisure GT and the like. Likewise, there are corn, soybean, cotton and rapeseed plant varieties endowed with a resistance to glufosinate by gene recombination technologies, and these are already marketed under the trade name of Liberty Link (registered trademark) and the like. Likewise, cotton endowed with a resistance to bromoxynil by gene recombination technologies is already marketed under the trade name of BXN.

The above-described "crops" include also crops endowed with a capacity of synthesizing toxins such as Bacillus-derived selective toxins and the like, using gene recombination technologies.

Toxins expressed in such gene recombinant plants include insecticidal proteins derived from Bacillus cereus and Bacillus popilliae; δ-endotoxins such as Cry1Ab, Cry1Ac, Cry1F, Cry1Fa2, Cry2Ab, Cry3A, Cry3Bb1, Cry9C and the like derived from Bacillus thuringiensis; insecticidal proteins such as VIP1, VIP2, VIP3, VIP3A and the like; nematode-derived insecticidal proteins; toxins produced by animals such as scorpion toxin, spider toxin, bee toxin, insect-specific neurotoxins and the like; filamentous fungi toxins; plant lectin; agglutinin; protease inhibitors such as trypsin inhibitor, serine protease inhibitor, patatin, cystatin, papain inhibitor and the like; ribosome inactivation proteins (RIP) such as lysine, corn-RIP, abrin, saporin, briodin and the like; steroid metabolizing enzymes such as 3-hydroxy steroid oxidase, ecdysteroid-UDP-glucosyltransferase, cholesterol oxidase and the like; ecdysone inhibitors; HMG-CoA reductases; ion channel inhibitors such as a sodium channel inhibitor, calcium channel inhibitor and the like; juvenile hormone esterase; diuretic hormone receptor; stilbene synthase; bibenzyl synthase; chitinase; glucanase and the like.

Further, the toxins to be manifested in such gene recombinant crops include also hybrid toxins, partially deleted toxins and modified toxins of δ-endotoxin proteins such as Cry1Ab, Cry1Ac, Cry1F Cry1Fa2, Cry2Ab, Cry3A, Cry3Bb1, Cry9C or the like, and insecticidal proteins such as and VIP 1, VIP 2, VIP 3, VIP 3A or the like. A hybrid toxin is produced by a novel combination of different domains of these proteins, using a gene recombinant technology. As a partially deleted toxin, Cry1Ab in which a part of an amino acid sequence is deleted is known. As a modified toxin, there is a toxin obtained by substitution of one or more of amino acids in a natural type toxin.

Examples of these toxins and recombinant plants which are capable of synthesizing these toxins are described in EP-A-0 374 753, WO 93/07278, WO 95/34656, EP-A-0 427 529, EP-A-451878, WO 03/052073, and the like.

Toxins contained in these recombinant plants impart, particularly, a resistance to coleopteran harmful insects, dipteran harmful insects and lepidopteran harmful insects to a plant.

The gene recombinant plants which contain one or more insecticidal harmful insect-resistant genes and manifest one or more toxins are already known, and some of them are commercially available. Examples of these gene recombinant plants include YieldGard (registered trademark) (a corn cultivar expressing Cry1Ab toxin), YieldGard Rootworm (registered trademark) (a corn cultivar expressing Cry3Bb1 toxin), YieldGard Plus (registered trademark) (a corn cultivar expressing Cry1Ab and Cry3Bb1 toxins), Herculex I (registered trademark) (a corn cultivar expressing Cry1Fa2 toxin and phosphinothricin N-acetyltransferase (PAT) for imparting a resistance to gluphosinate), NuCOTN33B (registered trademark) (a cotton cultivar expressing Cry1Ac toxin), Bollgard I (registered trademark) (a cotton cultivar expressing Cry1Ac toxin), Bollgard II (registered trademark) (a cotton cultivar expressing Cry1Ac and Cry2Ab toxins), VIPCOTTM (a cotton cultivar expressing VIP toxin), NewLeaf (registered trademark) (a potato cultivar expressing Cry3A toxin), NatureGard (registered trademark) Agrisure (registered trademark) GT Advantage (GA21 glyphosate-resistant character), Agrisure (registered trademark) CB Advantage (Bt11 corn borer (CB) character), Protecta (registered trademark), and the like.

The above-described "crops" include also crops having an ability for producing anti-pathogen substances imparted using a gene recombination technology.

Examples of the anti-pathogenic substance include PR proteins (PRP, EP-A-0392225); ion channel inhibitors such as sodium channel inhibitors, calcium channel inhibitors (KP1, KP4, KP6 toxins and the like produced by viruses

are known) and the like; stilbene synthase; bibenzyl synthase; chitinase; glucanase; substances produced by microorganisms such as peptide antibiotics, antibiotics having a heterocyclic ring, protein factors correlated with plant disease resistance (described in WO 03/000906) and the like. Such anti-pathogenic substances and gene recombinant plants producing them are described in EP-A-0392225, WO 95/33818, EP-A-0353191 and the like.

The above-described "crops" include also crops endowed with useful characters such as an oil component modifying character, amino acid content enhancing character and the like, using a gene recombination technology. Examples thereof include VISTIVE (registered trademark)(low linolenic soybean having reduced linolenic acid content), high-lysine (high-oil) corn (corn having increased lysine or oil content), and the like.

Further, also included in the above-described "crops" are stacked plant varieties obtained by combination of some of the above-described classical herbicide characters or herbicide-resistant genes, insecticidal harmful insect-resistant genes, anti-pathogenic substance producing genes, useful characters such as an oil component modifying character, amino acid content enhancing character, and the like.

When the inventive compound is used for a crop having an acquired herbicide-resistance, weeds can be controlled comprehensively by a systematic treatment and/or a mixing treatment with a herbicide to which the crop is resistant (for example, glyphosate or its salt, glufosinate or its salt, dicamba or its salt, imazethapyr or its salt, isoxaflurole and the like) and the inventive compound.

The inventive compound can also be mixed or used together with other other insecticides, nematocides, acaricides, fungicides, herbicides, phytohormone agents, plant growth regulators, synergists, fertilizers, soil improving agents, animal feeds and the like.

Examples of the insecticides include:

(1) Organophosphorus compounds:

Acephate, Aluminium phosphide, butathiofos, cadusafos, chlorethoxyfos, chlorfenvinphos, chlorpyrifos, chlorpyrifos-methyl, cyanophos (CYAP), diazinon, DCIP (dichlorodiisopropyl ether), dichlofenthion (ECP), dichlorvos (DDVP), dimethoate, dimethylvinphos, disulfoton, EPN, ethion, ethoprophos, etrimfos, fenthion (MPP), fenitrothion (MEP), fosthiazate, formothion, Hydrogen phosphide, isofenphos, isoxathion, malathion, mesulfenfos, methidathion (DMTP), monocrotophos, naled (BRP), oxydeprofos (ESP), parathion, phosalone, phosmet (PMP), pirimiphos-methyl, pyridafenthion, quinalphos, phenthoate (PAP), profenofos, propaphos, prothiofos, pyraclorfos, salithion, sulprofos, tebupirimfos, temephos, tetrachlorvinphos, terbufos, thiometon, trichlorphon (DEP), vamidothion) and the like;

(2) Carbamate compounds:

Alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb (MIPC), metolcarb, methomyl, methiocarb, NAC, oxamyl, pirimicarb, propoxur (PHC), XMC, thiodicarb, xylylcarb and the like;

(3) Synthetic pyrethroid compounds:

Acrinathrin, allethrin, benfluthrin, beta-cyfluthrin, bifenthrin, cycloprothrin, cyfluthrin, cyhalothrin, cypermethrin, deltamethrin, esfenvalerate, ethofenprox, fenpropathrin, fenvalerate, flucythrinate, flufenoprox, flumethrin, fluvalinate, halfenprox, imiprothrin, permethrin, prallethrin, pyrethrins, resmethrin, sigma-cypermethrin, silafluofen, tefluthrin, tralomethrin, transfluthrin, 2,3,5,6-tetrafluoro-4-(methoxymethyl)benzyl (EZ)-(1RS,3RS;1RS,3SR)-2,2-dimethyl-3-prop-1-enylcyclopropan e carboxylate, 2,3,5,6-tetrafluoro-4-methylbenzyl (EZ)-(1RS,3RS;1RS, 3SR)-2,2-dimethyl-3-prop-1-enylcyclopropan e carboxylate, 2,3,5,6-tetrafluoro-4-(methoxymethyl)benzyl (1RS, 3RS;1RS,3SR)-2,2-dimethyl-3-(2-methyl-1-propenyl)cyclop ropane carboxylate and the like;

(4) Nereistoxin compounds:

Cartap, bensultap, thiocyclam, monosultap, bisultap, and the like;

(5) Neonicotinoid compounds:

Imidacloprid, nitenpyram, acetamiprid, thiamethoxam, thiacloprid, dinotefuran, clothianidin, and the like;

(6) Benzoylurea compounds:

Chlorfluazuron, bistrifluron, diafenthiuron, diflubenzuron, fluazuron, flucycloxuron, flufenoxuron, hexaflumuron, lufenuron, novaluron, noviflumuron, teflubenzuron, triflumuron, and the like;

(7) Phenylpyrazole compounds:

Acetoprole, ethiprole, fipronil, vaniliprole, pyriprole, pyrafluprole, and the like;

(8) Bt toxin insecticides:

Live spores derived from and crystal toxins produced from Bacillus thuringiesis and a mixture thereof;

(9) Hydrazine compounds:

Chromafenozide, halofenozide, methoxyfenozide, tebufenozide, and the like;

(10) Organochlorine compounds:

Aldrin, dieldrin, dienochlor, endosulfan, methoxychlor, and the like;

(11) Natural insecticides:

Machine oil, nicotine-sulfate;

(12) Other insecticides:

Avermectin-B, bromopropylate, buprofezin, chlorphenapyr, cyromazine, D-D (1,3-Dichloropropene), emamectin-benzoate, fenazaquin, flupyrazofos, hydroprene, indoxacarb, metoxadiazone, milbemycin-A, pymetrozine, pyridalyl, pyriproxyfen, spinosad, sulfluramid, tolfenpyrad, triazamate, flubendiamide, lepimectin, cyflumetofen, Arsenic acid, benclothiaz, Calcium cyanamide, Calcium polysulfide, chlordane, DDT, DSP, flufenerim, flonicamid, flurimfen, formetanate, metam-ammonium, metam-sodium, Methyl bromide, nidinotefuran, Potassium oleate, protrifenbute, spiromesifen, Sulfur, metaflumizone, spirotetramat, pyrifluquinazon, 3-bromo-1-(3-chloro-2-pyridyl)-4'-cyano-2'-methyl-6'-(methyl carbamoyl)pyrazole-5-carboxanilide, 3-bromo-4'-chloro-1-(3-chloro-2-pyridyl)-2'-methyl-6'-(methy lcarbamoyl)pyrazole-5-carboxanilide, tralopyril, and the like.

Examples of the acaricide (acaricidic active component) include:

Acequinocyl, amitraz, benzoximate, bifenazate, bromopropylate, chinomethionat, chlorobenzilate, CPCBS (chlorfenson), clofentezine, cyflumetofen, kelthane (dicofol), etoxazole, fenbutatin oxide, fenothiocarb, fenpyroximate, fluacrypyrim, fluproxyfen, hexythiazox, propargite (BPPS), polynactins, pyridaben, Pyrimidifen, tebufenpyrad, tetradifon, spirodiclofen, amidoflumet, cyenopyrafen, and the like.

Examples of the nematicide (nematicidal active component) include:

DCIP, fosthiazate, levamisol hydrochloride, methyisothiocyanate, morantel tartarate, and the like.

Examples of the fungicide include:

Acibenzolar-S-methyl, amobam, ampropylfos, anilazine, azoxystrobin, benalaxyl, benodanil, benomyl, benthiavalicarb, benthiazole, bethoxazin, bitertanol, blasticidin-S, Bordeaux mixture, boscalid, bromuconazole, buthiobate, calcium hypochlorite, calcium polysulfide, captan, carbendazol, carboxin, carpropamid, chlobenthiazone, chloroneb, chloropicrin, chlorothalonil (TPN), chlorthiophos, cinnamaldehyde, clozylacon, CAN (2,6-Dichloro-4-nitroaniline), copper hydroxide, copper sulfate, cyazofamid, cyfluphenamid, cymoxanil, cyproconazole, cyprodinil, cyprofuram, dazomet, debacarb, dichlofluanid, D-D (1,3-Dichloropropene), diclocymet, diclomezine, diethofencarb, difenoconazole, diflumetorim, dimefluazole, dimethirimol, dimethomorph, diniconazole-M, dinocap, edifenphos, epoxiconazole, nickel dimethyldithiocarbamate, etaconazole, ethaboxam, ethirimol, etridiazole, famoxadone, fenamidone, fenarimol, fenbuconazole, Fendazosulam, fenhexamid, fenoxanil, fenpiclonil, fenpropidin, fenpropimorph, fentiazon, triphenyltin hydroxide (fentin hydroxide), ferimzone, fluazinam, fludioxonil, flumetover, flumorph, fluoroimide, fluotrimazole, fluoxastrobin, fluquinconazole, flusilazole, flusulfamide, flutolanil, flutriafol, fosetyl-Al, fthalide, fuberidazole,

furalaxyl, furametpyr, furcarbanil, furconazole-cis, hexaconazole, hymexazol, IBP (IBP), imazalil, imibenconazole, iminoctadine-albesilate, iminoctadine-triacetate, iodocarb, ipconazole, prodione, iprovalicarb, isoprothiolane, kasugamycin, kresoxim-methyl, mancozeb, maneb, mepanipyrim, mepronil, metalaxyl, metalaxyl-M, metam-sodium, methasulfocarb, Methyl bromide, metconazole, methfuroxam, metominostrobin, metrafenone, metsulfovax, mildiomycin, milneb, myclobutanil, myclozolin, nabam, orysastrobin, ofurace, oxadixyl, oxolinic acid, oxpoconazole, oxycarboxin, oxytetracycline, pefurazoate, penconazole, pencycuron, picoxystrobin, polycarbamate, polyoxin, potassium hydrogen carbonate, probenazole, prochloraz, procymidone, propamocarb-hydrochloride, propiconaole, propineb, proquinazid, prothiocarb, prothioconazole, pyracarbolid, pyraclostrobin, pyrazophos, pyributicarb, pyrifenox, pyrimethanil, pyroquilon, quinoxyfen, quintozene (PCNB), silthiopham, simeconazole, sipconazole, sodium hydrogen carbonate (sodium bibarbonate), sodium hypochlorite, spiroxamine, SSF-129((E)-2[2-(2,5-dimethylphenoxymethyl)phenyl]-2-methoxy imino-N-methylacetamide, streptomycin, sulfur, tebuconazole, tecloftalam, tetraconazole, thiabendazole, thiadinil, thiram (TMTD), thifluzamide, thiophanate-methyl), tolclofos-methyl, TPN (TPN), triadimefon, triadimenol, triazoxide, triclamide, tricyclazole, tridemorph, triflumizole, trifloxystrobin, triforine, triticonazole, validamycin, vinclozolin, viniconazole, zineb, ziram and zoxamide.

Examples of the herbicide, phytohormone agent and plant growth regulator include:

Abscisic acid, acetochlor, acifluorfen-sodium, alachlor, alloxydim, ametryn, amicarbazone, amidosulfuron, aminoethoxyvinylglycine, aminopyralid, AC94,377, amiprofos-methyl, ancymidol, asulam, atrazine, aviglycine, azimsulfuron, beflubutamid, benfluralin, benfuresate, bensulfuron-methyl, bensulide (SAP), bentazone, benthiocarb, benzamizole, benzfendizone, benzobicyclon, benzofenap, benzyl adenine, benzylaminopurine, bialaphos, bifenox, Brassinolide, bromacil, bromobutide, butachlor, butafenacil, butamifos, butylate, cafenstrole, calcium carbonate, calcium peroxide, carbaryl, chlomethoxynil, chloridazon, chlorimuron-ethyl, chlorphthalim, chlorpropham, chlorsulfuron, chlorthal-dimethyl, chlorthiamid (DCBN), choline chloride, cinidon-ethyl, cinmethylin, cinosulfuron, clethodim, clomeprop, cloxyfonac-sodium, chlormequat chloride, 4-CPA (4-chlorophenoxyacetic acid), cliprop, clofencet, cumyluron, cyanazine, cyclanilide, cyclosulfamron, cyhalofop-butyl, 2,4-D salt (2,4-Dichlorophenoxyacetic acid salts), dichlorprop (2,4-DP), daimuron, dalapon (DPA), dimethenamid-P, daminozide, dazomet, n-Decyl alcohol, dicamba-sodium (MDBA), dichlobenil (DBN), diflufenican, dikegulac, dimepiperate, dimethametryn, dimethenamid, diquat, dithiopyr, diuron, endothal, epocholeone, esprocarb, ethephon, ethidimuron, ethoxysulfuron, ethychlozate, etobenzanid, fenarimol, fenoxaprop-ethyl, fentrazamide, flazasulfuron, florasulam, fluazifop-butyl, fluazolate, flucarbazone, flufenacet, flufenpyr, flumetralin, flumioxazin, flupropanate-sodium, flupyrsulfuron-methyl-sodium, flurprimidol, fluthiacet-methyl, foramsulfuron, forchlorfenuron, formesafen, gibberellin, glufosinate, glyphosate, halosulfuron-methyl, hexazinone, imazamox, imazapic, imazapyr, imazaquin, imazosulfuron, inabenfide, indole acetic acid (IAA), indole butyric acid, iodosulfuron, ioxynil-octanoate, isouron, isoxachlortole, isoxadifen, karbutilate, lactofen, lenacil, linuron, LGC-42153 (LGC-42153), maleic hydrazide, mecoprop (MCPP), MCP salt (2-Methyl-4-chlorophenoxyacetic acid salts), MCPA-thioethyl, MCPB (2-Methyl-4-chlorophenoxybutanoic acid ethyl ester), mefenacet, mefluidide, mepiquat, mesosulfuron, mesotrione, methyl daimuron, metamifop, metolachlor, metribuzin, metsulfuron-methyl, molinate, naphthylacetic acid, NAD (1-naphthaleneacetamide), naproanilide, napropamide, n-decyl alcohol, nicosulfuron, n-phenylphthalamic acid, orbencarb, oxadiazon, oxaziclomefone, oxine-sulfate, paclobutrazol, paraquat, pelargonic acid, pendimethalin, penoxsulam, pentoxazone, pethoxamide, phenmedipham, picloram, picolinafen, piperonyl butoxide, piperophos, pretilachlor, primisulfuron-methyl, procarbazone, prodiamine, profluazol, profoxydim, prohexadione-calcium, prohydrojasmon, prometryn, propanil, propoxycarbazone, propyzamide, pyraclonil, pyraflufen-ethyl, pyrazolate, pyrazosulfuron-ethyl, pyrazoxyfen, pyribenzoxim, pyributicarb, pyridafol, pyridate, pyriftalid, pyriminobac-methyl, pyrithiobac, quiclorac, quinoclamine, quizalofop-ethyl, rimsulfuron, sethoxydim, siduron, simazine, simetryn, sodium chlorate, sulfosulfuron, swep (MCC), tebuthiuron, tepraloxydim, terbacil, terbucarb (MBPMC), thenylchlor, thiazafluron, thidiazuron, thifensulfuron-methyl, triaziflam, tribufos, triclopyr, tridiphane, trifloxysulfuron, trifluralin, trinexapac-ethyl, tritosulfuron, uniconazole-P, vemolate (PPTC), and the like.

Examples of the synergistic include:

Ppiperonyl butoxide, sesamex, N-(2-ethylhexyl)-8,9,10-trinorborn-5-ene-2,3-dicarboxyimide (MGK 264), WARF-antiresistant, diethylmaleate, and the like.

Examples of the crop injury reducing agent include:

Benoxacor, cloquintocet-mexyl, cyometrinil, daimuron, dichlormid, fenchlorazole-ethyl, fenclorim, flurazole, fluxofenim, furilazole, mefenpyr-diethyl, MG191, naphthalic anhydride, oxabetrinil, and the like.

EXAMPLES

The present invention will be illustrated further in detail by production examples, formulation examples, test examples and the like below.

In production examples and reference production examples, data measured in a deuterated chloroform solvent using tetramethylsilane as an internal standard are shown for $^1$H-NMR unless otherwise stated, and data measured in a deuterated chloroform solvent using trichlorofluoromethane as an internal standard are shown for $^{19}$F-NMR unless otherwise stated.

First, reference production examples are shown for production intermediates of the inventive compound.

Reference Production Example 1

To 7 ml of N,N-dimethylformamide was added 0.2 g of sodium hydride (60% oily), and 0.7 g of 2-trifluoromethylphenol was added at °C, and the mixture was stirred for 15 minutes. To this was added 2-cyano-4-chloropyridine, and the mixture was stirred at 60°C for 6 hours. The reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.35 g of 4-(2-trifluoromethylphenoxy) pyridine-2-carbonitrile.

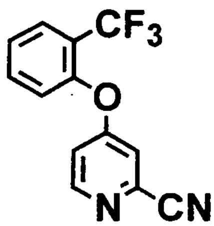

$^1$H-NMR:7.03(dd,1H),7.17(d,1H),7.20(d,1H),7.46(t,1H),7.68(t,1 H), 7.80(d, 1H), 8.57(d, 1H)

Reference Production Example 2

To 3 ml of ethanol was added 0.22 g of sodium hydrogen carbonate and 0.18 g of hydroxylamine hydrochloride, and the mixture was heated under reflux for 60 minutes. The reaction mixture was allowed to cool, then, 0.35 g of 4-(2-trifluoromethylphenoxy)pyridine-2-carbonitrile was added at 0°C, and the mixture was stirred for 3 hours and concentrated. To the resultant residue was added water and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.38 g of 4-(2-trifluoromethylphenoxy)pyridine-2-carboxamide oxime.

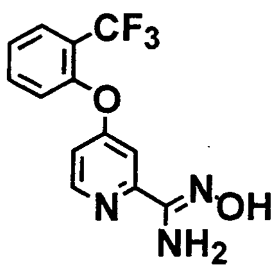

$^1$H-NMR (DMSO-d6): 5.84 (bs, 2H), 7.13 (d, 1H), 7.17 (d, 1H), 7.45 (d, 1H), 7.54 (t, 1H), 7.81 (t, 1H), 7.89 (d, 1H), 8.50 (d, 1H), 9.91 (s, 1H)

Reference Production Example 3

Into 6 ml of N,N-dimethylformamide was suspended 0.16 g of sodium hydride (60% oily), and 0.61 g of 2-trifluoromethylbenzyl alcohol was added under water cooling, and the resultant mixture was stirred for 15 minutes.

Thereafter, to the mixture was added 0.4 g of 2-cyano-4-chloropyridine, and the mixture was stirred for 2 hours. The resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.55 g of 4-(2-trifluoromethylbenzyloxy)pyridine-2-carbonitrile.

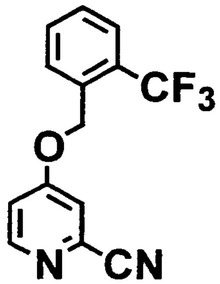

$^{1}$H-NMR:5.34 (s, 2H), 7.05 (d, 1H), 7.28 (s, 1H), 7.50 (brs, 1H), 7.62 (br s,2H), 7.75(d, 1H), 8.53(d, 1H)

Reference Production Example 4

To 4 ml of ethanol was added 0.33 g of sodium hydrogen carbonate and 0.28 g of hydroxylamine hydrochloride, and the mixture was heated under reflux for 60 minutes. The resultant reaction liquid was allowed to cool, then, 0.55 g of 4-(2-trifluoromethylbenzyloxy)pyridine-2-carbonitrile was added at 0°C and the mixture was stirred for 4 hours and concentrated. To the resultant residue was added water and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.55 g of 4-(2-trifluoromethylbenzyloxy)pyridine-2-carboxamide oxime.

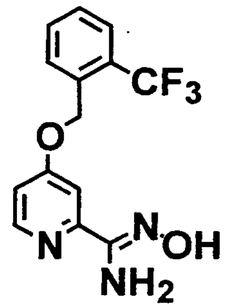

$^{1}$H-NMR (DMSO-d6): 5.36 (s, 2H), 5.82 (bs, 2H), 7.09 (dd, 1H), 7.41 (d, 1H), 7.62 (t, 1H), 7.7 2-7.79 (m, 2H), 7.82 (d, 1H), 8.41 (d, 1H), 9.89 (s, 1H)

Reference Production Example 5

To 10 ml of 1,4-dioxane was added 0.73 g of 4-chloropyridine-2-carbonitrile, 1.46 g of potassium carbonate, 0.18 g of tetrakis(triphenylphosphinepalladium) and 1 g of 3-trifluoromethylphenylboronic acid and the mixture was stirred at 80°C for 8 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.72 g of 4-(3-trifluoromethylphenyl) pyridine-2-carbonitrile.

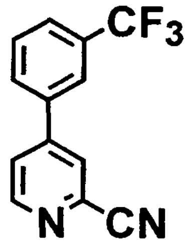

$^1$H-NMR:7.69(t, 1H), 7.74(dd, 1H), 7.79(d, 1H), 7.82(d, 1H), 7.87(s, 1 H), 7.93 (dd, 1H), 8.82 (dd, 1H)

Reference Production Example 6

To 6 ml of ethanol was added 0.49 g of sodium hydrogen carbonate and 0.4 g of hydroxylamine hydrochloride, and the mixture was heated under reflux for 60 minutes. The resultant reaction mixture was allowed to cool, then, added to 0.72 g of 4-(3-trifluoromethylphenyl)pyridine-2-carbonitrile at 0°C, and the mixture was stirred for 14 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.8 g of 4-(3-trifluoromethylphenyl)pyridine-2-carboxamide oxime.

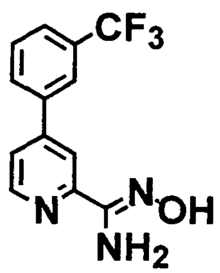

$^1$ H-NMR (DMSO-d6): 5.93 (bs, 2H), 7.79 (t, 1H), 7.84 (dd, 1H), 7.87 (d, 1H), 8.08-8.12 (m, 2H ), 8.13 (d, 1H), 8.68 (d, 1H)

Reference Production Example 7

To 10 ml of 1,4-dioxane was added 0.73 g of 4-chloropyridine-2-carbonitrile, 1.46g of potassium carbonate, 0.18 g of tetrakis(triphenylphosphinepalladium) and 1 g of 2-trifluoromethylphenylboronic acid, and the mixture was stirred at 90°C for 9 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.7 g of 4-(2-trifluoromethylphenyl)pyridine-2-carbonitrile.

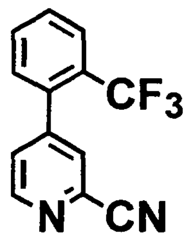

$^1$H-NMR:7.29 (d, 1H), 7.49 to 7.51 (m, 1H), 7.59-7.69 (m, 3H), 7.82 (d, 1H), 8.77 (dd, 1H)

Reference Production Example 8

To 6 ml of ethanol was added 0.47 g of sodium hydrogen carbonate and 0.39 g of hydroxylamine hydrochloride, and the mixture was heated under reflux for 60 minutes. The resultant reaction mixture was allowed to cool, then, added

to 0.70 g of 4-(2-trifluoromethylphenyl)pyridine-2-carbonitrile at 0°C, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.65 g of 4-(2-trifluoromethylphenyl)pyridine-2-carboxamide oxime.

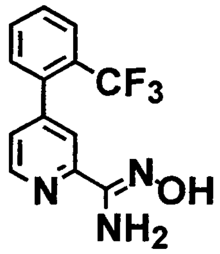

$^1$H-NMR (DMSO-d6): 5.93 (bs, 2H), 7.40 (dd, 1H), 7.49 (dd, 1H), 7.70 (t, 1H), 7.77-7.81 (m, 2 H), 7.90 (d, 1H), 8.65 (d, 1H), 10.00 (s, 1H)

Reference Production Example 9

(Step 9-1)

To 8 ml of 1,4-dioxane was added 0.61 g of 4-chloropyridine-2-carbonitrile, 1.22 g of potassium carbonate, 0.15 g of tetrakis(triphenylphosphinepalladium) and 1 g of 3-trifluoromethoxyphenylboronic acid, and the mixture was stirred at 90 °C for 10 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant coarse product was used in the subsequent reaction without purification.

(Step 9-2)

To 8 ml of ethanol was added the above-described coarse product, 0.74 g of sodium hydrogen carbonate and 0.61 g of hydroxylamine hydrochloride, and the mixture was heated under reflux for 60 minutes. The resultant reaction mixture was allowed to cool, then, the coarse product was added at 0°C and the mixture was stirred for 5 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.7 g of 4-(3-trifluoromethoxyphenyl)pyridine-2-carboxamide oxime.

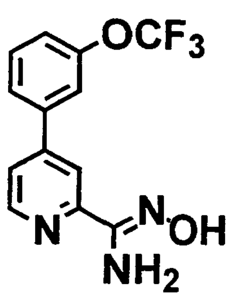

$^1$ H-NMR (DMSO-d6): 5.92 (bs, 2H), 7.51 (d, 1H), 7.68 (t, 1H), 7.77-7.80 (m, 2H), 7.84 (d, 1H) ,8.10 (dd, 1H), 8.66 (dd, 1H), 10.00 (s, 1H)

Reference Production Example 10

To 6 ml of N, N-dimethylformamide was added 1. 61 g of cesium carbonate, 0.8 g of 2-trifluoromethylaniline and 0.6 g of 4-methylsulfonylpyridine-2-carbonitrile. The mixture was stirred at 85°C for 4 hours, then, poured into a

saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.45 g of 4-(2-trifluoromethylphenylamino)pyridine-2-carbonitrile.

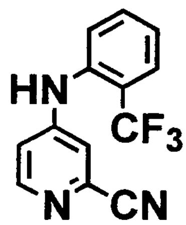

$^1$H-NMR:6.28 (s, 1H), 6.89 (dd, 1H), 7.12 (d, 1H), 7.38 (t, 1H), 7.49 (d, 1 H), 7.64 (t, 1H), 7.75 (d, 1H), 8.36 (d, 1H)

Reference Production Example 11

To 2 ml of tetrahydrofuran was added 0.06 g of sodium hydride (60% oily) and 0.3 g of 4-(2-trifluoromethylphenylamino)pyridine-2-carbonitrile. Then, 0.19 g of methyl iodide was added, and the mixture was stirred for 5 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.3 g 4-(N-2-trifluoromethylphenyl-N-methylamino)pyridine-2-carbon itrile.

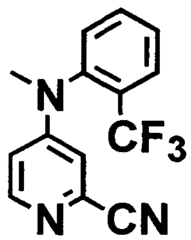

$^1$H-NMR:3.28 (s, 3H), 6.40-6.66 (m, 2H), 7.27 (d, 1H), 7.60 (t, 1H), 7.74 (td, 1H), 7.86 (d, 1H), 8.26 (s, 1H)

Reference Production Example 12

To 4 ml of ethanol was added 0.19 g of sodium hydrogen carbonate and 0.16 g of hydroxylamine hydrochloride, and the mixture was heated under reflux for 60 minutes. The resultant reaction mixture was allowed to cool, then, 0.3 g of 4-(N-2-trifluoromethylphenyl-N-methylamino)pyridine-2-carbon itrile was added at 0°C, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.3 g of 4-(N-2-trifluoromethylphenyl-N-methylamino)pyridine-2-carbox amide oxime.

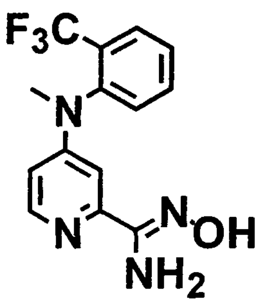

$^1$H-NMR (DMSO-d6): 3.21 (s, 3H), 5.70 (s, 2H), 6.50-6.74 (m, 2H), 7.50 (d, 1H), 7.67 (t, 1H), 7.86 (t, 1H), 7.92 (d, 1H), 8.18 (d, 1H), 9.69 (s, 1H)

Reference Production Example 13

To 6 ml of 1,4-dioxane was added 0.4 g of 4-chloropyridine-2-carbonitrile, 0.8 g of potassium carbonate, 0.1 g of tetrakis(triphenylphosphinepalladium) and 0.65 g of 2-trifluoromethoxyphenylboronic acid, and the mixture was stirred at 90 °C for 16 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.2 g of 4-(2-trifluoromethoxyphenyl) pyridine-2-carbonitrile.

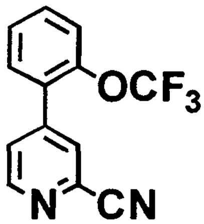

$^1$H-NMR:7.42-7.49 (m, 3H), 7.52-7.53 (m, 1H), 7.64 (dd, 1H), 7.81 (dd, 1 H), 8.79 (dd, 1H)

Reference Production Example 14

To 1 ml of ethanol was added 0.06 g of sodium hydrogen carbonate and 0.05 g of hydroxylamine hydrochloride, and the mixture was heated under reflux for 60 minutes. The resultant reaction mixture was allowed to cool, then, 0.1 g of 4-(2-trifluoromethoxyphenyl)pyridine-2-carbonitrile was added at 0°C, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.1 g of 4-(2-trifluoromethoxyphenyl)pyridine-2-carboxamide oxime.

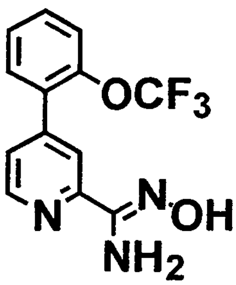

1 H-NMR (DMSO-d6): 5.92 (s, 2H), 7.52-7.65 (m, 5H), 7.50 (d, 1H), 7.96 (dd, 1H), 8.67 (dd, 1H ), 10.00 (s, 1H)

Reference Production Example 15

To 6 ml of 1,4-dioxane was added 0.5 g of 4-chloropyridine-2-carbonitrile, 1.1 g of potassium carbonate, 0.13 g of tetrakis(triphenylphosphinepalladium) and 1.12 g of 2,4-bis(trifluoromethyl)phenylboronic acid, and the mixture was stirred at 90°C for 16 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.74 g of 4-[2,4-bis(trifluoromethyl)phenyl]pyridine-2-carbonitrile.

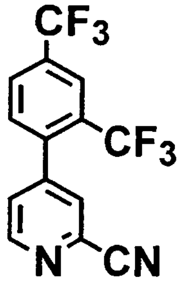

1 H-NMR:7.47 (d, 1H), 7.50 (dd, 1H), 7.67 (s, 1H), 7.95 (d, 1H), 8.09 (s, 1 H), 8.82 (dd, 1H)

Reference Production Example 16

To 4 ml of ethanol was added 0.37 g of sodium hydrogen carbonate and 0.31 g of hydroxylamine hydrochloride, and the mixture was heated under reflux for 60 minutes. The resultant reaction mixture was allowed to cool, then, 0.7 g of 4-[2,4-bis(trifluoromethyl)phenyl]pyridine-2-carbonitrile was added at room temperature and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0. 69 g of 4-[2,4-bis(trifluoromethyl)phenyl]pyridine-2-carboxamide oxime.

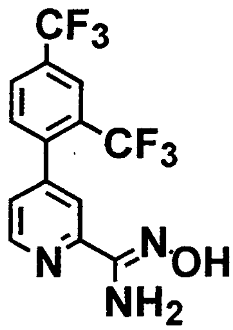

1 H-NMR (DMSO-d6): 5.94 (s, 2H), 7.45 (dd, 1H), 7.76 (d, 1H), 7.83 (s, 1H), 8.18-8.22 (m, 2H) , 8.69 (dd, 1H), 10.02 (s, 1H)

Reference Production Example 17

To 8 ml of 1,4-dioxane was added 0.6 g of 4-chloropyridine-2-carbonitrile, 1.44 g of potassium carbonate, 0.15 g of tetrakis(triphenylphosphinepalladium) and 1.1 g of 2-fluoro-5-trifluoromethylphenylboronic acid, and the mixture was stirred at 90°C for 10 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.6 g of 4-(2-fluoro-5-trifluoromethylphenyl)pyridine-2-carbonitrile.

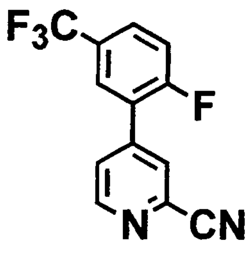

1 H-NMR:7.39 (t, 1H), 7.71-7.79 (m, 3H), 7.91 (t, 1H), 8.84 (dd, 1H)

Reference Production Example 18

To 8 ml of ethanol was added 0.63 g of sodium hydrogen carbonate and 0.52 g of hydroxylamine hydrochloride, and the mixture was heated under reflux for 60 minutes. The resultant reaction mixture was allowed to cool, then, 0.6 g of 4-(2-fluoro-5-trifluoromethylphenyl)pyridine-2-carbonitrile was added at room temperature, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.6 g of 4-(2-fluoro-5-trifluoromethylphenyl)pyridine-2-carboxamide oxime.

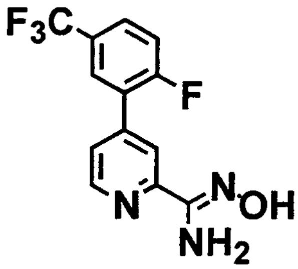

$^1$H-NMR (DMSO-d6): 5.93 (s, 2H), 7.65 (t, 1H), 7.70 (d, 1H), 7.92-7.97 (m, 1H), 8.04 (dd, 1H) , 8.06 (s, 1H), 8.70 (d, 1H), 10.02 (s, 1H)

Reference Production Example 19

To 8 ml of 1,4-dioxane was added 0.6 g of 4-chloropyridine-2-carbonitrile, 1.44 g of potassium carbonate, 0.15 g of tetrakis(triphenylphosphinepalladium) and 1.1 g of 2-fluoro-3-trifluoromethylphenylboronic acid, and the mixture was stirred at 90°C for 14 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.86 g of 4-(2-fluoro-3-trifluoromethylphenyl)pyridine-2-carbonitrile.

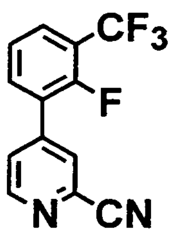

$^1$H-NMR:7.44 (t, 1H), 7.66-7.73 (m, 2H), 7.77 (t, 1H), 7.90 (s, 1H), 8.83 (dd, 1H)

Reference Production Example 20

To 5 ml of ethanol was added 0.44 g of sodium hydrogen carbonate and 0.37 g of hydroxylamine hydrochloride, and the mixture was heated under reflux for 60 minutes. The resultant reaction mixture was allowed to cool, then, 0.7 g of 4-(2-fluoro-3-trifluoromethylphenyl)pyridine-2-carbonitrile was added at room temperature, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.7 g of 4-(2-fluoro-3-trifluoromethylphenyl)pyridine-2-carboxamide oxime.

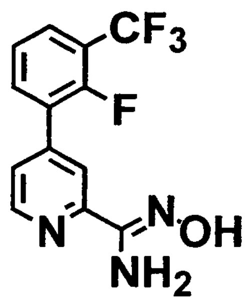

$^1$H-NMR (DMSO-d6): 5.93 (s, 2H), 7.58 (t, 1H), 7.66 (d, 1H), 7.91 (t, 1H), 8.00 (t, 1H), 8.05 ( s, 1H), 8.71 (d, 1H), 10.01 (s, 1H)

Reference Production Example 21

To 8 ml of 1,4-dioxane was added 0.53 g of 4-chloropyridine-2-carbonitrile, 1.26 g of potassium carbonate, 0.13 g of tetrakis(triphenylphosphinepalladium) and 1 g of 2-trifluoromethyl-4-methoxyphenylboronic acid, and the mixture was stirred at 90°C for 12 hours. Thereafter, the resultant reaction mixture was poured into a saturated ammonium chloride aqueous solution, and the mixture was extracted with t-butyl methyl ether three times. The organic layers obtained by extraction were combined and washed with saturated saline, dried over anhydrous magnesium sulfate, then, concentrated. The resultant residue was subjected to silica gel column chromatography to obtain 0.6 g of 4-(2-trifluoromethyl-4-methoxyphenyl)pyridine-2-carbonitrile

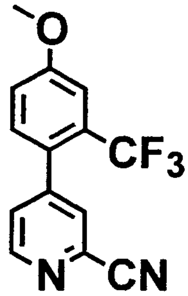

$^1$H-NMR:3.92(s, 3H), 7.15(dd, 1H), 7.22(d, 1H), 7.31(d, 1H), 7.48(d, 1 H), 7.66 (s, 1H), 8.74 (dd, 1H)

Reference Production Example 22

To 4 ml of ethanol was added 0.36 g of sodium hydrogen carbonate and 0.3 g of hydroxylamine hydrochloride, and the mixture was heated under reflux for 60 minutes. The resultant reaction mixture was allowed to cool, then, 0.6 g of 4-(2-trifluoromethyl-4-methoxyphenyl)pyridine-2-carbonitrile was added at room temperature, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.65 g of 4-(2-trifluoromethyl-4-methoxyphenyl)pyridine-2-carboxamide oxime.

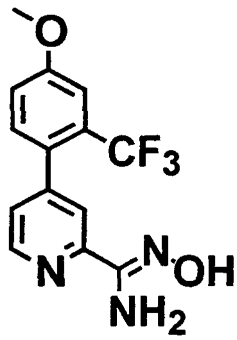

1 H-NMR (DMSO-d6): 3.89 (s, 3H), 5.91 (s, 2H), 7.32-7.37 (m, 3H), 7.42 (d, 1H), 7.78 (s, 1H), 8.62 (d, 1H), 9.98 (s, 1H)

Reference Production Example 23

To 13 ml of 1,4-dioxane was added 2 g of 2-iodo-5-chlorobenzo trifluoride, 2.15 g of potassium carbonate, 0.23 g of tetrakis(triphenylphosphinepalladium) and 0.96 g of 4-pyridineboronic acid, and the mixture was stirred at 110°C for 18 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 1.1 g of 4-(2-trifluoromethyl-4-chlorophenyl)pyridine.

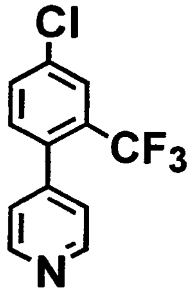

1H-NMR:7.24-7.27 (m, 3H), 7.59 (dd, 2H), 7.77 (d, 1H), 8.67 (dd, 1H)

Reference Production Example 24

To 8 ml of chloroform was added 1.1 g of 4-(2-trifluoromethyl-4-chlorophenyl)pyridine and 1.7 g of metachloroperbenzoic acid, and the mixture was stirred at room temperature for 8 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 1.2 g of 4-(2-trifluoromethyl-4-chlorophenyl)pyridine-N-oxide.

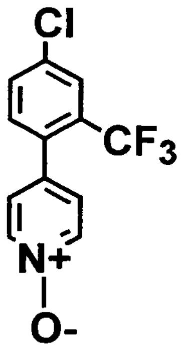

1 H-NMR:7.23 (d, 2H), 7.29 (d, 1H), 7.62 (dd, 1H), 7.78 (d, 1H), 8.25 (d, 2 H)

Reference Production Example 25

To 9 ml of acetonitrile was added 1.2 g of 4-(2-trifluoromethyl-4-chlorophenyl)pyridine-N-oxide, 1.22 ml of triethylamine and 1.75 g of trimethylsilyl cyanide, and the mixture was stirred at 90°C for 15 hours. Thereafter, the reaction liquid was allowed to cool to room temperature and concentrated. The resultant residue was subjected to silica gel column chromatography to obtain 1 g of 4-(2-trifluoromethyl-4-chlorophenyl)pyridine-2-carbonitrile.

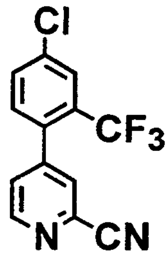

$^1$H-NMR:7.26 (s, 1H), 7.48 (d, 1H), 7.64 (d, 1H), 7.66 (s, 1H), 7.81 (d, 1H ), 8.78 (d, 1H)

Reference Production Example 26

To 7 ml of ethanol was added 0.59 g of sodium hydrogen carbonate and 0.49 g of hydroxylamine hydrochloride, and the mixture was heated under reflux for 60 minutes. The resultant reaction mixture was allowed to cool, then, 1 g of 4-(2-trifluoromethyl-4-chlorophenyl)pyridine-2-carbonitrile was added at room temperature, and the mixture was stirred for 3 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.9 g of 4-(2-trifluoromethyl-4-chlorophenyl)pyridine-2-carboxamide oxime.

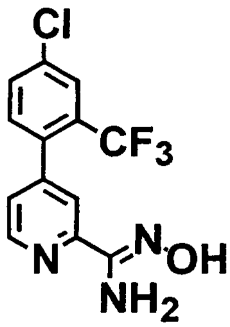

$^1$H-NMR (DMSO-d6): 5.93 (s, 2H), 7.40 (dd, 1H), 7.53 (d, 1H), 7.80 (s, 1H), 7.88 (dd, 1H), 7.9 8 (d, 1H), 8.66 (dd, 1H), 10.01 (s, 1H)

Reference Production Example 27

To 8 ml of 1,4-dioxane was added 1 g of 2-bromo-3-fluorobenzo trifluoride, 1.37 g of potassium carbonate, 0.14 g of tetrakis(triphenylphosphinepalladium) and 0.61 g of 4-pyridineboronic acid, and the mixture was stirred at 100°C for 18 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.3 g of 4-(2-trifluoromethyl-6-fluorophenyl)pyridine.

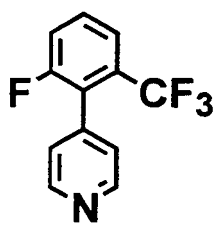

$^1$ H-NMR:7.25 (d, 2H), 7.38 (t, 1H), 7.51-7.56 (m, 1H), 7.60 (d, 1H), 8.70 (dd,2H)

Reference Production Example 28

To 3 ml of chloroform was added 0.3 g of 4-(2-trifluoromethyl-6-fluorophenyl)pyridine and 0.5 g of metachloroperbenzoic acid, and the mixture was stirred at 0°C for 2 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 0.23 g of 4-(2-trifluoromethyl-6-fluorophenyl)pyridine-N-oxide.

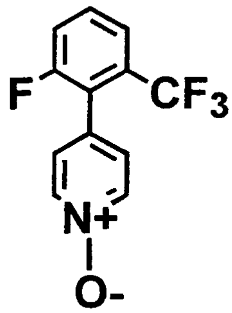

$^{1}$H-NMR:7.23 (d, 2H), 7.40 (t, 1H), 7.54-7.63 (m, 2H), 8.27-8.29 (m, 2H)

Reference Production Example 29

To 2 ml of acetonitrile was added 0.23 g of 4-(2-trifluoromethyl-6-fluorophenyl)pyridine-N-oxide, 0.25 ml of triethylamine and 0.36 g of trimethylsilyl cyanide, and the mixture was stirred at 90°C for 10 hours. Thereafter, the reaction liquid was allowed to cool to room temperature and concentrated. The resultant residue was subjected to silica gel column chromatography to obtain 0.22 g of 4-(2-trifluoromethyl-6-fluorophenyl)pyridine-2-carbonitrile.

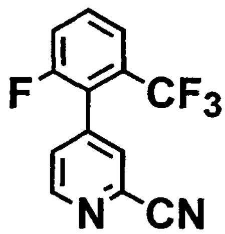

$^{1}$ H-NMR:7.43 (t, 1H), 7.49 (d, 1H), 7.58-7.67 (m, 3H), 8.82 (d, 1H)

Reference Production Example 30

To 2 ml of ethanol was added 0.14 g of sodium hydrogen carbonate and 0.12 g of hydroxylamine hydrochloride, and the mixture was heated under reflux for 60 minutes. The resultant reaction mixture was allowed to cool, then, 0.22 g of 4-(2-trifluoromethyl-6-fluorophenyl)pyridine-2-carbonitrile was adde at room temperature, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.24 g of 4-(2-trifluoromethyl-6-fluorophenyl)pyridine-2-carboxamide oxime.

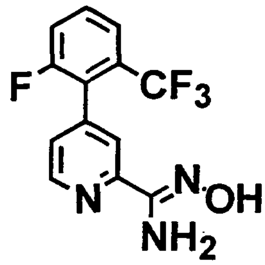

$^1$H-NMR (DMSO-d6) : 5.93 (s, 2H), 7.44 (dd, 1H), 7.71-7.79 (m, 4H), 8.68 (dd, 1H), 10.00 (s, 1 H)

Reference Production Example 31

To 9 ml of 1,4-dioxane was added 1.5 g of 2-iodo-3-chlorobenzo trifluoride, 1. 62 g of potassium carbonate, 0.17 g of tetrakis(triphenylphosphinepalladium) and 0.72 g of 4-pyridineboronic acid, and the mixture was stirred at 90°C for 4 hours and at 120°C for 10 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.5 g of 4-(2-chloro-6-trifluoromethylphenyl)pyridine.

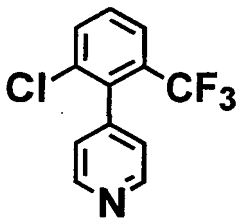

$^1$ H-NMR:7.18 (d 2H), 7.49 (t, 1H), 7.69-7.73 (m, 2H), 8.71 (d, 2H)

Reference Production Example 32

To 4 ml of chloroform was added 0.5 g of 4-(2-chloro-6-trifluoromethylphenyl)pyridine and 0.77 g of metachloroperbenzoic acid, and the mixture was stirred at 0°C for 4 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 0.4 g of 4-(2-chloro-6-trifluoromethylphenyl)pyridine-N-oxide.

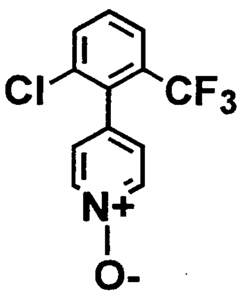

$^1$H-NMR: 7.15 (d,2H), 7.52 (t, 1H), 7.71-7.74 (m, 2H), 8.27-8.30 (m, 2H)

Reference Production Example 33

(Step 33-1)

To 3 ml of acetonitrile was added 0.4 g of 4-(2-chloro-6-trifluoromethylphenyl)pyridine-N-oxide, 0.41 ml of triethylamine and 0.44 g of trimethylsilyl cyanide, and the mixture was stirred at 90°C for 16 hours. Thereafter, the reaction liquid was allowed to cool to room temperature and concentrated. The resultant residue was used in the subsequent reaction without purification.

(Step 33-2)

To 3 ml of ethanol was added the above-described residue, 0.18 g of sodium hydrogen carbonate and 0.15 g of hydroxylamine hydrochloride, and the mixture was heated under reflux for 60 minutes. The resultant reaction mixture was allowed to cool, then, the coarse product was added at 0°C and the mixture was stirred for 2 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.45 g of 4-(2-chloro-6-trifluoromethylphenyl)pyridine-2-carboxamide oxime.

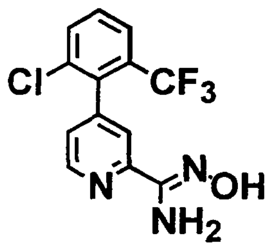

$^1$H-NMR (DMSO-d6): 5.93 (s, 2H), 7.37 (d, 1H), 7.70-7.74 (m, 2H), 7.90 (d, 1H),7.97 (d, 1H), 8.69 (d, 1H), 9.98 (s,1H)

Reference Production Example 34

To 12 ml of N,N-dimethylformamide was added 1.5 g of 2-bromo-5-fluorobenzo trifluoride, 4.82 g of cesium carbonate, 0.21 g of tetrakis(triphenylphosphinepalladium) and 0.91 g of 4-pyridineboronic acid, and the mixture was stirred at 85°C for 10 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 1.3 g of 4-(4-fluoro-6-trifluoromethylphenyl) pyridine.

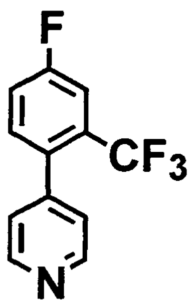

$^1$H-NMR: 7.25 (d, 2H), 7.30-7.33 (m, 2H), 7.50 (dd, 1H), 8.66-8.67 (m, 2H )

Reference Production Example 35

To 10 ml of chloroform was added 1.3 g of 4-(4-fluoro-6-trifluoromethylphenyl)pyridine and 2.2 g of metachloroperbenzoic acid, and the mixture was stirred at 0°C for 6 hours. Thereafter, the reaction liquid was poured into a saturated sodium sulfite aqueous solution, and the mixture was extracted with chloroform three times. The organic layers

obtained by extraction were combined and washed with saturated saline, further dried over anhydrous magnesium sulfate, then, concentrated. The resultant residue was subjected to silica gel column chromatography to obtain 1.3 g of 4-(4-fluoro-6-trifluoromethylphenyl)pyridine-N-oxide.

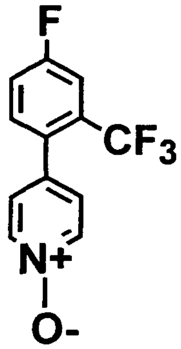

$^1$H-NMR: 7.39 (d, 2H), 7.57 (t, 1H), 7.67 (td, 1H), 7.81 (dd, 1H), 8.29-8. 30(m,2H)

Reference Production Example 36

To 11 ml of acetonitrile was added 1.3 g of 4-(4-fluoro-6-trifluoromethylphenyl)pyridine-N-oxide, 1.52 ml of triethylamine and 1.1 g of trimethylsilyl cyanide, and the mixture was stirred at 90°C for 14 hours. Thereafter, the reaction liquid was allowed to cool to room temperature and concentrated. The resultant residue was subjected to silica gel column chromatography to obtain 1.2 g of 4-(4-fluoro-6-trifluoromethylphenyl)pyridine-2-carbonitrile.

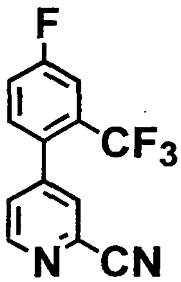

$^1$H-NMR: 7.30 (dd, 1H), 7.38 (td, 1H), 7.48 (dd, 1H), 7.54 (dd, 1H), 7.66( s, 1H), 8.78 (dd, 1H)

Reference Production Example 37

To 9 ml of ethanol was added 0.59 g of sodium hydrogen carbonate and 0.47 g of hydroxylamine hydrochloride, and the mixture was heated under reflux for 60 minutes. The resultant reaction mixture was allowed to cool, then, 1.2 g of 4-(4-fluoro-6-trifluoromethylphenyl)pyridine-2-carbonitrile was added at room temperature, and the mixture was stirred for 3 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 1.1 g of 4-(4-fluoro-6-trifluoromethylphenyl)pyridine-2-carboxamide oxime.

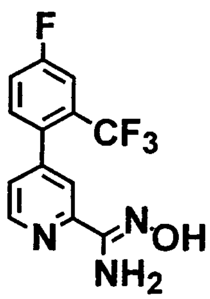

$^1$H-NMR (DMSO-d6): 5.92 (s, 2H), 7.40 (dd, 1H), 7.56 (dd, 1H), 7.67 (td, 1H), 7.79 (s, 1H), 7. 82 (dd, 1H), 8.65 (dd, 1H), 9.99 (s, 1H)

Reference Production Example 38

To 8 ml of 1,4-dioxane was added 0.6 g of 4-chloropyridine-2-carbonitrile, 1.44 g of potassium carbonate, 0.15 g of tetrakis(triphenylphosphinepalladium) and 1.1 g of 3-trifluoromethyl-4-fluorophenylboronic acid, and the mixture was stirred at 90°C for 15 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.4 g of 4-(3-trifluoromethyl-4-fluorophenyl)pyridine-2-carbonitrile.

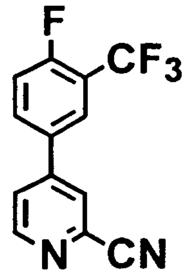

$^1$H-NMR: 7.40 (t, 1H), 7.70 (dd, 1H), 7.81-7.89 (m, 3H), 8.81 (dd, 1H)

Reference Production Example 39

To 3 ml of ethanol was added 0.19 g of sodium hydrogen carbonate and 0.16 g of hydroxylamine hydrochloride, and the mixture was heated under reflux for 60 minutes. The resultant reaction mixture was allowed to cool, then, 0.4 g of 4-(3-trifluoromethyl-4-fluorophenyl)pyridine-2-carbonitrile was added at room temperature, and the mixture was stirred for 3 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.34 g of 4-(3-trifluoromethyl-4-fluorophenyl)pyridine-2-carboxamide oxime.

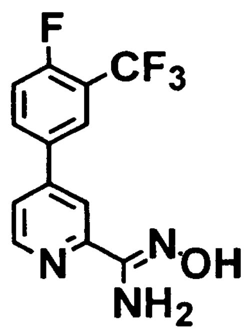

$^1$H-NMR (DMSO-d6) : 5.92 (s, 2H), 7.69 (dd, 1H), 7.82 (dd, 1H), 8.10-8.14 (m, 2H), 8.16-8.20 (m, 1H), 8.66 (dd, 1H), 9.98 (s, 1H)

Reference Production Example 40

To 12 ml of N,N-dimethylformamide was added 2 g of 2-bromo-4-fluorobenzo trifluoride, 6.44 g of cesium carbonate, 0.29 g of tetrakis(triphenylphosphinepalladium) and 1.21 g of 4-pyridineboronic acid, and the mixture was stirred at 90°C for 12 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 1.6 g of 4-(5-fluoro-2-trifluoromethylphenyl)

pyridine.

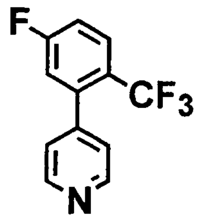

$^1$H-NMR: 7.03 (dd, 1H), 7.20-7.25 (m, 1H), 7.27 (d, 2H), 7.79 (dd, 2H), 8. 68-8.69(m,2H)

Reference Production Example 41

To 13 ml of chloroform was added 1.6 g of 4-(5-fluoro-2-trifluoromethylphenyl)pyridine and 2.64 g of metachloroperbenzoic acid, and the mixture was stirred at 0°C for 8 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 1.35 g of 4-(5-fluoro-2-trifluoromethylphenyl)pyridine-N-oxide.

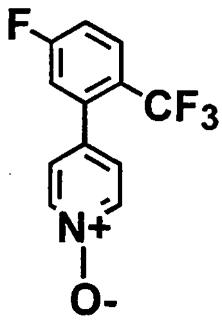

$^1$H-NMR: 7.42 (d, 2H), 7.47 (dd, 1H), 7.53 (td, 1H), 7.96 (dd, 1H), 8.30-8 .33(m,2H)

Reference Production Example 42

To 10 ml of acetonitrile was added 1.3 g of 4-(5-fluoro-2-trifluoromethylphenyl)pyridine-N-oxide, 1.41 ml of triethylamine and 1.5 g of trimethylsilyl cyanide, and the mixture was stirred at 90°C for 20 hours. Thereafter, the reaction liquid was allowed to cool to room temperature and concentrated. The resultant residue was subjected to silica gel column chromatography to obtain 1.1 g of 4-(5-fluoro-2-trifluoromethylphenyl)pyridine-2-carbonitrile.

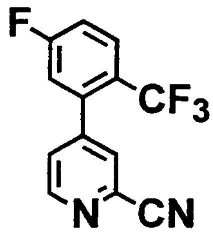

$^1$H-NMR: 7.03 (dd, 1H), 7.26-7.32 (m, 1H), 7.50 (dd, 1H), 7.68 (dd, 1H), 7 .84 (s, 1H), 8.80 (dd, 1H)

Reference Production Example 43

To 9 ml of ethanol was added 0.59 g of sodium hydrogen carbonate and 0.49 g of hydroxylamine hydrochloride, and the mixture was heated under reflux for 60 minutes. The resultant reaction mixture was allowed to cool, then, 1.2 g of 4-(5-fluoro-2-trifluoromethylphenyl)pyridine-2-carbonitrile was added at room temperature, and the mixture was

stirred for 3 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 1 g of 4-(5-fluoro-2-trifluoromethylphenyl)pyridine-2-carboxamide oxime.

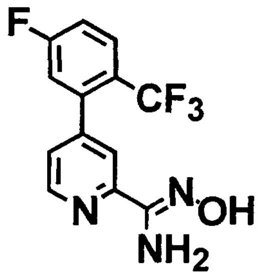

$^1$H-NMR(DMSO-d6) : 5.93 (s, 2H) , 7.41-7.47 (m, 2H) , 7.55 (td, 1H) , 7.81 (s, 1H) , 7.98 (dd, 1H ) , 8.66 (dd, 1H), 10.00 (s, 1H)

Reference Production Example 44

To 8 ml of 1,4-dioxane was added 0.5 g of 4-chloropyridine-2-carbonitrile, 1.23 g of potassium carbonate, 0.13 g of tetrakis(triphenylphosphinepalladium) and 1 g of 2-chloro-5-trifluoromethylphenylboronic acid, and the mixture was stirred at 90°C for 15 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.37 g of 4-(2-chloro-5-trifluoromethylphenyl)pyridine-2-carbonitrile.

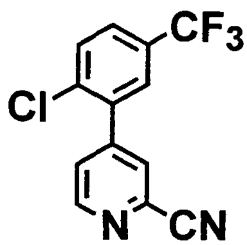

$^1$H-NMR: 7.59 (s, 1H), 7.62 (dd, 1H), 7.69 (d,2), 7.82 (dd, 1H), 8.84 (dd, 1H)

Reference Production Example 45

To 2 ml of ethanol was added 0.13 g of sodium hydrogen carbonate and 0.1 g of hydroxylamine hydrochloride, and the mixture was heated under reflux for 60 minutes. The resultant reaction mixture was allowed to cool, then, 0.28 g of 4-(2-chloro-5-trifluoromethylphenyl)pyridine-2-carbonitrile was added at room temperature, and the mixture was stirred for 3 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.3 g of 4-(2-chloro-5-trifluoromethylphenyl)pyridine-2-carboxamide oxime.

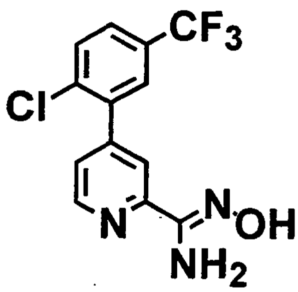

$^1$H-NMR (DMSO-d6): 5.93 (s, 2H), 7.57 (dd, 1H), 7.85-7.91 (m, 3H), 7.94 (dd, 1H), 8.70 (dd,1 H), 10.01 (s, 1H)

Reference Production Example 46

To 20 ml of N,N-dimethylformamide was added 2 g of 2-bromo-6-fluorobenzo trifluoride, 6.44 g of cesium carbonate, 0.29 g of tetrakis(triphenylphosphinepalladium) and 1.35 g of 4-pyridineboronic acid, and the mixture was stirred at 90°C for 10 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 1.4 g of 4-(3-fluoro-2-trifluoromethylphenyl) pyridine.

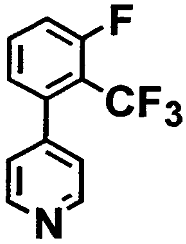

$^1$H-NMR: 7.06 (d, 1H), 7.22-7.31 (m, 3H), 7.54-7.60 (m, 1H), 8.65-8.67 ( m, 2H)

Reference Production Example 47

To 12 ml of chloroform was added 1.4 g of 4-(3-fluoro-2-trifluoromethylphenyl)pyridine and 2.31 g of metachloroperbenzoic acid, and the mixture was stirred at 0°C for 5 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 1.1 g of 4-(3-fluoro-2-trifluoromethylphenyl)pyridine-N-oxide.

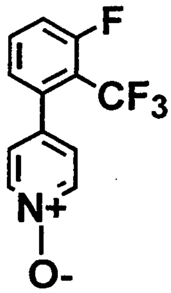

$^1$H-NMR: 7.30 (d, 1H), 7.40-7.43 (m, 2H), 7.61 (dd, 1H), 7.82 (td, 1H), 8. 28-8.31(m,2H)

Reference Production Example 48

To 9 ml of acetonitrile was added 1.1 g of 4-(3-fluoro-2-trifluoromethylphenyl)pyridine-N-oxide, 1.19 ml of triethylamine and 1.3 g of trimethylsilyl cyanide, and the mixture was stirred at 90°C for 20 hours. Thereafter, the reaction liquid was allowed to cool to room temperature and concentrated. The resultant residue was subjected to silica gel column chromatography to obtain 1 g of 4-(3-fluoro-2-trifluoromethylphenyl)pyridine-2-carbonitrile.

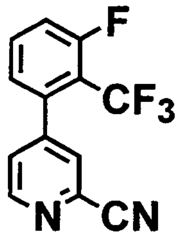

$^1$H-NMR: 7.05 (d, 1H), 7.35 (t, 1H), 7.46 (dd, 1H), 7.60-7.66 (m, 2H), 8.7 8 (dd, 1H)

Reference Production Example 49

To 7 ml of ethanol was added 0.47 g of sodium hydrogen carbonate and 0.39 g of hydroxylamine hydrochloride, and the mixture was heated under reflux for 60 minutes. The resultant reaction mixture was allowed to cool, then, 1 g of 4-(3-fluoro-2-trifluoromethylphenyl)pyridine-2-carbonitrile was added at room temperature, and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 1 g of 4-(3-fluoro-2-trifluoromethylphenyl)pyridine-2-carboxamide oxime.

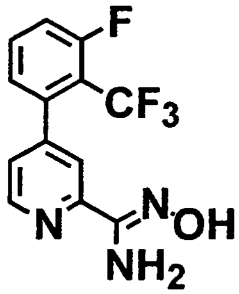

$^1$H-NMR(DMSO-d6): 5.93 (s, 2H), 7.29 (d, 1H), 7.42 (dd, 1H), 7.62 (dd, 1H), 7.77 (s, 1H), 7.8 5-7.88 (m, 1H), 8.65 (d, 1H), 10.00 (s, 1H)

Reference Production Example 50

To 13 ml of N,N-dimethylformamide was added 2 g of 1-bromo-2,5-bis(trifluoromethyl)benzene, 5.34 g of cesium carbonate, 0.24 g of tetrakis(triphenylphosphinepalladium) and 1.12 g of 4-pyridineboronic acid, and the mixture was stirred at 90°C for 13 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 1.6 g of 4-(2,5-bistrifluoromethylphenyl) pyridine.

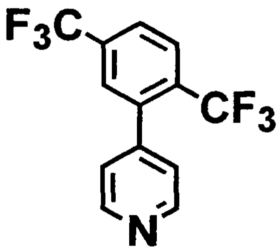

$^1$H-NMR: 7.28 (d, 2H), 7.59 (s, 1H), 7.82 (d, 1H), 7.94 (d, 1H), 8.70-8.72 (m,2H)

Reference Production Example 51

To 10 ml of chloroform was added 1.5 g of 4-(2,5-bistrifluoromethylphenyl)pyridine and 2.05 g of metachloroperbenzoic acid, and the mixture was stirred at 0°C for 6 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 1.4 g of 4-(2,5-bistrifluoromethylphenyl)pyridine-N-oxide.

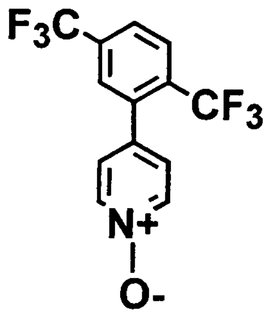

$^1$H-NMR: 7.48 (d, 2H), 7.92 (s, 1H), 8.07 (d, 1H), 8.14 (d, 1H), 8.31-8.33 (m,2H)

Reference Production Example 52

To 10 ml of acetonitrile was added 1.4 g of 4-(2,5-bistrifluoromethylphenyl)pyridine-N-oxide, 1.44 ml of triethylamine and 1.5 g of trimethylsilyl cyanide, and the mixture was stirred at 90°C for 20 hours. Thereafter, the reaction liquid was allowed to cool to room temperature and concentrated. The resultant residue was subjected to silica gel column chromatography to obtain 1.4 g of 4-(2,5-bistrifluoromethylphenyl)pyridine-2-carbonitrile.

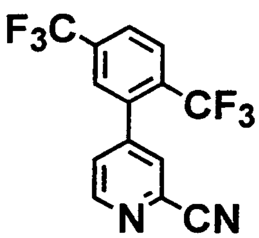

$^1$H-NMR: 7.52 (dd, 1H), 7.58 (s, 1H), 7.70 (s, 1H), 7.90 (d, 1H), 7.99 (d,1 H), 8.83 (dd, 1H)

Reference Production Example 53

To 8 ml of ethanol was added 0.56 g of sodium hydrogen carbonate and 0.46 g of hydroxylamine hydrochloride, and the mixture was heated under reflux for 60 minutes. The resultant reaction mixture was allowed to cool, then, 1.4 g of 4-(2,5-bistrifluoromethylphenyl)pyridine-2-carbonitrile was added at 0°C, and the mixture was stirred for 4 hours and concentrated. To the resultant residue was added water and the mixture was extracted with ethyl acetate three times. The organic layers obtained by extraction were combined and washed with saturated saline. The mixture was

dried over anhydrous magnesium sulfate, then, concentrated. The resultant residue was subjected to silica gel column chromatography to obtain 1.4 g of 4-(2,5-bistrifluoromethylphenyl)pyridine-2-carboxamide oxime.

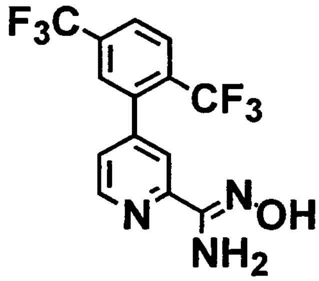

$^1$H-NMR(DMSO-d6): 5.94 (s, 2H), 7.48 (dd, 1H), 7.84 (s, 1H), 7.90 (s, 1H), 8.09 (d, 1H), 8.15 (d, 1H), 8.68 (dd, 1H), 10.01 (s, 1H)

Reference Production Example 54

To 6 ml of N,N-dimethylformamide and 0.18 ml of methanol were added 0.17 g of sodium hydride (60% oily) at 0 °C . Ten minutes after, 0.8 g of 4-(5-fluoro-2-trifluoromethylphenyl)pyridine was added, and the mixture was stirred at room temperature for 6 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.7 g of 4-(5-methoxy-2-trifluoromethylphenyl)pyridine.

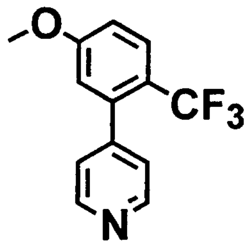

$^1$H-NMR: 3.87 (s, 3H), 6.78 (d, 1H), 7.00 (dd, 1H), 7.26-7.28 (m, 2H), 7.6 9 (d, 1H), 8.65-8.66 (m,2H)

Reference Production Example 55

To 5 ml of chloroform was added 0.7 g of 4-(5-methoxy-2-trifluoromethylphenyl)pyridine and 1.1 g of metachloroperbenzoic acid, and the mixture was stirred at 0°C for 8 hours. Thereafter, the reaction liquid was poured into a saturated sodium sulfite aqueous solution, and the mixture was extracted with chloroform three times. The organic layers obtained by extraction were combined and washed with saturated saline, further dried over anhydrous magnesium sulfate, then, concentrated. The resultant residue was subjected to silica gel column chromatography to obtain 0.6 g of 4-(5-methoxy-2-trifluoromethylphenyl)pyridine-N-oxide.

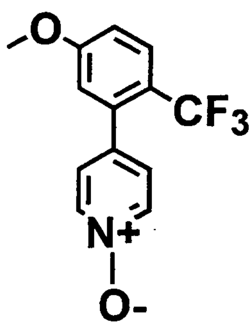

$^1$H-NMR: 3.89 (s, 3H), 6.80 (d, 1H), 7.02 (dd, 1H), 7.25-7.27 (m, 2H), 7.7 0 (d, 1H) , 8.23-8.26 (m, 2H)

Reference Production Example 56

To 6 ml of acetonitrile was added 0.6 g of 4-(5-methoxy-2-trifluoromethylphenyl)pyridine-N-oxide, 0.77 ml of triethylamine and 0.82 g of trimethylsilyl cyanide, and the mixture was stirred at 90°C for 20 hours. Thereafter, the reaction liquid was allowed to cool to room temperature and concentrated. The resultant residue was subjected to silica gel column chromatography to obtain 0.56 g of 4-(5-methoxy-2-trifluoromethylphenyl)pyridine-2-carbonitrile

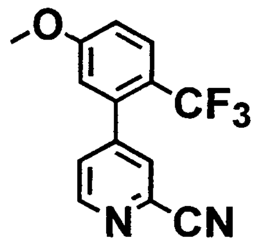

$^1$H-NMR: 3.89 (s, 3H), 6.76 (d, 1H), 7.05 (dd, 1H), 7.50 (dd, 1H), 7.68 (s, 1H), 7.73 (d, 1H), 8.76 (dd, 1H)

Reference Production Example 57

To 4 ml of ethanol was added 0.24 g of sodium hydrogen carbonate and 0.2 g of hydroxylamine hydrochloride, and the mixture was heated under reflux for 60 minutes. The resultant reaction mixture was allowed to cool, then, 0.56 g of 4-(5-methoxy-2-trifluoromethylphenyl)pyridine-2-carbonitrile was added at 0°C and the mixture was stirred for 2 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 1.4 g of 4-(5-methoxy-2-trifluoromethylphenyl)pyridine-2-carboxamide oxime.

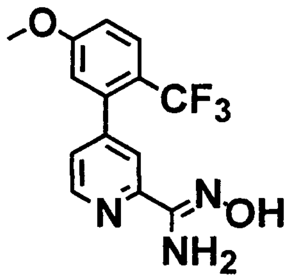

$^1$H-NMR(DMSO-d6) : 3.87 (s, 3H), 5.92 (s, 2H), 6.99 (d, 1H), 7.20 (dd, 1H), 7.40 (dd, 1H), 7.7 8-7.82 (m, 2H), 8.64 (dd, 1H), 10.00 (s, 1H)

Reference Production Example 58

(Step 58-1)

To 6 ml of 1,4-dioxane was added 0.4 g of 4-chloropyridine-2-carbonitrile, 0.8 g of potassium carbonate, 0.1 g of tetrakis(triphenylphosphinepalladium) and 0.39 g of phenylboronic acid, and the mixture was stirred at 90°C for 10 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was used in the subsequent reaction without purification.

(Step 58-2)

To 5 ml of ethanol was added 0.41 g of sodium hydrogen carbonate and 0.34 g of hydroxylamine hydrochloride, and the mixture was heated under reflux for 60 minutes. The resultant reaction mixture was allowed to cool, then, the

coarse product was added at 0°C and the mixture was stirred for 2 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.35 g of 4-phenylpyridine-2-carboxamide oxime.

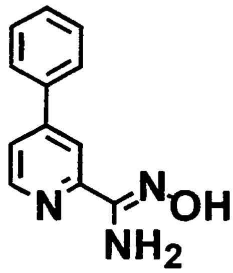

$^1$H-NMR (DMSO-d6): 5.91 (s, 2H), 7.47-7.57 (m, 3H), 7.73 (dd, 1H), 7.76-7.79 (m, 2H), 8.10 ( dd, 1H), 8.63 (dd, 1H), 9.95 (s, 1H)

Reference Production Example 59

To 6 ml of 1,4-dioxane was added 0.4 g of 4-chloropyridine-2-carbonitrile, 0.8 g of potassium carbonate, 0.1 g of tetrakis(triphenylphosphinepalladium) and 0.43 g of 2-methylphenylboronic acid, and the mixture was stirred at 90 °C for 10 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.45 g of 4-(2-methylphenyl)pyridine-2-carbonitrile.

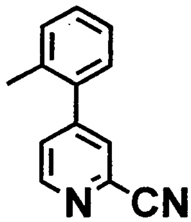

$^1$H-NMR:2.29(s, 3H), 7.19 (d, 1H), 7.30-7.39 (m, 3H), 7.49 (dd, 1H), 7.68 (dd, 1H), 8.76 (dd, 1H)

Reference Production Example 60

To 5 ml of ethanol was added 0.39 g of sodium hydrogen carbonate and 0.32 g of hydroxylamine hydrochloride, and the mixture was heated under reflux for 60 minutes. The resultant reaction mixture was allowed to cool, then, 0.45 g of 4-(2-methylphenyl)pyridine-2-carbonitrile was added at 0°C and the mixture was stirred for 5 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.28 g of 4-(2-methylphenyl)pyridine-2-carboxamide oxime.

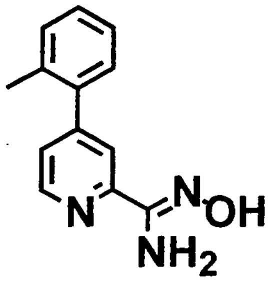

$^1$H-NMR (DMSO-d6): 5.91 (s, 2H), 7.25-7.36 (m, 4H), 7.43 (dd, 1H), 7.78 (t, 1H), 8.62 (dd, 1H ), 9.95 (s, 1H)

Reference Production Example 61

To 6 ml of 1,4-dioxane was added 0.4 g of 4-chloropyridine-2-carbonitrile, 0.8 g of potassium carbonate, 0.1 g of tetrakis(triphenylphosphinepalladium) and 0.44 g of 2-fluorophenylboronic acid, and the mixture was stirred at 90°C for 8 hours. Thereafter, the resultant reaction mixture was poured into a saturated ammonium chloride aqueous solution, and the mixture was extracted with ethyl acetate three times. The organic layers obtained by extraction were combined and washed with saturated saline, dried over anhydrous magnesium sulfate, then, concentrated. The resultant residue was subjected to silica gel column chromatography to obtain 0.3 g of 4-(2-fluorophenyl)pyridine-2-carbonitrile.

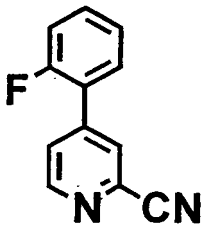

$^1$H-NMR: 7.22-7.27 (m, 1H), 7.31 (t, 1H), 7.46-7.52 (m, 2H), 7.70-7.72 ( m, 1H), 7.91 (bs, 1H), 8.78 (dd, 1H)

Reference Production Example 62

To 3 ml of ethanol was added 0.26 g of sodium hydrogen carbonate and 0.21 g of hydroxylamine hydrochloride, and the mixture was heated under reflux for 60 minutes. The resultant reaction mixture was allowed to cool, then, 0.3 g of 4-(2-fluorophenyl)pyridine-2-carbonitrile was added at 0°C and the mixture was stirred for 4 hours and concentrated. To the resultant residue was added water and the mixture was extracted with ethyl acetate three times. The organic layers obtained by extraction were combined and washed with saturated saline. The mixture was dried over anhydrous magnesium sulfate, then, concentrated. The resultant residue was subjected to silica gel column chromatography to obtain 0.3 g of 4-(2-fluorophenyl)pyridine-2-carboxamide oxime.

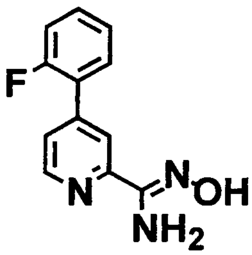

$^1$H-NMR (DMSO-d6): 5.91 (s, 2H), 7.35-7.42 (m, 2H), 7.51-7.57 (m, 1H), 7.62 (dt, 1H), 7.66 ( td, 1H), 8.04 (t, 1H), 8.66 (dd, 1H), 9.98 (s, 1H)

Next, production examples of the inventive compound will be shown.

Production Example 1

According to the procedure of Reference Production Method 2, 4-(2-trifluoromethylphenoxy)pyridine-2-carboxamide oxime was prepared.

Zero point four grams (0.4 g) of the above-described 4-(2-trifluoromethylphenoxy)pyridine-2-carboxamide oxime and 0.28 g of 1,1'-carbonyldiimidazole were added to 2.5 ml of tetrahydrofuran, and the mixture was stirred at room temperature for 1 hour. Thereafter, to the resultant mixture was added 0.27 g of 1,8-diazabicyclo[5,4,0]undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.4 g of 3-[4-(2-trifluoromethylphenoxy)pyridin-2-yl]-1,2,4-oxadiazol -5-one (hereinafter, referred to as the inventive compound (1)).

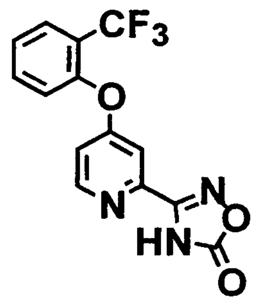

Inventive compound (1)

$^1$H-NMR (DMSO-d6): 7.26 (dd, 1H), 7.39 (d, 1H), 7.50 (d, 1H), 7.57 (t, 1H), 7.84 (t, 1H), 7.92 (d, 1H), 8.67 (d, 1H)

Production Example 2

According to the procedure of Reference Production Example 4, 4-(2-trifluoromethylbenzyloxy)pyridine-2-carboxamide oxime was prepared.

Zero point five five grams (0.55 g) of the above-described 4-(2-trifluoromethylbenzyloxy)pyridine-2-carboxamide oxime and 0.37 g of 1,1'-carbonyldiimidazole were added to 3 ml of tetrahydrofuran, and the mixture was stirred at room temperature for 1 hour. Thereafter, to the resultant mixture was added 0.35 g of 1,8-diazabicyclo[5,4,0]undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.56 g of 3-[4-(2-trifluoromethylbenzyloxy)pyridin-2-yl]-1,2,4-oxadiaz ol-5-one (hereinafter, referred to as the inventive compound (2)).

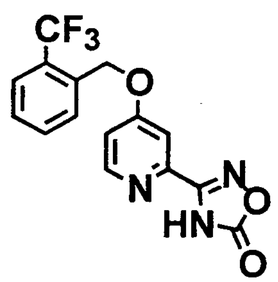

Inventive compound (2)

$^1$H-NMR (DMSO-d6): 5.43 (s, 2H), 7.32 (dd, 1H), 7.57 (d, 1H), 7.64 (t, 1H), 7.74-7.85 (m, 3H) , 8.60 (d, 1H), 13.14 (brs, 1H)

Production Example 3

According to Reference Production Example 4, 4-(3-trifluoromethylphenoxy)pyridine-2-carboxamide oxime was prepared from 4-(3-trifluoromethylphenoxy)pyridine-2-carbonitrile.

Zero point four three grams (0.43 g) of the above-described 4-(3-trifluoromethylphenoxy)pyridine-2-carboxamide oxime and 0.31 g of 1,1'-carbonyldiimidazole were added to 3 ml of tetrahydrofuran, and the mixture was stirred at room temperature for 1 hour. Thereafter, to the resultant mixture was added 0.29 g of 1,8-diazabicyclo[5,4,0]undec-7-ene, and the mixture was stirred for 2 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.42 g of 3-[4-(3-trifluoromethylphenoxy)pyridin-2-yl]-1,2,4-oxadiazol -5-one (hereinafter, referred to as the inventive compound (3)) .

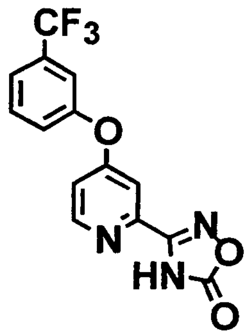

Inventive compound (3)

$^1$H-NMR (DMSO-d6): 7.25 (dd, 1H), 7.44 (d, 1H), 7.61 (d, 1H), 7.71-7.79 (m, 3H), 8.67 (d, 1H) , 13.21 (brs, 1H)

Production Example 4

According to the procedure of Reference Production Example 6, 4-(3-trifluoromethylphenyl)pyridine-2-carboxamide oxime was prepared.

Zero point eight grams (0.8 g) of the above-described 4-(3-trifluoromethylphenyl)pyridine-2-carboxamide oxime and 0.65 g of 1,1'-carbonyldiimidazole were added to 5.6 ml of tetrahydrofuran, and the mixture was stirred at room temperature for 1 hour. Thereafter, to the resultant mixture was added 0.61 g of 1,8-diazabicyclo[5,4,0]undec-7-ene, and the mixture was stirred for 5 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.72 g of 3-[4-(3-trifluoromethylphenyl)pyridin-2-yl]-1,2,4-oxadiazol-5-one (hereinafter, referred to as the inventive compound (4)).

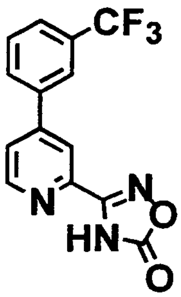

Inventive compound (4)

$^1$H-NMR (DMSO-d6): 7.82 (t, 1H), 7.91 (d, 1H), 8.10 (dd, 1H), 8.22 (d, 1H), 8.24 (s, 1H), 8.33 (dd, 1H), 8.86 (dd, 1H), 13.29 (bs, 1H)

Production Example 5

According to the procedure of Reference Production Example 8, 4-(2-trifluoromethylphenyl)pyridine-2-carboxamide oxime was prepared.

Zero point six five grams (0.65 g) of the above-described 4-(2-trifluoromethylphenyl)pyridine-2-carboxamide oxime and 0.53 g of 1,1'-carbonyldiimidazole were added to 5 ml of tetrahydrofuran, and the mixture was stirred at room temperature for 2 hours. Thereafter, to the resultant mixture was added 0.49 g of 1,8-diazabicyclo[5,4,0]undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.65 g of 3-[4-(2-trifluoromethylphenyl)pyridin-2-yl]-1,2,4-oxadiazol-5-one (hereinafter, referred to as the inventive compound (5)).

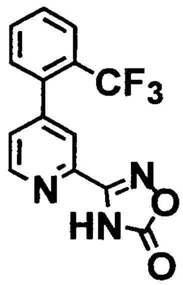

Inventive compound (5)

$^1$H-NMR (DMSO-d6): 7.54 (d, 1H) ,7.67 (dd, 1H) ,7.74 (t, 1H) ,7.83 (t, 1H), 7.91-7.95 (m, 2H) ,8.86 (dd, 1H), 13.26 (bs, 1H)

Production Example 6

According to the procedure of Reference Production Example 9 (Step 9-2), 4-(3-trifluoromethoxyphenyl)pyridine-2-carboxamide oxime was prepared.

Zero point seven grams (0.7 g) of the above-described 4-(3-trifluoromethoxyphenyl)pyridine-2-carboxamide oxime and 0.54 g of 1,1'-carbonyldiimidazole were added to 5 ml of tetrahydrofuran, and the mixture was stirred at room temperature for 2 hours. Thereafter, to the resultant mixture was added 0.5 g of 1,8-diazabicyclo[5,4,0]undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.64 g of 3-[4-(3-trifluoromethoxyphenyl)pyridin-2-yl]-1,2,4-oxadiazol -5-one (hereinafter, referred to as the inventive compound (6)).

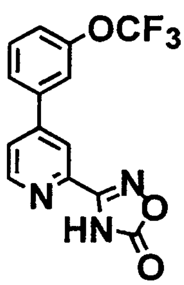

Inventive compound (6)

$^1$H-NMR (DMSO-d6): 7.55 (d, 1H), 7.72 (dd, 1H), 7.93-7.97 (m, 2H), 8.05 (dd, 1H), 8.29 (d, 1H ), 8.85 (d, 1H), 13.23 (bs, 1H)

Production Example 7

According to the procedure of Reference Production Example 14, 4-(2-trifluoromethoxyphenyl)pyridine-2-carboxamide oxime was prepared.

Zero point one gram (0.1 g) of the above-described 4-(2-trifluoromethoxyphenyl)pyridine-2-carboxamide oxime and 0.09 g of 1,1'-carbonyldiimidazole were added to 1 ml of tetrahydrofuran, and the mixture was stirred at room temperature for 1 hour and 20 minutes. Thereafter, to the resultant mixture was added 0.08 g of 1,8-diazabicyclo[5,4,0] undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.08 g of 3-[4-(2-trifluoromethoxyphenyl)pyridin-2-yl]-1,2,4-oxadiazol -5-one (hereinafter, referred to as the inventive compound (7)).

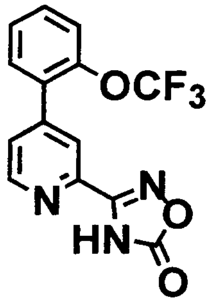

Inventive compound (7)

$^1$H-NMR (DMSO-d6): 7.58-7.63 (m, 2H), 7.65-7.74 (m, 2H), 7.81 (dd, 1H), 8.07 (dd, (dd, 1H), 13.26 (bs, 1H)

Production Example 8

According to the procedure of Reference Production Example 16, 4-[2,4-bis(trifluoromethyl)phenyl]pyridine-2-carboxamide oxime was prepared.

Zero point six nine grams (0.69 g) of the above-described 4-[2,4-bis(trifluoromethyl)phenyl)pyridine-2-carboxamide oxime and 0.45 g of 1,1'-carbonyldiimidazole were added to 4 ml of tetrahydrofuran, and the mixture was stirred at room temperature for 2 hours. Thereafter, to the resultant mixture was added 0.42 g of 1,8-diazabicyclo [5,4,0] undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.65 g of 3-{4-[2,4-bis(trifluoromethyl)phenyl]pyridin-2-yl}-1,2,4-oxa diazol-5-one (hereinafter, referred to as the inventive compound (8)).

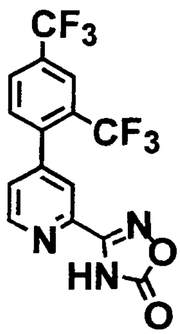

Inventive compound (8)

$^1$H-NMR (DMSO-d6): 7.72 (d, 1H), 7.80 (d, 1H), 7.99 (s, 1H), 8.22-8.25 (m, 2H), 8.89 (d, 1H), 13.30 (bs, 1H)

Production Example 9

According to the procedure of Production Example 8, the inventive compound (8) was prepared.

Zero point three grams (0.3 g) of the above-described inventive compound (8) and 0.18 g of 1,8-diazabicyclo [5,4,0]undec-7-ene, were added to 2 ml of pyridine, and under room temperature, 0.15 g of 2,2-dimethylbutanoyl chloride was added, and the mixture was stirred for 6 hours, then, concentrated. The resultant residue was subjected to silica gel column chromatography to obtain 0.11 g of 4-(2,2-dimethylbutanoyl)-3-{4-[2,4-bis(trifluoromethyl)pheny 1]pyridin-2-yl}-1,2,4-oxadiazol-5-one (hereinafter, referred to as the inventive compound (9)).

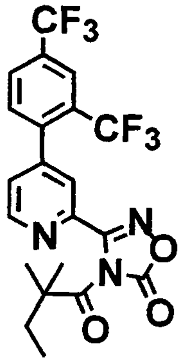

Inventive compound (9)

$^1$H-NMR: 1.03 (t, 3H), 1.44 (s, 6H), 1.95 (q, 2H), 7.43 (d, 1H), 7.50 (d, 1H ), 7.94 (d, 1H), 7.96 (s, 1H), 8.08 (s, 1H), 8.66 (dd, 1H)

Production Example 10

According to the procedure of Reference Production Example 18, 4-(2-fluoro5-trifluoromethylphenyl)pyridine-2-carboxamide oxime was prepared.

Zero point six grams (0.6 g) of the above-described 4-(2-fluoro-5-trifluoromethylphenyl)pyridine-2-carboxamide oxime and 0. 46 g of 1,1'-carbonyldiimidazole were added to 4 ml of tetrahydrofuran, and the mixture was stirred at room temperature for 2 hours. Thereafter, to the resultant mixture was added 0. 43 g of 1, 8-diazabicyclo [5, 4, 0] undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.54 g of 3-[4-(2-fluoro-5-trifluoromethylphenyl)pyridin-2-yl]-1,2,4-o xadiazol-5-one (hereinafter, referred to as the inventive compound (10)).

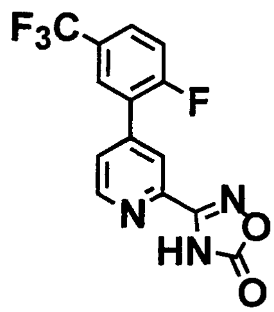

Inventive compound (10)

$^1$H-NMR (DMSO-d6): 7.69 (t, 1H), 7.95-8.00 (m, 2H), 8.14 (d, 1H), 8.22 (s, 1H), 8.89 (d, 1H), 13.27 (bs, 1H)

Production Example 11

According to the procedure of Reference Production Example 20,

4-(2-fluoro-3-trifluoromethylphenyl)pyridine-2-carboxamide oxime was prepared.

Zero point seven grams (0.7 g) of the above-described 4-(2-fluoro-3-trifluoromethylphenyl)pyridine-2-carboxamide oxime and 0.53 g of 1,1' -carbonyldiimidazole were added to 5 ml of tetrahydrofuran, and the mixture was stirred at room temperature for 2 hours. Thereafter, to the resultant mixture was added 0.5 g of 1,8-diazabicyclo [5, 4, 0] undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.6 g of 3-[4-(2-fluoro-3-trifluoromethylphenyl)pyridin-2-yl]-1,2,4-o xadiazol-5-one (hereinafter, referred to as the inventive compound (11)).

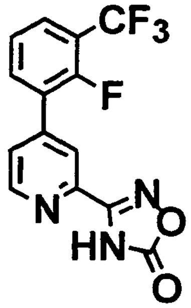

Inventive compound (11)

$^1$H-NMR (DMSO-d6): 7.61 (t, 1H), 7.91-7.98 (m, 2H), 8.07 (t, 1H), 8.19 (s, 1H), 8.91 (dd, 1H), 13.29 (bs, 1H)

Production Example 12

According to the procedure of Reference Production Example 22,
4-(2-trifluoromethyl-4-methoxyphenyl)pyridine-2-carboxamide oxime was prepared.

Zero point six five grams (0.65 g) of the above-described 4-(2-trifluoromethyl-4-methoxyphenyl)pyridine-2-carboxamide oxime and 0.49 g of 1,1'-carbonyldiimidazole were added to 4 ml of tetrahydrofuran, and the mixture was stirred at room temperature for 2 hours. Thereafter, to the resultant mixture was added 0.46 g of 1,8-diazabicyclo[5,4,0] undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.61 g of 3-[4-(2-trifluoromethyl-4-methoxyphenyl)pyridin-2-yl]-1,2,4-oxadiazol-5-one (hereinafter, referred to as the inventive compound (12)).

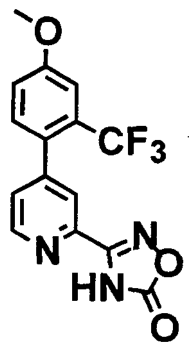

Inventive compound (12)

1H-NMR (DMSO-d6): 3.91 (s,3H), 7.37-7.39 (m, 2H), 7.48 (d, 1H), 7.63 (d 1H), 7.89 (s, 1H), 8.83 (d, 1H), 13.27 (bs, 1H)

Production Example 13

According to the procedure of Reference Production Example 26,

4-(2-trifluoromethyl-4-chlorophenyl)pyridine-2-carboxamide oxime was prepared.

Zero point nine grams (0.9 g) of the above-described 4-(2-trifluoromethyl-4-chlorophenyl)pyridine-2-carboxamide oxime and 0.8 g of 1,1'-carbonyldiimidazole were added to 7 ml of tetrahydrofuran, and the mixture was stirred at room temperature for 2 hours. Thereafter, to the resultant mixture was added 0. 76 g of 1,8-diazabicyclo[5,4,0]undec-7-ene, and the mixture was stirred for 5 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.7 g of 3-[4-(2-trifluoromethyl-4-chlorophenyl)pyridin-2-yl]-1,2,4-o xadiazol-5-one (hereinafter, referred to as the inventive compound (13)).

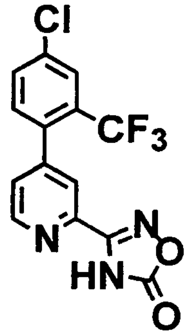

Inventive compound (13)

$^1$H-NMR (DMSO-d6): 7.58 (d, 1H), 7.67 (dd, 1H), 7.91-7.94 (m, 2H), 8.02 (d 1H), 8.87 (dd, 1H), 13.28 (bs, 1H)

Production Example 14

According to the procedure of Reference Production Example 30,
4-(2-trifluoromethyl-6-fluorophenyl)pyridine-2-carboxamide oxime was prepared.

Zero point two four grams (0.24 g) of the above-described 4-(2-trifluoromethyl-6-fluorophenyl)pyridine-2-carboxamide oxime and 0.18 g of 1,1'-carbonyldiimidazole were added to 2 ml of tetrahydrofuran, and the mixture was stirred at room temperature for 2 hours. Thereafter, to the resultant mixture was added 0.76 g of 1,8-diazabicyclo [5,4,0] undec-7-ene at 0°C, then, the mixture was stirred at room temperature for 1 hour. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.2 g of 3-[4-(2-trifluoromethyl-6-fluorophenyl)pyridin-2-yl]-1,2,4-o xadiazol-5-one (hereinafter, referred to as the inventive compound (14)).

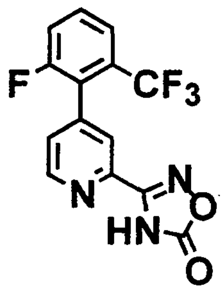

Inventive compound (14)

$^1$H-NMR (DMSO-d6): 7.72-7.83 (m, 4H), 8.00 (s, 1H), 8.90 (dd, 1H), 13.27 (bs, 1H)

Production Example 15

According to the procedure of Reference Production Example 33, Step (33-2), 4-(2-chloro-6-trifluoromethylphenyl)pyridine-2-carboxamide oxime was prepared.

Zero point four five grams (0.45 g) of the above-described 4-(2-chloro-6-trifluoromethylphenyl)pyridine-2-carboxamide oxime and 0.32 g of 1,1'-carbonyldiimidazole were added to 3 ml of tetrahydrofuran, and the mixture was stirred at room temperature for 2 hours. Thereafter, to the resultant mixture was added 0.3 g of 1,8-diazabicyclo[5,4,0]undec-7-ene at 0°C, then, the mixture was stirred at room temperature for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.38 g of 3-[4-(2-chloro-6-trifluoromethylphenyl)pyridin-2-yl]-1,2,4-o xadiazol-5-one (hereinafter, referred to as the inventive compound (15)).

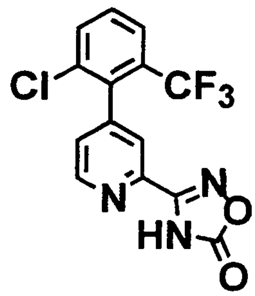

Inventive compound (15)

$^1$H-NMR (DMSO-d6) : 7.67 (d, 1H), 7.76 (t, 1H), 7.92-7.95 (m, 2H), 8.00 (d, 1H), 8.91 (d, 1H), 13.28 (bs, 1H)

Production Example 16

According to the procedure of Reference Production Example 37, 4-(4-fluoro-6-trifluoromethylphenyl)pyridine-2-carboxamide oxime was prepared.

One point one gram (1.1 g) of the above-described 4-(4-fluoro-6-trifluoromethylphenyl)pyridine-2-carboxamide oxime and 0.84 g of 1,1'-carbonyldiimidazole were added to 7 ml of tetrahydrofuran, and the mixture was stirred for 1.5 hours at room temperature. Thereafter, to the resultant mixture was added 0.78 g of 1, 8-diazabicyclo[5,4,0]undec-7-ene at 0°C, then, the mixture was stirred at room temperature for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 1 g of 3-[4-(4-fluoro-6-trifluoromethylphenyl)pyridin-2-yl]-1,2,4-o xadiazol-5-one (hereinafter, referred to as the inventive compound (16)).

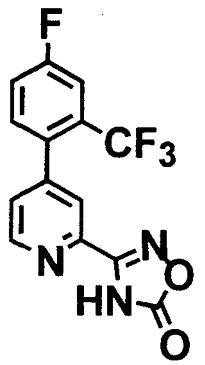

Inventive compound (16)

$^1$H-NMR (DMSO-d6): 7.62 (dd, 1H), 7.66 (dd, 1H), 7.72 (td, 1H), 7.86 (dd, 1H), 7.93 (s, 1H), 8 .86 (dd, 1H), 13.27 (bs, 1H)

Production Example 17

According to the procedure of Reference Production Example 39,

4-(3-trifluoromethyl-4-fluorophenyl)pyridine-2-carboxamide oxime was prepared.

Zero point three four grams (0.34 g) of the above-described 4-(3-trifluoromethyl-4-fluorophenyl)pyridine-2-carboxamide oxime and 0.26 g of 1,1'-carbonyldiimidazole were added to 2 ml of tetrahydrofuran, and the mixture was stirred at room temperature for 2 hours. Thereafter, to the resultant mixture was added 0.24 g of 1,8-diazabicyclo[5,4,0]undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.3 g of 3-[4-(3-trifluoromethyl-4-fluorophenyl)pyridin-2-yl]-1,2,4-o xadiazol-5-one (hereinafter, referred to as the inventive compound (17)).

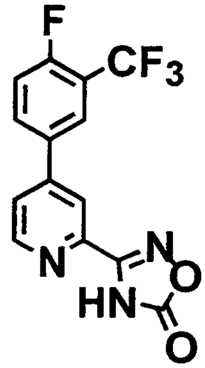

Inventive compound (17)

$^1$H-NMR (DMSO-d6): 7.73 (dd, 1H), 8.08 (dd, 1H), 8.27-8.33 (m, 3H), 8.85 (dd, 1H)

Production Example 18

According to the procedure of Reference Production Example 43,
4-(5-fluoro-2-trifluoromethylphenyl)pyridine-2-carboxamide oxime was prepared.

One gram (1 g) of the above-described 4-(5-fluoro-2-trifluoromethylphenyl)pyridine-2-carboxamide oxime and 1.21 g of 1,1'-carbonyldiimidazole were added to 10 ml of tetrahydrofuran, and the mixture was stirred at room temperature for 2 hours. Thereafter, to the resultant mixture was added 1.2 g of 1,8-diazabicyclo[5,4,0]undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.9 g of 3-[4-(5-fluoro-2-trifluoromethylphenyl)pyridin-2-yl]-1,2,4-o xadiazol-5-one (hereinafter, referred to as the inventive compound (18)).

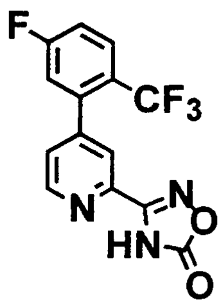

Inventive compound (18)

$^1$H-NMR (DMSO-d6): 7.52 (dd, 1H), 7.59 (td, 1H), 7.69 (dd, 1H), 7.96 (s, 1H), 8.01 (dd, 1H), 8 .87(dd, 1H), 13.29 (bs, 1H)

Production Example 19

According to the procedure of Reference Production Example 45, 4-(2-chloro-5-trifluoromethylphenyl)pyridine-2-carboxamide oxime was prepared.

Zero point three grams (0.3 g) of the above-described 4-(2-chloro-5-trifluoromethylphenyl)pyridine-2-carboxamide oxime and 0.22 g of 1,1'-carbonyldiimidazole were added to 2 ml of tetrahydrofuran, and the mixture was stirred at room temperature for 2 hours. Thereafter, to the resultant mixture was added 0.2 g of 1,8-diazabicyclo[5,4,0]undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.28 g of 3-[4-(2-chloro-5-trifluoromethylphenyl)pyridin-2-yl]-1,2,4-o xadiazol-5-one (hereinafter, referred to as the inventive compound (19)).

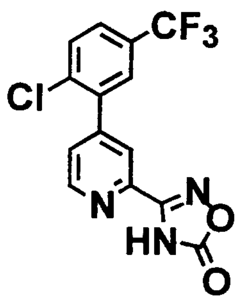

Inventive compound (19)

$^1$H-NMR (DMSO-d6) : 7.84 (dd, 1H), 7.92 (s, 2H), 7.96 (s, 1H), 8.10 (dd, 1H), 8.90 (dd, 1H), 13 . 26 (bs, 1H)

Production Example 20

According to the procedure of Reference Production Example 49, 4-(3-fluoro-2-trifluoromethylphenyl)pyridine-2-carboxamide oxime was prepared.

One point three grams (1.3 g) of the above-described 4-(3-fluoro-2-trifluoromethylphenyl)pyridine-2-carboxamide oxime and 0.99 g of 1,1'-carbonyldiimidazole were added to 9 ml of tetrahydrofuran, and the mixture was stirred at room temperature for 4 hours. Thereafter, to the resultant mixture was added 0.93 g of 1,8-diazabicyclo[5,4,0]undec-7-ene, and the mixture was stirred for 5 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.95 g of 3-[4-(3-fluoro-2-trifluoromethylphenyl)pyridin-2-yl]-1,2,4-o xadiazol-5-one (hereinafter, referred to as the inventive compound (20)).

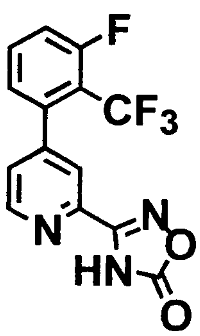

Inventive compound (20)

$^1$H-NMR (DMSO-d6): 7.33 (d, 1H), 7.64-7.70 (m, 2H), 7.83-7.90 (m, 1H), 7.95 (s, 1H), 8.85 (d ,1H), 13.28 (bs, 1H)

Production Example 21

According to the procedure of Reference Production Example 53, 4-(2,5-bistrifluoromethylphenyl)pyridine-2-carboxamide oxime was prepared.

One point four grams (1.4 g) of the above-described 4-(2,5-bistrifluoromethylphenyl)pyridine-2-carboxamide oxime and 1 g of 1,1'-carbonyldiimidazole were added to 8 ml of tetrahydrofuran, and the mixture was stirred for 4 hours and 30 minutes at room temperature. Thereafter, to the resultant mixture was added 0.94 g of 1,8-diazabicyclo[5,4,0] undec-7-ene, and the mixture was stirred for 5 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 1.3 g of 3-[4-(2,5-bistrifluoromethylphenyl)pyridin-2-yl]-1,2,4-oxadi azol-5-one (hereinafter, referred to as the inventive compound (21)).

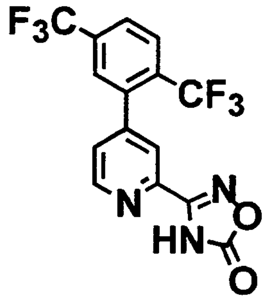

Inventive compound (21)

$^1$H-NMR (DMSO-d6): 7.74 (dd, 1H), 7.97 (s, 1H), 8.02 (s, 1H), 8.13 (d, 1H), 8.18 (d, 1H), 8.89 (dd, 1H), 13.28 (bs, 1H)

Production Example 22

According to the procedure of Reference Production Example 57, 4-(5-methoxy-2-trifluoromethylphenyl)pyridine-2-carboxamide oxime was prepared.

Zero point five grams (0.5 g) of the above-described 4-(5-methoxy-2-trifluoromethylphenyl)pyridine-2-carboxamide oxime and 0.37 g of 1,1'-carbonyldiimidazole were added to 3 ml of tetrahydrofuran, and the mixture was stirred for 3 hours at room temperature. Thereafter, to the resultant mixture was added 0.34 g of 1,8-diazabicyclo[5,4,0]undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.5 g of 3-[4-(5-methoxy-2-trifluoromethylphenyl)pyridin-2-yl]-1,2,4-oxadiazol-5-one (hereinafter, referred to as the inventive compound (22)).

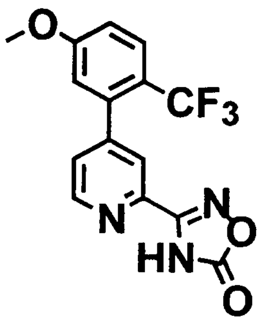

Inventive compound (22)

$^1$H-NMR (DMSO-d6): 3.88 (s, 3H), 7.06 (d, 1H), 7.24 (dd, 1H), 7.66 (dd, 1H), 7.83 (d, 1H), 7.9 2 (s, 1H), 8.85

(dd, 1H), 13.25 (bs, 1H)

Production Example 23

According to the procedure of Reference Production Example 62, 4-(2-fluorophenyl)pyridine-2-carboxamide oxime was prepared.

Zero point three nine grams (0.39 g) of the above-described 4-(2-fluorophenyl)pyridine-2-carboxamide oxime and 0.34 g of 1,1'-carbonyldiimidazole were added to 3 ml of tetrahydrofuran, and the mixture was stirred at room temperature for 2 hours. Thereafter, to the resultant mixture was added 0.32 g of 1,8-diazabicyclo[5,4,0]undec-7-ene, and the mixture was stirred for 6 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.31 g of 3-[4-(2-fluorophenyl)pyridin-2-yl]-1,2,4-oxadiazol-5-one (hereinafter, referred to as the inventive compound (23)).

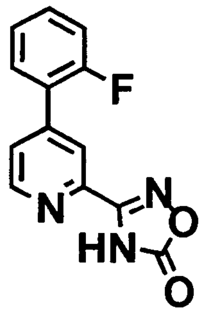

Inventive compound (23)

$^1$H-NMR (DMSO-d6): 7.39-7.46 (m, 2H), 7.56-7.62 (m, 1H), 7.75 (td, 1H), 7.88 (dt, 1H), 8.14 (s, 1H), 8.86 (dd, 1H), 13.25 (bs, 1H)

Production Example 24

According to the procedure of Reference Production Example 60, 4-(2-methylphenyl)pyridine-2-carboxamide oxime was prepared.

Zero point two eight grams (0.28 g) of the above-described 4-(2-methylphenyl)pyridine-2-carboxamide oxime and 0.28 g of 1,1'-carbonyldiimidazole were added to 3 ml of tetrahydrofuran, and the mixture was stirred for 3 hours at room temperature. Thereafter, to the resultant mixture was added 0.26 g of 1,8-diazabicyclo[5,4,0]undec-7-ene, and the mixture was stirred for 4 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.26 g of 3-[4-(2-methylphenyl) pyridin-2-yl]-1,2,4-oxadiazol-5-one (hereinafter, referred to as the inventive compound (24)).

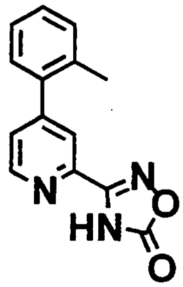

Inventive compound (24)

$^1$H-NMR (DMSO-d6): 2.29 (s, 3H), 7.31-7.42 (m, 4H), 7.70 (dd, 1H), 7.91 (dd, 1H), 8.82 (dd,1 H), 13.22 (bs, 1H)

Production Example 25

According to the procedure of Reference Production Example 58, Step (58-2), 4-phenylpyridine-2-carboxamide oxime was prepared.

Zero point two grams (0.2 g) of the above-described 4-phenylpyridine-2-carboxamide oxime and 0.55 g of 1,1'-carbonyldiimidazole were added to 5 ml of tetrahydrofuran, and the mixture was stirred at room temperature for 2 hours. Thereafter, to the resultant mixture was added 0.52 g of 1,8-diazabicyclo[5,4,0]undec-7-ene, and the mixture was stirred for 3 hours. To the reaction solution was added water and 10% HCl, and the mixture was extracted with ethyl acetate three times. The organic layers obtained by extraction were combined and dried over anhydrous magnesium sulfate, then, concentrated. The resultant residue was subjected to silica gel column chromatography to obtain 0.24 g of 3-(4-phenylpyridin-2-yl)-1,2,4-oxadiazol-5-one (hereinafter, referred to as the inventive compound (25)).

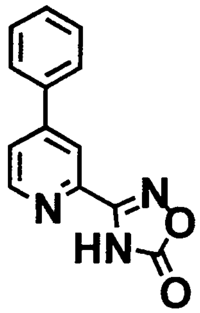

Inventive compound (25)

$^1$H-NMR (DMSO-d6): 7.54-7.60 (m, 3H), 7.88-7.91 (m, 2H), 8.00 (dd, 1H), 8.24 (dd, 1H), 8.82 (dd, 1H)

Next, formulation examples will be shown. Here, parts are by weight.

Formulation Example 1

Each 10 parts of the inventive compounds (1) to (25) were dissolved in a mixture composed of 35 parts of xylene and 35 parts of N,N-dimethylformamide, to the resultant mixtures were added 14 parts of polyoxyethylene styryl phenyl ether and 6 parts of calcium dodecylbenzenesulfonate, and the mixtures were thoroughly stirred and mixed, to obtain 10% emulsifiable concentrates.

Formulation Example 2

Each 20 parts of the inventive compounds (1) to (25) were added to a mixture of 4 parts of sodium lauryl sulfate, 2 parts of calcium ligninsulfonate, 20 parts of synthetic hydrated silicon oxide fine powder and 54 parts of diatomaceous earth, and the mixtures were thoroughly stirred and mixed, to obtain 20% wettable powders.

Formulation Example 3

To each 2 parts of the inventive compounds (1) to (25) were added 1 part of synthetic hydrated silicon oxide fine powder, 2 parts of calcium ligninsulfonate, 30 parts of bentonite and 65 parts of kaolin clay, and the mixtures were stirred and mixed sufficiently. Then, a suitable amount of water was added to the mixtures, and the mixtures were further stirred, and granulated by a granulator, and dried under ventilation to obtain 2% granules.

Formulation Example 4

Each 1 part of the inventive compounds (1) to (25) were dissolved in a suitable amount of acetone, and 5 parts of synthetic hydrated silicon oxide fine powder, 0.3 parts of 2- (2-pyridylazo) phenol [PAP] and 93. 7 parts of Fubasami clay were added to them, and the mixtures were stirred and mixed sufficiently, and acetone was removed by distillation to obtain 1% powders.

Formulation Example 5

Each 10 parts of the inventive compounds (1) to (25); 35 parts of a mixture of polyoxyethylene alkyl ether sulfate ammonium salt and white carbon (weight ratio=1:1); and 55 parts of water were mixed, and finely ground by a wet gring method, to obtain 10% flowable formulations.

Formulation Example 6

Each 0.1 part of the inventive compounds (1) to (25) were dissolved in 5 parts of xylene and 5 parts of trichloroethane, and these were mixed with 89.9 parts of deodorized kerosene, to obtain 0.1% oil solutions.

Formulation Example 7

Each 10 mg of the inventive compounds (1) to (25) were dissolved in 0.5 ml of acetone, and the solutions were used to treat 5 g of an animal solid feedstuff powder (breeding-propagation solid feedstuff powder CE-2, manufactured by CLEA Japan Inc.),, and these were mixed uniformly. Then, acetone was dried by distillation, to obtain poison baits.

Formulation Example 8

Each 5 parts of the inventive compounds (1) to (25) were dissolved in 80 parts of diethylene glycol monoethyl ether, and these were mixed with 15 parts of propylene carbonate, to obtain spot-on liquids.

Formulation Example 9

Each 10 parts of the inventive compounds (1) to (25) were dissolved in 70 parts of diethylene glycol monoethyl ether, and these were mixed with 20 parts of 2-octyldodecanol, to obtain pour-on liquids.

Formulation Example 10

To each 0.5 parts of the inventive compounds (1) to (25) were added 60 parts of NIKKOL TEALS-42 (42% aqueous solution of triethanolamine lauryl sulfate, manufactured by Nikko Chemicals Co., Ltd.) and 20 parts of propylene glycol, and these were stirred and mixed sufficiently to give uniform solutions, then, 19.5 parts of water was added and the mixtures were further stirred and mixed sufficiently, to obtain shampoos in the form of a uniform solution.

Formulation Example 11

Each 0.1 part of the inventive compounds (1) to (25) were dissolved in 2 ml of propylene glycol, and the solutions were impregnated into porous ceramic plates of 4.0×4.0 cm, thickness: 1.2 cm, to obtain heat mode smoking agents.

Formulation Example 12

Each 5 parts of the inventive compounds (1) to (25) and 95 parts of an ethylene-methyl methacrylate copolymer (the proportion of methyl methacrylate in the copolymer = 10 wt%, Acrift WD301, manufactured by Sumitomo Chemical Co., Ltd.) were melt-kneaded in a closed pressure kneader (manufactured by Moriyama Manufacturing Co., Ltd.), and the resultant kneaded materials were extruded through a molding dice from an extrusion molding machine, to obtain rod-shaped molded bodies of a length of 15 cm and a diameter of 3 mm.

Formulation Example 13

Each 5 parts of the inventive compounds (1) to (25) and 95 parts of a soft vinyl chloride resin were melt-kneaded in a closed pressure kneader (manufactured by Moriyama Manufacturing Co., Ltd.), and the resultant kneaded materials were extruded through a molding dice from an extrusion molding machine, to obtain rod-shaped molded bodies of a length of 15 cm and a diameter of 3 mm.

Next, the noxious arthropod controlling effect of the inventive compound is shown by test examples.

Test Example 1

The inventive compounds (6), (9), (11), (13), (14), (15) and (17) to (23) were formulated according to Formulation

Example 5. These formulations were diluted with water so that the inventive compound concentration was 500 ppm, to prepare spray solutions for test.

Cucumber was planted on a plastic cup and grown until spreading of the first true leaf, and parasitized by about 30 insects of Aphis gossypii. One day after, any of the above-described spray solutions for test was sprayed each in a proportion of 20 ml/cup on the cucumber. Six days after spraying, the number of Aphis gossypii was checked, and the control value was calculated according to the following formula.

$$\text{Control value (\%)} = \{1-(Cb \times Tai)/(Cai \times Tb)\} \times 100$$

Letters in the formula represent the following meanings.

Cb: the insect number before treatment on non-treated district
Cai: the insect number in observation on non-treated district
Tb: the insect number before treatment on treated district
Tai: the insect number in observation on treated district

As a result, the control value was 90% or more in the districts treated with the spray solutions for test containing any of the above-described inventive compounds.

Test Example 2

The inventive compounds (1), (4), (5), (6), (11), (12), (14), (15), (16) and (19) to (22) were formulated according to Formulation Example 1 described above. These formulations were diluted with water so that the inventive compound concentration was 500 ppm, to prepare spray solutions for test.

About sixty Tetranychus urticae female adult insects were released on Phaseolus vulgaris seedlings (seven days after sowing, primary leaf spreading stage) planted on a plastic cup, and left for one day. Each 30 ml of any of the above-described spray solutions for test was sprayed on the seedlings. Eight days and thirteen days after spraying, the number of living acarine on the Phaseolus vulgaris leaves was checked, and the control ratio was calculated according to the following formula.

$$\text{Control ratio (\%)} = 100 \times \{1-(\text{living acarine number on treated district})/(\text{living acarine number on non-treated district})\}$$

As a result, the control ratio was 90% or more on 8 days and 13 days after the treatment in the districts treated with the spray solutions for test containing the above-described inventive compound.

Reference Test Example

A compound represented by the following formula (A) (described in Japanese Patent Application National Publication (Laid-Open) No. 2001-520666. Hereinafter, referred to as comparative compound (A)) and a compound represented by the following formula (B) (described in JP-A No. 2002-205991. Hereinafter, referred to as comparative compound (B)):

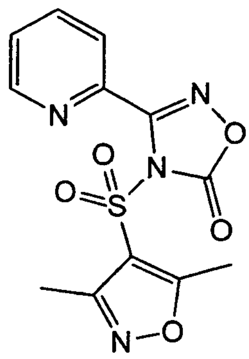

(A)

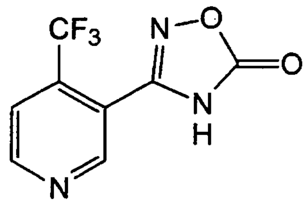

(B)

were tested under the same conditions as in Test Example 2. The control value was less than 30% for the comparative compound (A) and the comparative compound (B).

Industrial Applicability

The inventive compound has an excellent effect for control of a pest, thus, the inventive compound is useful as an active ingredient of a pesticidal composition.

## Claims

**1.** A pyridine compound represented by general formula (I):

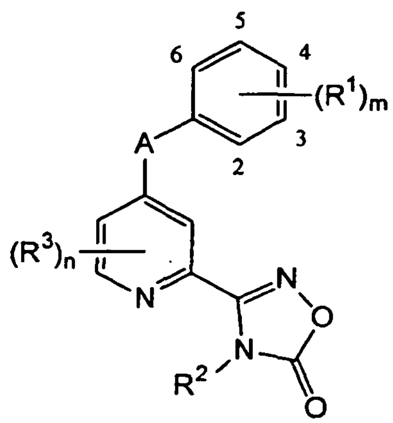

(I)

[wherein, $R^1$ represents a C1-C7 alkyl group optionally substituted by halogen, a C1-C7 alkoxy group optionally substituted by halogen, a C1-C7 alkylthio group optionally substituted by halogen, a nitro group, a cyano group or halogen,
m represents an integer of 1 to 5,
A represents a single bond, an oxygen atom, a sulfur atom, $NR^{10}$, $CH_2$ or $CH_2O$,
$R^{10}$ represents a C1-C7 alkyl group optionally substituted by halogen, a C3-C7 alkenyl group optionally substituted by halogen, a C3-C7 alkynyl group optionally substituted by halogen, a C2-C7 alkoxyalkyl group, a cyanomethyl group or hydrogen,
$R^2$ represents a C1-C7 alkyl group optionally substituted by halogen, a cyanomethyl group, a (C3-C7 cycloalkyl) methyl group optionally substituted by one or more members selected from Group $\alpha$, a benzyl group optionally substituted by one or more members selected from Group $\beta$ or hydrogen, alternatively represents any one group selected from the following $Q^1$, $Q^2$, $Q^3$, $Q^4$ and $Q^5$

$Q^1$= 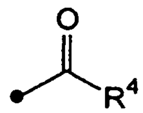 $Q^2$= 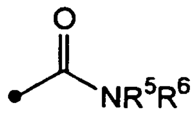

$Q^3$= 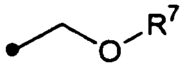 $Q^4$= 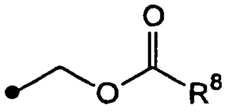 $Q^5$= 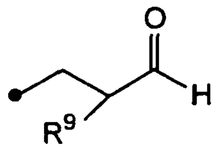

(wherein, $R^4$ represents hydrogen, a C1-C7 alkyl group optionally substituted by halogen or a C3-C7 cycloalkyl group optionally substituted by one or more members selected from Group α,
$R^5$ and $R^6$ are the same or mutually different and represent a C1-C7 alkyl group optionally substituted by halogen, a C3-C7 alkenyl group optionally substituted by halogen, a C1-C7 alkoxy group optionally substituted by halogen or a C3-C7 cycloalkyl group optionally substituted by one or more members selected from Group α, alternatively $R^5$, $R^6$ and the nitrogen atom to which they are bonding represent a pyrrolidin-1-yl group optionally substituted by one or more members selected from Group α, a piperidino group optionally substituted by one or more members selected from Group α, a hexamethyleneimin-1-yl group optionally substituted by one or more members selected from Group α, a morpholino group optionally substituted by one or more members selected from Group α or a thiomorpholin-4-yl group optionally substituted by one or more members selected from Group α,
$R^7$ represents a C1-C7 alkyl group optionally substituted by halogen, a phenyl group optionally substituted by one or more members selected from Group β, a benzyl group optionally substituted by one or more members selected from Group β or a C3-C7 cycloalkyl group optionally substituted by one or more members selected from Group α,
$R^8$ represents a C1-C7 alkyl group optionally substituted by halogen, a phenyl group optionally substituted by one or more members selected from Group β or a C3-C7 cycloalkyl group optionally substituted by one or more members selected from Group α,
$R^9$ represents a C1-C7 alkyl group optionally substituted by halogen, or hydrogen;
$R^3$ represents a C1-C7 alkyl group optionally substituted by halogen, a C1-C7 alkoxy group optionally substituted by halogen, a C3-C7 cycloalkyl group optionally substituted by one or more members selected from Group α, a C3-C7 cycloalkyloxy group optionally substituted by one or more members selected from Group α, or halogen, and
n represents an integer of 0 to 3;
Group α: a group consisting of halogen, C1-C7 alkyl groups and C1-C7 haloalkyl groups.
Group β: a group consisting of halogen, a cyano group, a nitro group, C1-C7 alkyl groups, C1-C7 haloalkyl groups, C1-C7 alkoxy groups and C1-C7 haloalkoxy groups.

**2.** The pyridine compound according to Claim 1, wherein at least one $R^1$ is a C1-C7 haloalkyl group or a C1-C7 haloalkoxyl group.

**3.** The pyridine compound according to Claim 1, wherein at least one $R^1$ is a C1-C3 fluoroalkyl group or a C1-C3 fluoroalkoxy group.

**4.** The pyridine compound according to Claim 2 or 3 wherein at least one $R^1$ is a substituent at the 2-position or 3-position on the benzene ring.

**5.** The pyridine compound according to Claim 1, wherein n is 0.

**6.** The pyridine compound according to Claim 1, wherein $R^2$ is hydrogen.

**7.** A pesticidal composition comprising as an active ingredient the pyridine compound according to Claim 1.

**8.** A method of controlling pest comprising applying an effective amount of the pyridine compound according to Claim 1 to a pest or a place where a pest inhabits; excepting that the place where the pest inhabits is human or animal.

## Patentansprüche

1. Pyridinverbindung, dargestellt durch die allgemeine Formel (I):

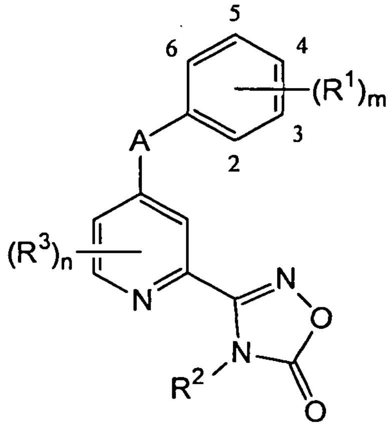

(I)

wobei $R^1$ für eine C1-C7 Alkylgruppe, die optional mit Halogen substituiert ist, eine C1-C7 Alkoxygruppe, die optional mit Halogen substituiert ist, eine C1-C7 Alkylthiogruppe, die optional mit Halogen substituiert ist, eine Nitrogruppe, eine Cyanogruppe oder Halogen steht,
m für eine ganze Zahl von 1 bis 5 steht,
A für eine Einfachbindung, ein Sauerstoffatom, ein Schwefelatom, $NR^{10}$, $CH_2$ oder $CH_2$ O steht,
$R^{10}$ für eine C1-C7 Alkylgruppe, die optional mit Halogen substituiert ist, eine C3-C7 Alkenylgruppe, die optional mit Halogen substituiert ist, eine C3-C7 Alkynylgruppe, die optional mit Halogen substituiert ist, eine C2-C7 Alkoxyalkylgruppe, eine Cyanomethylgruppe oder Wasserstoff steht,
$R^2$ für eine C1-C7 Alkylgruppe, die optional mit Halogen substituiert ist, eine Cyanomethylgruppe, eine (C3-C7 Cycloalkyl)methylgruppe, die optional mit einem oder mehreren der Gruppe $\alpha$ substituiert ist, eine Benzylgruppe, die optional mit einem oder mehreren der Gruppe $\beta$ substituiert ist, oder Wasserstoff steht, oder alternativ für irgend eine Gruppe aus den folgenden $Q^1$, $Q^2$, $Q^3$, $Q^4$ und $Q^5$ steht

$Q^1$= 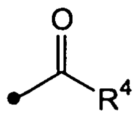 $Q^2$= 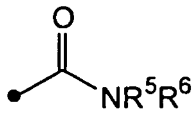

$Q^3$= 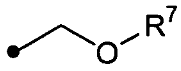 $Q^4$= 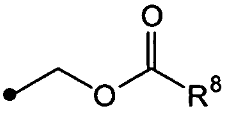 $Q^5$= 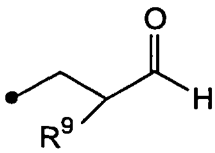

wobei $R^4$ für Wasserstoff, eine C1-C7 Alkylgruppe, die optional mit Halogen substituiert ist, oder eine C3-C7 Cycloalkylgruppe, die optional mit einem oder mehreren der Gruppe $\alpha$ substituiert ist, steht,
$R^5$ und $R^6$ gleich oder wechselseitig verschieden sind und für eine C1-C7 Alkylgruppe, die optional mit Halogen substituiert ist, eine C3-C7 Alkenylgruppe, die optional mit Halogen substituiert ist, eine C1-C7 Alkoxygruppe, die optional mit Halogen substituiert ist, oder eine C3-C7 Cycloalkylgruppe, die optional mit einem oder mehreren der Gruppe $\alpha$ substituiert ist, stehen, oder alternativ
$R^5$, $R^6$ und das Stickstoffatom, an das sie binden, für eine Pyrrolidin-1-ylgruppe, die optional mit einem oder mehreren der Gruppe $\alpha$ substituiert ist, eine Piperidingruppe, die optional mit einem oder mehreren der Gruppe $\alpha$ substituiert ist, eine Hexamethylenimin-1-ylgruppe, die optional mit einem oder mehreren der Gruppe $\alpha$ substituiert ist, eine Morpholingruppe, die optional mit einem oder mehreren der Gruppe $\alpha$ substituiert ist, oder eine Thiomorpholin-4-ylgruppe, die optional mit einem oder mehreren der Gruppe $\alpha$ substituiert ist, stehen,
$R^7$ für eine C1-C7 Alkylgruppe, die optional mit Halogen substituiert ist, eine Phenylgruppe, die optional mit einem oder mehreren der Gruppe $\beta$ substituiert ist, eine Benzylgruppe, die optional mit einem oder mehreren der Gruppe

β substituiert ist, oder eine C3-C7 Cycloalkylgruppe, die optional mit einem oder mehreren der Gruppe α substituiert ist, steht,
$R^8$ für eine C1-C7 Alkylgruppe, die optional mit Halogen substituiert ist, eine Phenylgruppe, die optional mit einem oder mehreren der Gruppe β substituiert ist, oder eine C3-C7 Cycloalkylgruppe, die optional mit einem oder mehreren der Gruppe α substituiert ist, steht,
$R^9$ für eine C1-C7 Alkylgruppe, die optional mit Halogen substituiert ist, oder Wasserstoff, steht;
$R^3$ für eine C1-C7 Alkylgruppe, die optional mit Halogen substituiert ist, eine C1-C7 Alkoxygruppe, die optional mit Halogen substituiert ist, eine C3-C7 Cycloalkylgruppe, die optional mit einem oder mehreren der Gruppe a substituiert ist, eine C3-C7 Cycloalkyloxygruppe, die optional mit einem oder mehreren der Gruppe α substituiert ist, oder Halogen, steht, und
n für eine ganze Zahl von 0 bis 3 steht;
Gruppe α: eine Gruppe, bestehend aus Halogen, C1-C7 Alkylgruppen und C1-C7 Haloalkylgruppen;
Gruppe β: eine Gruppe, bestehend aus Halogen, eine Cyanogruppe, eine Nitrogruppe, C1-C7 Alkylgruppen, C1-C7 Haloalkylgruppen, C1-C7 Alkoxygruppen und C1-C7 Haloalkoxygruppen.

2. Pyridinverbindung nach Anspruch 1, wobei mindestens ein $R^1$ eine C1-C7 Haloalkylgruppe oder eine C1-C7 Haloalkoxygruppe ist.

3. Pyridinverbindung nach Anspruch 1, wobei mindestens ein $R^1$ eine C1-C3 Fluoralkylgruppe oder eine C1-C3 Fluoralkoxygruppe ist.

4. Pyridinverbindung nach Anspruch 2 oder 3 wobei mindestens ein $R^1$ ein Substituent an der 2-Position oder 3-Position an dem Benzolring ist.

5. Pyridinverbindung nach Anspruch 1, wobei n 0 ist.

6. Pyridinverbindung nach Anspruch 1, wobei $R^2$ Wasserstoff ist.

7. Schädlingsbekämpungszusammensetzung, umfassend die Pyridinverbindung nach Anspruch 1 als ein wirksamer Inhaltsstoff.

8. Verfahren zur Schädlingsbekämpfung, umfassend das Applizieren einer wirksamen Menge der Pyridinverbindung nach Anspruch 1 an einem Schädling oder an einer Stelle, die von einem Schädling bewohnt wird; ausgenommen, dass die Stelle, die von dem Schädling bewohnt wird, ein Mensch oder Tier ist.

## Revendications

1. Composé de pyridine représenté par la formule générale (I) :

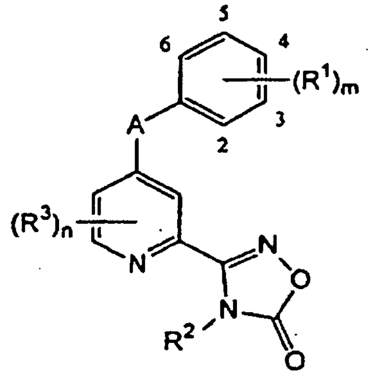

(I)

où, $R^1$ représente un groupe alkyle en C1-C7 éventuellement substitué par un halogène, un groupe alcoxy en C1-C7 éventuellement substitué par un halogène, un groupe alkylthio en C1-C7 éventuellement substitué par un halogène, un groupe nitro, un groupe cyano ou un halogène,
m représente un nombre entier de 1 à 5,

A représente une liaison simple, un atome d'oxygène, un atome de soufre, $NR^{10}$, $CH_2$ ou $CH_2O$,
$R^{10}$ représente un groupe alkyle en C1-C7 éventuellement substitué par un halogène, un groupe alcényle en C3-C7 éventuellement substitué par un halogène, un groupe alcynyle en C3-C7 éventuellement substitué par un halogène, un groupe alcoxyalkyle en C2-C7, un groupe cyanométhyle ou l'hydrogène,
$R^2$ représente un groupe alkyle en C1-C7 éventuellement substitué par un halogène, un groupe cyanométhyle, un groupe (cycloalkyle en C3-C7)méthyle éventuellement substitué par un ou plusieurs éléments choisis dans le groupe $\alpha$, un groupe benzyle éventuellement substitué par un ou plusieurs éléments choisis dans le groupe $\beta$ ou l'hydrogène, représente sinon un groupe choisi parmi les $Q^1$, $Q^2$, $Q^3$, $Q^4$ et $Q^5$ suivants

$Q^1$= 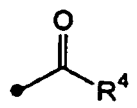 $Q^2$= 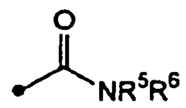

$Q^3$= 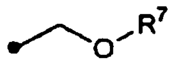 $Q^4$= 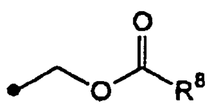 $Q^5$= 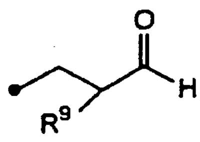

où, $R^4$ représente l'hydrogène, un groupe alkyle en C1-C7 éventuellement substitué par un halogène ou un groupe cycloalkyle en C3-C7 éventuellement substitué par un ou plusieurs éléments choisis dans le groupe $\alpha$,
$R^5$ et $R^6$ sont identiques ou mutuellement différents et représentent un groupe alkyle en C1-C7 éventuellement substitué par un halogène, un groupe alcényle en C3-C7 éventuellement substitué par un halogène, un groupe alcoxy en C1-C7 éventuellement substitué par un halogène ou un groupe cycloalkyle en C3-C7 éventuellement substitué par un ou plusieurs éléments choisis dans le groupe $\alpha$, sinon
$R^5$, $R^6$ et l'atome d'azote auquel ils sont liés représentent un groupe pyrrolidin-1-yle éventuellement substitué par un ou plusieurs éléments choisis dans le groupe $\alpha$, un groupe pipéridino éventuellement substitué par un ou plusieurs éléments choisis dans le groupe $\alpha$, un groupe hexaméthylèneimin-1-yle éventuellement substitué par un ou plusieurs éléments choisis dans le groupe $\alpha$, un groupe morpholino éventuellement substitué par un ou plusieurs éléments choisis dans le groupe $\alpha$ ou un groupe thiomorpholin-4-yle éventuellement substitué par un ou plusieurs éléments choisis dans le groupe $\alpha$,
$R^7$ représente un groupe alkyle en C1-C7 éventuellement substitué par un halogène, un groupe phényle éventuellement substitué par un ou plusieurs éléments choisis dans le groupe $\beta$, un groupe benzyle éventuellement substitué par un ou plusieurs éléments choisis dans le groupe $\beta$ ou un groupe cycloalkyle en C3-C7 éventuellement substitué par un ou plusieurs éléments choisis dans le groupe $\alpha$,
$R^8$ représente un groupe alkyle en C1-C7 éventuellement substitué par un halogène, un groupe phényle éventuellement substitué par un ou plusieurs éléments choisis dans le groupe $\beta$ ou un groupe cycloalkyle en C3-C7 éventuellement substitué par un ou plusieurs éléments choisis dans le groupe $\alpha$,
$R^9$ représente un groupe alkyle en C1-C7 éventuellement substitué par un halogène, ou l'hydrogène ;
$R^3$ représente un groupe alkyle en C1-C7 éventuellement substitué par un halogène, un groupe alcoxy en C1-C7 éventuellement substitué par un halogène, un groupe cycloalkyle en C3-C7 éventuellement substitué par un ou plusieurs éléments choisis dans le groupe $\alpha$, un groupe cycloalkyloxy en C3-C7 éventuellement substitué par un ou plusieurs éléments choisis dans le groupe $\alpha$, ou un halogène, et
n représente un nombre entier de 0 à 3 ;
Groupe $\alpha$ : un groupe constitué d'un halogène, de groupes alkyle en C1-C7 et de groupes haloalkyle en C1-C7.
Groupe $\beta$ : un groupe constitué d'un halogène, d'un groupe cyano, d'un groupe nitro, de groupes alkyle en C1-C7, de groupes haloalkyle en C1-C7, de groupes alcoxy en C1-C7 et de groupes haloalcoxy en C1-C7.

**2.** Composé de pyridine selon la revendication 1, dans lequel au moins un $R^1$ est un groupe haloalkyle en C1-C7 ou un groupe haloalcoxyle en C1-C7.

**3.** Composé de pyridine selon la revendication 1, dans lequel au moins un $R^1$ est un groupe fluoroalkyle en C1-C3 ou un groupe fluoro-alcoxy en C1-C3.

**4.** Composé de pyridine selon la revendication 2 ou 3, dans lequel au moins un $R^1$ est un substituant à la position 2 ou la position 3 sur le cycle benzène.

**5.** Composé de pyridine selon la revendication 1, dans lequel n est égal à 0.

**6.** Composé de pyridine selon la revendication 1, dans lequel $R^2$ est l'hydrogène.

**7.** Composition pesticide comprenant comme un ingrédient actif le composé de pyridine selon la revendication 1.

**8.** Procédé de contrôle de parasites comprenant l'application d'une quantité efficace du composé de pyridine selon la revendication 1 sur un parasite ou un endroit où habite un parasite ; à l'exception que l'endroit où habite le parasite est un être humain ou un animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001520666 A
- JP 2002205991 A
- EP 0374753 A
- WO 9307278 A
- WO 9534656 A
- EP 0427529 A
- EP 451878 A
- WO 03052073 A
- EP 0392225 A
- WO 03000906 A
- WO 9533818 A
- EP 0353191 A


### Non-patent literature cited in the description


- *Organic Letters,* 2006, 581-584
- *Journal of Organic Chemistry,* 1995, vol. 60, 3020-3027
- *Tetrahedron,* 2002, vol. 58, 4369-4373
- *Journal of Organic Chemistry,* 1987, vol. 52, 748-753
- Proceedings of the National Academy of Sciences of the United States of America. *Proc. Natl. Acad. Sci. USA,* 1990, vol. 87, 7175-7179
- *Weed Science,* 2005, vol. 53, 728-746
- **GURA T.** Repairing the Genome's Spelling Mistakes. *Science,* 1999, vol. 285, 316-318